(19) Europäisches Patentamt
European Patent Office
Office européen des brevets

(11) EP 1 606 288 B1

(12) # FASCICULE DE BREVET EUROPEEN

(45) Date de publication et mention de la délivrance du brevet:
**03.06.2009 Bulletin 2009/23**

(21) Numéro de dépôt: **04742333.0**

(22) Date de dépôt: **24.03.2004**

(51) Int Cl.:
***C07D 417/04*** (2006.01) ***C07D 453/02*** (2006.01)
***A61K 31/496*** (2006.01)

(86) Numéro de dépôt international:
**PCT/FR2004/000723**

(87) Numéro de publication internationale:
**WO 2004/087700 (14.10.2004 Gazette 2004/42)**

(54) **DERIVES DU BENZENESULFONAMIDE, LEUR PROCEDE DE PREPARATION ET LEUR UTILISATION POUR LE TRAITEMENT DE LA DOULEUR**

BENZOSULFONAMIDE DERIVATE, DEREN VERFAHREN ZUM HERSTELLUNG, UND DEREN VERWENDUNG ZUR BEHANDLUNG VON SCHMERZEN

BENZENESULPHONAMIDE DERIVATIVES, METHOD FOR PRODUCTION AND USE THEREOF FOR TREATMENT OF PAIN

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL LT LV MK**

(30) Priorité: **25.03.2003 FR 0303602**
**11.04.2003 FR 0304530**

(43) Date de publication de la demande:
**21.12.2005 Bulletin 2005/51**

(73) Titulaire: **LABORATOIRES FOURNIER SA**
**21000 Dijon (FR)**

(72) Inventeurs:
- **BARTH, Martine**
  **F-21380 ASNIERES-LES-DIJON (FR)**
- **BONDOUX, Michel**
  **F-21121 FONTAINE-LES-DIJON (FR)**
- **DODEY, Pierre**
  **F-21121 FONTAINE-LES-DIJON (FR)**
- **MASSARDIER, Christine**
  **F-21000 DIJON (FR)**
- **THOMAS, Didier**
  **F-21850 SAINT-APOLLINAIRE (FR)**
- **LUCCARINI, Jean-Michel**
  **F-21000 DIJON (FR)**

(74) Mandataire: **Hubert, Philippe**
**Cabinet Beau de Loménie**
**158, rue de l'Université**
**75007 Paris (FR)**

(56) Documents cités:
**WO-A-01/30734**
**WO-A-97/25315**
**WO-A-98/17655**
**WO-A-02/053516**
**WO-A-02/089792**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 606 288 B1

Printed by Jouve, 75001 PARIS (FR)

## Description

La présente invention concerne des nouveaux composés de type benzènesulfonamide, leur procédé de préparation et leur utilisation pour obtenir des compositions pharmaceutiques.

Ces nouveaux composés sont utiles en thérapeutique, particulièrement pour le traitement de la douleur.

## Art antérieur

On connaît déjà des composés comportant dans leur structure un groupement du type benzènesulfonamide. Par exemple on peut citer selon EP 236 163 et EP 236 164 des dérivés N-α-arylsulfonylaminoacyl-p-amidino-phénylalaninamides qui sont des inhibiteurs sélectifs de la thrombine et sont utiles comme anti-thrombotiques. On connaît aussi, selon EP 614 911, des composés de structure assez proche des précédentes, comportant simultanément un groupe arylsulfamoyle et un groupe phénylamidine substitué, qui ont la propriété de se fixer sur les récepteurs du neuropeptide Y et qui peuvent présenter une utilité pour soigner l'hypertension, l'angine de poitrine, l'athérosclérose, la dépression, l'anxiété, l'inflammation, l'allergie ou les surcharges graisseuses.

EP 558 961 suggère également l'utilisation de composés du type arylsulfonamide d'acides aminés substitués pour le traitement de la thrombose en raison de propriétés anticoagulantes.

Des études relatives aux propriétés antithrombotiques de composés présentant dans leur structure un groupe arylsulfonamide et un groupe phénylamidine, ont également été publiées dans Pharmazie 1984 vol. 39 (5) pages 315-317 et Pharmazie 1986 vol 41 (4) p233-235.

Dans un même domaine d'activité pharmacologique, WO 92/16549 A1 décrit des dérivés de la phénylalanine comportant un groupe arylsulfonamide, qui sont des inhibiteurs de protéinase, notamment des inhibiteurs de la thrombine.

On connaît aussi, selon WO 97/25315, des composés de structure N-(arylsulfonyl)amino-acides, utiles pour traiter les maladies inflammatoires.

Parmi les documents de l'art antérieur proposant des éléments de structure de type arylsulfonamide, on peut citer WO 96/40639, WO 97/24349, WO 98/03503, WO 98/24783 et WO 99/00387, relatifs à des composés antagonistes du récepteur $B_2$ de la bradykinine. On connaît également des composés antagonistes du récepteur $B_1$ de la bradykinine, peptidiques ou non peptidiques, selon les documents WO 01/05783, WO 02/099388 et WO 97/09346.

## Objet de l'invention

L'invention concerne de nouveaux composés comportant l'enchaînement benzènesulfonamide substitué, lesdits composés étant notamment utiles en tant que principes actifs de médicaments destinés au traitement de la douleur, particulièrement les hyperalgésies et les algésies majeures.

## Description

Selon la présente invention, on propose en tant que produit industriel nouveau, un composé de type benzènesulfonamide caractérisé en ce qu'il est choisi parmi l'ensemble constitué par :

a) les composés de formule :

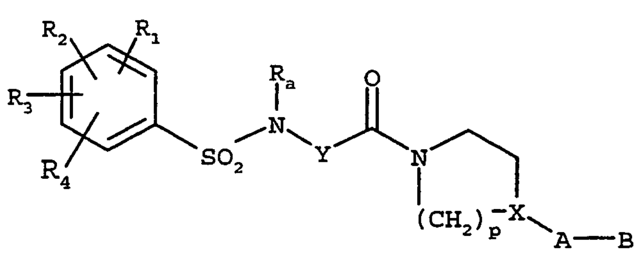

I

dans laquelle

- $R_1$, $R_2$, $R_3$, $R_4$ représentent chacun indépendamment un ou plusieurs des atomes

ou groupes d'atomes choisi(s) parmi l'atome d'hydrogène, les halogènes, les groupes alkyle en $C_1$-$C_3$, alcoxy en $C_1$-$C_3$, $CF_3$ ou $OCF_3$,

- $R_a$ représente un groupe alkyle en $C_1$-$C_4$,
- Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH_2-$,
- X représente CH ou un atome d'azote,
- p représente 2 ou 3,
- A représente une liaison simple, un atome d'azote éventuellement substitué par un groupe méthyle, ou un groupe alkylène en $C_1$-$C_5$, linéaire ou ramifié, éventuellement hydroxylé ou dont l'un des atomes de carbone est oxydé en une fonction cétone, à la condition que A et X ne représentent pas ensemble un atome d'azote,
- B représente un hétérocycle azoté ou un groupe amine éventuellement substitué par un ou deux groupes alkyle en $C_1$-$C_4$,

b) les sels d'addition des composés de formule I ci-dessus avec un acide.

L'invention concerne également, lorsque les composés de formule I comprennent un centre d'asymétrie, chacun des isomères optiques purs ou en mélange, ainsi que leurs sels respectifs ou le mélange de leurs sels.
Selon l'invention, on préconise aussi un procédé pour la préparation des composés de formule I ainsi que de leurs sels d'addition.
On préconise également l'utilisation d'une substance choisie parmi les composés de formule I et leurs sels d'addition non toxiques pour la préparation d'un médicament, utile en thérapeutique humaine ou animale, destiné à la prévention ou au traitement de pathologies liées à la douleur, notamment les hyperalgésies consécutives à un état inflammatoire ou les algésies majeures liées à d'autres états pathologiques tels que, par exemple, le cancer.

**Description détaillée**

Dans la formule I, on entend par groupe alkyle une chaîne hydrocarbonée, linéaire, ramifiée ou cyclisée. Un groupe alkyle en $C_1$-$C_3$ est notamment un groupe méthyle, éthyle, propyle, cyclopropyle ou 1-méthyl-éthyle. Un groupe alkyle en $C_1$-$C_4$ comprendra notamment, en plus des exemples cités ci-dessus, les groupes butyle, 1-méthylpropyle et 1,1-diméthyléthyle.
Par groupe alcoxy en $C_1$-$C_3$, on comprend un groupe OR dans lequel R est un groupe alkyle en $C_1$-$C_3$, le terme alkyle ayant la signification donnée ci-dessus. Un tel groupe est par exemple un groupe méthoxy, éthoxy, propoxy ou 1-méthyléthoxy.
Par groupe alkylène en $C_2$-$C_5$ saturé, il faut comprendre un groupe $-(CH_2)_n-$dans lequel n est 2, 3, 4 ou 5 s'il s'agit d'un groupe linéaire ou, par exemple, un groupe $-CH(CH_3)-CH_2-CH_2-$ ou $-C(CH_3)_2-CH_2-$ s'il s'agit d'un groupe ramifié. Dans le cas d'un groupe alkylène interrompu par un atome d'oxygène, on entend par exemple les groupes $-CH_2-CH_2-O-CH_2-$, $-CH_2-O-CH_2-CH_2-$ ou encore $-CH_2-CH_2O-CH_2-CH_2-$. Par groupe alkylène en $C_2$-$C_4$ insaturé, il faut comprendre un groupe comprenant 2 à 4 atomes de carbone dont 2 consécutifs sont liés par une liaison éthylénique, par exemple un groupe $-CH_2-CH=CH-CH_2-$, $-CH=CH-CH_2-$, $-CH=C(CH_3)-CH_2-$, $CH_2-CH=CH-$ ou $-CH=CH-CH(CH_3)-$.
On entend par halogène un atome de fluor, de chlore ou de brome et, préférentiellement, un atome de fluor ou de chlore.
Par groupes alkylamino ou dialkylamino on entend les groupe NH(R) ou $N(R)_2$ dans lequel R est un groupe alkyle en $C_1$-$C_4$, le terme alkyle ayant la signification donnée ci-dessus.
Par hétérocycle azoté, on entend notamment les cycles azétidine, pyrrolidine, morpholine, pipéridine, pipérazine, N-($C_1$-$C_4$)alkylpipéridine, N-($C_1$-$C_4$)alkylpipérazine, quinuclidine, tropane, N-($C_1$-$C_4$)alkylhomopipérazine, aminopyridine, N-($C_1$-$C_4$)alkylimidazole.
Par sels d'addition, on entend les sels d'addition obtenus par réaction d'un composé de formule I contenant au moins une fonction basique sous sa forme non salifiée, avec un acide minéral ou organique. De préférence, il s'agira de sels d'addition pharmaceutiquement acceptables.
Parmi les acides minéraux convenant pour salifier un composé basique de formule I, on préfère les acides chlorhydrique, bromhydrique, phosphorique et sulfurique. Parmi les acides organiques convenant pour salifier un composé basique de formule I, on préfère les acides méthanesulfonique, benzènesulfonique, toluènesulfonique, maléique, fumarique, oxalique, citrique, tartrique, lactique et trifluoroacétique.
Parmi les composés de formule I, on préfère ceux qui remplissent au moins l'une des conditions suivantes:

- $R_1$ représente l'hydrogène, un halogène, un groupe alkyle en $C_1$-$C_3$ ou alcoxy en $C_1$-$C_3$;
- $R_2$ représente un halogène, un groupe alkyle en $C_1$-$C_3$ ou $CF_3$;
- $R_3$ représente l'hydrogène, un halogène, un groupe alkyle en $C_1$-$C_3$ ou alcoxy en $C_1$-$C_3$;
- $R_4$ représente l'hydrogène ou un groupe alkyle en $C_1$-$C_3$;
- $R_a$ représente un groupe méthyle;

- Y représente un groupe alkylène en $C_2$-$C_5$ saturé, éventuellement interrompu par un atome d'oxygène;
- p représente 2;
- A représente une liaison simple ou un groupe alkylène en $C_1$-$C_5$ linéaire ou ramifié;
- B représente un groupe azétidinyle, pyrrolidinyle, morpholinyle, pipéridinyle, pipérazinyle, N-($C_1$-$C_4$)alkylpipéridinyle, N-($C_1$-$C_4$)alkylpipérazinyle, quinuclidinyle, tropanyle, N-($C_1$-$C_4$)alkylhomopipérazinyle.

On préfère tout particulièrement les composés de formule I qui remplissent au moins l'une des conditions suivantes:

- $R_1$ représente un groupe alcoxy en $C_1$-$C_3$;
- $R_2$ et $R_3$ représentent chacun un groupe alkyle en $C_1$-$C_3$, de préférence un groupe méthyle en position 2,6 sur le cycle aromatique;
- $R_4$ représente l'hydrogène;
- Y représente un groupe alkylène en $C_3$-$C_5$ interrompu par un atome d'oxygène, préférentiellement un groupe $-CH_2CH_2-O-CH_2-$;
- B représente un groupe pipéridinyle, N-($C_1$-$C_4$)alkylpipéridinyle, N-($C_1$-$C_4$)alkylpipérazinyle.

On préfère également les composés de formule Ia:

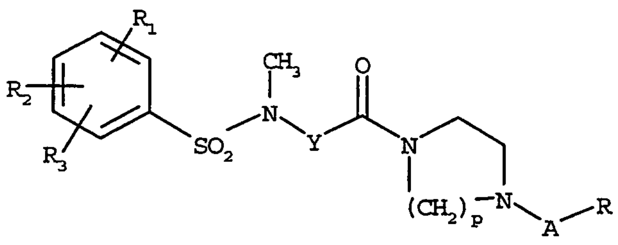

Ia

dans laquelle

- $R_1$, $R_2$, $R_3$ représentent chacun indépendamment un ou plusieurs des atomes ou groupes d'atomes choisi(s) parmi l'atome d'hydrogène, les halogènes, les groupes alkyle en $C_1$-$C_3$, alcoxy en $C_1$-$C_3$, $CF_3$ ou $OCF_3$,
- Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH_2-$,
- A représente une liaison simple ou un groupe $-(CH_2)_m-$,
- R représente un hétérocycle azoté saturé, notamment choisi parmi les cycles pyrrolidine, morpholine, pipéridine, quinuclidine, tropane, ou un groupe amine tertiaire, notamment un groupe dialkylamino,
- m et p représentent chacun indépendamment 2 ou 3.

On préfère également les composés de formule Ib:

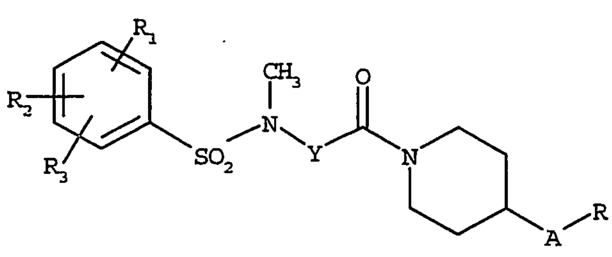

Ib

dans laquelle

- $R_1$, $R_2$, $R_3$ représentent chacun indépendamment un ou plusieurs des atomes ou groupes d'atomes choisi(s) parmi l'atome d'hydrogène, les halogènes, les groupes alkyle en $C_1$-$C_3$ , alcoxy en $C_1$-$C_3$, $CF_3$ ou $OCF_3$,
- Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH_2-$,

- A représente une liaison simple ou un groupe alkylène en $C_1$-$C_5$ saturé éventuellement hydroxylé,
- R représente un hétérocycle azoté saturé, notamment choisi parmi les cycles N-méthylpipérazine, N-méthylpipéridine, ou un groupe amine tertiaire, notamment un groupe dialkylamino.

Selon l'invention, on préconise un procédé général de préparation des composés de formule I ou de leurs sels d'addition, comprenant les étapes consistant à :

a) faire réagir un acide de formule :

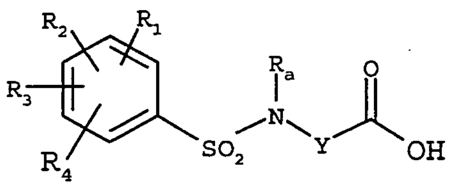

II

dans laquelle

$R_1$, $R_2$, $R_3$, $R_4$ représentent chacun indépendamment un ou plusieurs des atomes ou groupes d'atomes choisi (s) parmi l'atome d'hydrogène, les halogènes, les groupes alkyle en $C_1$-$C_3$, alcoxy en $C_1$-$C_3$, $CF_3$ ou $OCF_3$,
$R_a$ représente un groupe alkyle en $C_1$-$C_4$,
Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH_2-$,

avec un hétérocycle azoté de formule :

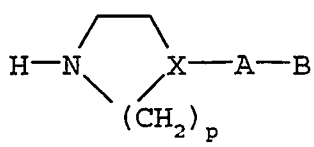

III

dans laquelle

X représente CH ou un atome d'azote,
p représente 2 ou 3,
A représente une liaison simple, un atome d'azote éventuellement substitué par un groupe méthyle (si X ne représente pas aussi un atome d'azote), ou un groupe alkylène en $C_1$-$C_5$, linéaire ou ramifié, éventuellement hydroxylé ou dont l'un des atomes de carbone est oxydé en une fonction cétone,
B représente un hétérocycle azoté ou un groupe amine éventuellement substitué par un ou deux groupes alkyle en $C_1$-$C_4$, étant entendu que, en cas de présence d'un atome d'azote non substitué, cet atome d'azote est protégé par un groupe aminoprotecteur tel que par exemple un groupe Boc (1,1-diméthyléthoxycarbonyle) ou Cbz (phénylméthoxycarbonyle),

dans un solvant tel que par exemple le tétrahydrofurane, le dichlorométhane ou le diméthylformamide, en présence d'activateurs tels que par exemple l'EDCI (1-(3-diméthylaminopropyl)-3-éthylcarbodiimide), le DIC (diisopropylcarbodiimide) ou le HOAT (1-hydroxy-7-azabenzotriazole), à une température généralement comprise entre la température ambiante et la température d'ébullition du solvant, pendant environ 2 à 15 heures, pour obtenir l'amide de formule :

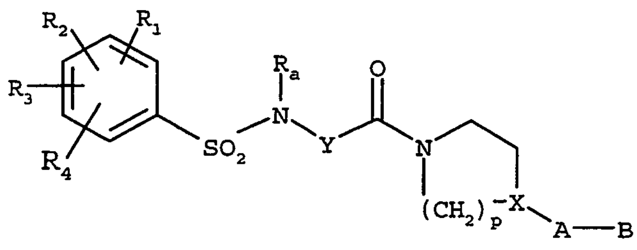

I

dans laquelle $R_1$, $R_2$, $R_3$, $R_4$, $R_a$, Y, p, X, A et B conservent la même signification que dans les composés de départ,

b) si nécessaire éliminer les groupes aminoprotecteurs, par exemple par action de l'acide trifluoroacétique en présence d'anisole si ledit groupe aminoprotecteur est un groupe Boc, ou par hydrogénation catalytique si le groupe protecteur est le groupe Cbz,

c) si nécessaire obtenir un sel d'addition du composé de formule I avec un acide minéral ou organique, selon des mises en oeuvre habituelles connues de l'homme du métier.

Ce procédé général de préparation d'une fonction amide au départ d'un acide carboxylique et d'une amine peut être modifié pour utiliser des activateurs immobilisés sur une résine insoluble, par exemple des résines à squelette polystyrène supportant des fonctions carbodiimide.

En variante du procédé général décrit ci-dessus, l'acide de formule II peut être transformé intermédiairement en chlorure d'acide de formule IIa,

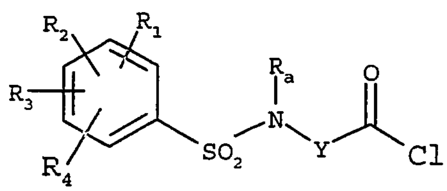

IIa

notamment par action d'un agent de chloration tel que le chlorure d'oxalyle ou le chlorure de thionyle, ledit chlorure d'acide étant ensuite mis en réaction avec l'hétérocycle azoté de formule III, selon une réaction classique conduite dans un solvant et de préférence en présence d'une base organique aprotique telle que par exemple la triéthylamine ou la pyridine, pour obtenir le composé de formule I.

Les acides de formule II dans laquelle Y représente un groupe $-CH_2-CH_2O-CH_2-$peuvent être préparés selon un procédé consistant à :

a) faire réagir un chlorure de benzènesulfonyle de formule :

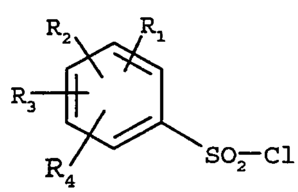

IV

dans laquelle $R_1$, $R_2$, $R_3$ et $R_4$ représentent chacun indépendamment un atome d'hydrogène ou d'halogène, un groupe alkyle en $C_1$-$C_3$, alcoxy en $C_1$-$C_3$, $CF_3$ ou $OCF_3$, avec un aminoalcool de formule

$HN(R_a)-CH_2-CH_2-OH$

dans laquelle $R_a$ représente un groupe alkyle en $C_1$-$C_4$,

dans un solvant tel que par exemple le dichlorométhane, en présence d'une base organique aprotique comme par exemple la triéthylamine ou la pyridine, à une température comprise entre environ 0 et 50 °C, pendant environ 1 à 3 heures, pour obtenir le sulfonamide de formule :

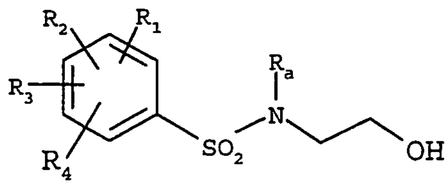

V

dans laquelle $R_1$, $R_2$, $R_3$, $R_4$ et $R_a$ restent inchangés,

b) faire réagir le composé de formule V obtenu ci-dessus avec un ester de l'acide bromacétique, préférentiellement l'ester *t*-butylique, en présence d'une base comme par exemple la soude et dans un milieu favorable au transfert de phase contenant des sels d'ammonium quaternaire, dans un mélange de solvants tels que l'eau et le toluène, à une température comprise entre environ 0 °C et 40 °C et pendant environ 1 à 5 heures, pour obtenir l'ester de formule :

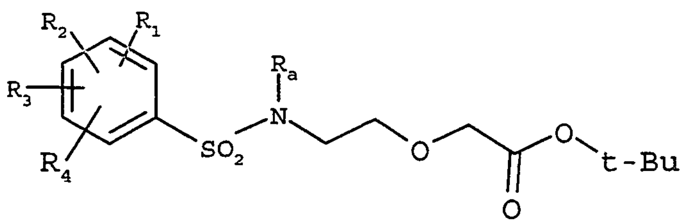

VI

c) hydrolyser l'ester de formule VI, par exemple par action de l'acide trifluoroacétique, la réaction étant conduite dans un solvant tel que le dichlorométhane, à une température comprise entre environ 0 °C et 50 °C et pendant environ 1 à 6 heures, pour obtenir l'acide de formule II :

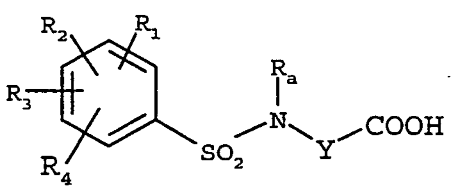

II

dans laquelle $R_1$, $R_2$, $R_3$, $R_4$ et $R_a$ restent inchangés et Y représente le groupe $-CH_2-CH_2-O-CH_2-$.

En variante du procédé general, les composés de formule I peuvent être obtenus en effectuant successivement les étapes consistant à :

a) faire réagir un composé acide de formule

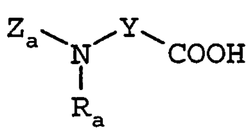

VII

dans laquelle $R_a$ représente un groupe alkyle en $C_1$-$C_4$,

Y représente un groupe alkylène en $C_2$-$C_5$ saturé, éventuellement interrompu par un atome d'oxygène, et $Z_a$ représente un groupe aminoprotecteur comme par exemple un groupe benzyle, avec un hétérocycle azoté de formule :

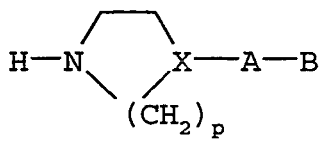

III

dans laquelle

X représente CH ou un atome d'azote,
p représente 2 ou 3,
A représente une liaison simple, un atome d'azote éventuellement substitué par un groupe méthyle (si X ne représente pas aussi un atome d'azote), ou un groupe alkylène en $C_1$-$C_5$, linéaire ou ramifié, éventuellement hydroxylé ou dont l'un des atomes de carbone est oxydé en une fonction cétone,
B représente un hétérocycle azoté ou un groupe amine éventuellement substitué par un ou deux groupes alkyle en $C_1$-$C_4$, étant entendu que, en cas de présence d'un atome d'azote non substitué, cet atome d'azote est protégé par un groupe aminoprotecteur différent du groupe aminoprotecteur utilisé pour le composé acide VII, tel que par exemple un groupe Boc (1, 1-diméthyléthoxycarbonyle),

dans un solvant tel que par exemple le tétrahydrofurane, le dichlorométhane ou le diméthylformamide, en présence d'activateurs tels que par exemple l'EDCI (1-(3-diméthylaminopropyl)-3-éthylcarbodiimide), le DIC (diisopropylcarbodiimide) ou le HOAT (1-hydroxy-7-azabenzotriazole), à une température généralement comprise entre la température ambiante et la température d'ébullition du solvant, pendant environ 2 à 15 heures, pour obtenir l'amide de formule :

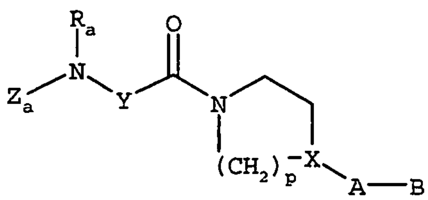

VIII

dans laquelle $Z_a$, $R_a$, Y, p, X, A et B conservent la même signification que dans les composés de départ,
b) éliminer le groupe amino protecteur $Z_a$, par exemple par hydrogénation catalytique si $Z_a$ est un groupe benzyle, pour obtenir l'amine secondaire de formule

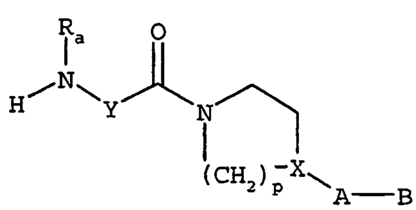

IX

dans laquelle $R_a$, Y, p, X, A et B conservent la même signification que dans le composé précédent,
c) faire réagir cette amine secondaire IX avec un chlorure de benzènesulfonyle de formule :

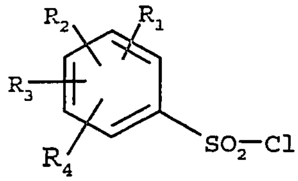

IV

dans laquelle $R_1$, $R_2$, $R_3$ et $R_4$ représentent chacun indépendamment un atome d'hydrogène ou d'halogène, un groupe alkyle en $C_1$-$C_3$, alcoxy en $C_1$-$C_3$, $CF_3$ ou $OCF_3$, dans un solvant tel que par exemple le dichlorométhane, en présence d'une base organique aprotique comme par exemple la triéthylamine ou la pyridine, à une température comprise entre environ 0 et 50 °C, pendant environ 1 à 3 heures, pour obtenir le sulfonamide de formule :

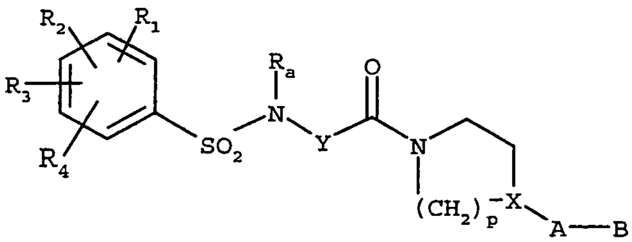

I

dans laquelle $R_1$, $R_2$, $R_3$, $R_4$, $R_a$, Y, p, X, A et B conservent la même signification que dans les composés de départ,
d) si nécessaire éliminer les groupes aminoprotecteurs, par exemple par action de l'acide trifluoroacétique en présence d'anisole si ledit groupe aminoprotecteur est un groupe Boc,
e) si nécessaire obtenir un sel d'addition du composé de formule I avec un acide minéral ou organique.

En variante du procédé ci-dessus, l'étape a) de formation de la liaison amide peut être réalisée en formant dans un premier temps le chlorure de l'acide de formule VII, par exemple par action du chlorure d'oxalyle ou du chlorure de thionyle dans un solvant aprotique anhydre, puis réaction du chlorure d'acide obtenu avec l'amine de formule III telle que décrite ci-dessus, dans un solvant et par exemple en présence d'une base aprotique telle que le triéthylamine.

Les dérivés hétérocycliques de formule III sont des composé connus, commercialisés ou décrits dans la littérature, ou peuvent être préparés en utilisant des procédés connus de l'homme de métier, par exemple par une réaction d'amination réductrice entre la pipérazine ou l'homopipérazine et une cétone, par exemple en présence d'isopropylate de titane, puis d'un agent réducteur tel que le cyanoborohydrure de sodium, ou, dans le cas de dérivés de la pipéridine, par hydrogénation catalytique de composés homologues de la pyridine.

L'invention sera mieux comprise à l'aide des exemples de préparation de composés, ainsi que des résultats d'essais pharmacologiques démontrant l'utilité de ces composés en thérapeutique. Ces exemples sont non limitatifs et ne sauraient limiter la portée de l'invention.

Parmi les abréviations utilisées dans la description, M signifie mole, mM signifie millimole ($10^{-3}$ mole). THF signifie tétrahydrofurane, DCM signifie dichlorométhane, DMF signifie diméthylformamide, TFA signifie acide trifluoroacétique. Dans le cas de composés présentant un centre d'asymétrie, l'absence d'indication particulière signifie que le composé est sous forme du mélange racémique. Dans les données spectrales de résonnance magnétique nucléaire, les déplacements chimiques sont indiqués en référence au TMS (tétraméthylsilane).

**PREPARATION I**

**N (2-hydroxyéthyl)-4-méthoxy-N,2,6-triméthylbenzènesulfonamide**

On prépare une solution de 1,76 g (23,4 mM) de 2-(méthylamino)éthanol dans 100 ml de DCM et on ajoute 5,4 g (53 mM) de triéthylamine. Le mélange est refroidi à 0 °C et on ajoute progressivement une solution de 5 g (21,3 mM) de chlorure de 2,6 diméthyl-4-méthoxybenzènesulfonyle dans 50 ml de DCM. Le mélange est ensuite agité pendant 3 heures à température ambiante, puis versé sur 50 ml d'acide chlorhydrique 0,5 N. La phase organique est séparée puis lavée à l'eau, séchée sur sulfate de magnésium et concentrée sous pression réduite. On obtient ainsi 5,8 g du composé attendu sous forme d'une huile incolore (rendement = 100 %).

RMN $^1$H (300 MHz, DMSO) $\delta$ : 6,80 (s, 2H) ; 4,70 (t, 1H) ; 3,80 (s, 3H) ; 3,48 (q, 2H) ; 3,09 (t, 2H) ; 2,69 (s,

3H) ; 2,54 (s, 6H).

**PREPARATION II**

**Acide [2-[[(4-méthoxy-2,6-diméthylphényl)sulfonyl]méthylamino]-éthoxy] acétique, 1,1-diméthyléthyl ester**

On prépare une solution de 4,85 g (17,7 mM) du composé obtenu selon la préparation I dans 100 ml de toluène et on ajoute 1,62 g de chlorure de tetrabutylammonium. Le mélange est refroidi à 0 ˚C puis on ajoute 100 ml de soude à 35 %, puis, progressivement, 3,95 ml (26,6 mM) de bromacétate de t-butyle. Le mélange est agité à température ambiante pendant 2 heures puis la phase organique est séparée par décantation, et lavée à l'eau jusqu'à pH neutre puis séchée sur sulfate de sodium. Après concentration sous pression réduite, on obtient une huile que l'on purifie par chromatographie sur gel de silice en éluant à l'aide d'un mélange cyclohexane/acétate d'éthyle (75/25 ; v/v). On obtient ainsi 6,5 g du composé attendu sous forme d'une huile incolore (rendement = 94 %).

RMN $^1$H (250 MHz, DMSO) δ : 6,80 (s, 2H) ; 3,89 (s, 2H) ; 3,80 (s, 3H) ; 3,56 (t, 2H) ; 3,21 (t, 2H) ; 2,71 (s, 3H) ; 2,53 (s, 3H) ; 1,41 (s, 9H).

**PREPARATION III**

**Acide [2-[[(4-méthoxy-2,6-diméthylphényl)sulfonyl]méthylamino]-éthoxy] acétique**

On dissout 6,4 g (16,5 mM) de l'ester obtenu selon la préparation II dans 80 ml de DCM, et on ajoute 8 ml d'acide trifluoroacétique. Le mélange est agité à température ambiante pendant 4 heures, puis concentré sous pression réduite. Le résidu d'évaporation est repris en solution dans 100 ml de soude 1N et la solution obtenue est lavée 2 fois par 30 ml d'acétate d'éthyle, puis acidifiée par une solution N d'acide chlorhydrique et extraite par 2 fois 80 ml d'acétate d'éthyle. La phase organique est lavée à l'eau, séchée sur sulfate de soude et concentrée sous pression. On obtient ainsi l'acide attendu sous forme d'une huile qui cristallise (rendement = 95 %).
F=82 ˚C

**Exemple 1**

**1-[[2-[[(4-méthoxy-2,6-diméthylphényl)sulfonyl]méthylamino]-éthoxy]acétyl]-4-[2-(1-pyrrolidinyl)éthyl]pipérazine, bis-trifluoroacétate**

On conditionne 482 mg de résine polystyrène greffée cyclohexyldiimide dans 5 ml de THF pendant 20 mn puis on ajoute 100 mg (0,31 mM) d'acide obtenu selon la préparation III en solution dans 2 ml de THF, 37 mg (0,20 mM) de 1-[2-(1-pyrrolidinyl)éthyl]pipérazine et 2 mg de HOAT (1-hydroxy-7-azabenzotriazole). Le mélange est agité pendant 4 heures à température ambiante puis filtré. La résine est rincée par 4 ml de THF que l'on joint au filtrat. Le filtrat est ensuite agité avec 50 mg de résine Amberlite IRA 400 (sous forme OH-), pendant 3 heures. Après filtration, la résine est rincée par 3 ml de THF que l'on joint au filtrat. Ce filtrat est ensuite traité avec 100 mg de résine polystyrène greffée isocyanate pendant 1 heure. La résine et séparée par filtration et la solution est concentrée sous pression réduite. Le résidu (63 mg) est purifié par chromatographie en phase inverse sur colonne X Terra Prep MS C18 (éluant A : eau + 0,05 % TFA, éluant B : acétonitrile + 0,05 % TFA, gradient : 10 % B (t = 0 à 2 mn) 60 % B (t = 2 à 17 mn) 100 % B (t = 17 mn à 18 mn) ; débit = 25 ml/mn ; détection UV de 120 à 260 nm. Le produit purifié est repris dans 1 ml d'acétonitrile et on le mélange avec 6 ml d'une solution à 1 % d'acide trifluoroacétique dans l'eau. La solution obtenue est ensuite lyophilisée et on obtient 19 mg du sel attendu sous forme d'une huile jaune (rendement = 13 %).

RMN $^1$H (300 MHz, $CD_3CN$) δ : 6,74 (s, 2H) ; 4,09 (s, 2H) ; 3,96 (s, 3H) ; 3,70 (m, 1H) ; 3,59 (t, 2H) ; 3,48 (t, 2H) ; 3,33 (m, 5H) ; 3,19 (t, 2H) ; 3,00 (m, 4H) ; 2,72 (s; 3H) ; 2,56 (s, 6H) ; 2,04 (m, 4H).

**PREPARATION IV**

**Acide [2-[[(2,4,6-triméthylphényl)sulfonyl]méthylamino]éthoxy]-acétique, 1,1-diméthyléthyl ester**

En opérant de façon analogue à la préparation II, au départ de N-(2-hydroxyéthyl)-N,2,4,6-tétraméthylbenzènesulfonamide, on obtient l'ester attendu sous forme d'une huile beige (rendement = 98 %).

RMN $^1$H (250 MHz, DMSO) δ : 7,06 (s, 2H) ; 4,01 (s, 2H) ; 3,58 (t, 2H) ; 3,25 (t, 2H) ; 2,72 (s, 3H) ; 2,51 (s, 6H) ; 2,27 (s, 3H) ; 1,43 (s, 9H).

**PREPARATION V**

**Acide [2-[[(2,4,6-triméthylphényl)sulfonyl]méthylamino]éthoxy]-acétique**

En opérant de façon analogue à la préparation III, au départ du composé obtenu selon la préparation IV, on obtient le produit attendu sous forme d'un solide beige (rendement = 83 %).
F = 58 °C

**PREPARATION VI**

**N-éthyl-N-(2-hydroxyéthyl)-4-méthoxy-2,6-diméthylbenzènesulfonamide**

En opérant de façon analogue à la préparation I, au départ de 2-(éthylamino)éthanol, on obtient le produit attendu sous forme d'une huile jaune (rendement = 99%)

RMN $^1H$ (250 MHz, DMSO) δ: 6,80 (s, 2H) ; 4,69 (t, 1H) ; 3,80 (s, 3H) ; 3,38 (m, 2H) ; 3,14 (s, 2H).

**PREPARATION VII**

**Acide [2-[[(4-méthoxy-2,6-diméthylphényl)sulfonyl]éthylamino]-éthoxy]acétique, 1,1-diméthyléthyl ester**

En opérant de façon analogue à la préparation II, au départ du composé obtenu selon la préparation VI, on obtient le produit attendu sous forme d'une huile incolore (rendement = 79%).

RMN $^1H$ (250 MHz, DMSO) δ : 6,79 (s, 2H) ; 3,86 (s, 2H) ; 3,79 (s, 3H) ; 3,47 (t, 2H) ; 3,24 (m, 4H) ; 2,53 (s, 6H) ; 1,40 (s, 9H) ; 1,00 (t, 3H).

**PREPARATION VIII**

**Acide [2-[[(4-méthoxy-2,6-diméthylphényl)sulfonyl]éthylamino]-éthoxy]acétique**

En opérant de façon analogue à la préparation III, au départ du composé obtenu selon la préparation VII, on obtient le produit attendu sous forme d'un solide blanc (rendement = 88%).

RMN $^1H$ (250 MHz, DMSO) δ : 6,79 (s, 2H) ; 3,89 (s, 2H) ; 3,79 (s, 3H) ; 3,48 (t, 2H) ; 3,24 (q, 2H) ; 3,21 (t, 2H) ; 2,53 (s, 6H) ; 1,00 (t, 3H).

**PREPARATION IX**

**N-(2-hydroxyéthyl)-4-méthoxy-2,6-diméthyl-N-(1-méthyléthyl)benzènesulfonamide**

En opérant de façon analogue à la préparation I, au départ de 2-[(1-méthyléthyl)amino]éthanol, on obtient le produit attendu sous forme d'une huile incolore (rendement = 58%).

RMN $^1H$ (300 MHz, DMSO) δ : 6,80 (s, 2H) ; 4,68 (t, 1H) ; 3,80 (s, 3H) ; 3,73 (quin, 1H) ; 3,27 (dt, 2H) ; 3,20 (q, 2H) ; 3,12 (t, 2H) ; 2,53 (s, 6H) ; 1,06 (d, 6H) ; 0,99 (t, 3H).

**PREPARATION X**

**Acide [2-[[(4-méthoxy-2,6-diméthylphényl)sulfonyl)(1-méthyléthyl)amino)-éthoxy]acétique, 1,1-diméthyléthyl ester**

En opérant de façon analogue à la préparation II, au départ du composé obtenu selon la préparation IX, on obtient le produit attendu sous forme d'une huile incolore (rendement = 95%).

RMN $^1H$ (250 MHz, DMSO) δ : 6,80 (s, 2H) ; 3,86 (s, 2H) ; 3,80 (s, 3H) ; 3,74 (quin, 1H) ; 3,82 (m, 2H) ; 3,26 (m, 2H) ; 2,53 (s, 6H) ; 1,40 (s, 9H) ; 1,07 (d, 6H).

**PREPARATION XI**

**Acide [2-[[(4-méthoxy-2,6-diméthylphényl)sulfonyl](1-méthyléthyl)amino]-éthoxy] acétique**

En opérant de façon analogue à la préparation III, au départ du composé obtenu selon la préparation X, on

obtient le produit attendu sous forme d'un solide blanc (rendement = 85%).
F=96˚C

## PREPARATION XII

**N-cyclopropyl-N-(2-hydroxyéthyl)-4-méthoxy-2,6-diméthylbenzènesulfonamide**

En opérant de façon analogue à la préparation I, au départ de 2-(cyclopropylamino)éthanol, on obtient le produit attendu sous forme d'un solide blanc (rendement = 77%).
F = 58˚C

## PREPARATION XIII

**Acide [2-[cyclopropyl[(4-méthoxy-2,6-diméthylphényl)sulfonyl] amino]éthoxy]acétique, 1,1-diméthyléthyl ester**

En opérant de façon analogue à la préparation II, au départ du composé obtenu selon la préparation XII, on obtient le produit attendu sous forme d'une huile incolore (rendement = 84%).

RMN $^1$H (300 MHz, DMSO) δ : 6,80 (s, 2H) ; 3,98 (s, 2H) ; 3,80 (s, 3H) ; 3,69 (t, 2H) ; 3,44 (t, 2H) ; 2,50 (s, 6H) ; 2,47 (m, 1H) ; 1,42 (s, 9H) ; 0,48 (m, 2H) ; 0,16 (m, 2H).

## PREPARATION XIV

**Acide [2-[cyclopropyl[(4-méthoxy-2,6-diméthylphényl)sulfonyl]amino] éthoxy]acétique**

En opérant de façon analogue à la préparation III, au départ du composé obtenu selon la préparation XIII, on obtient le produit attendu sous forme d'un solide blanc (rendement = 85%).
F = 100˚C

## PREPARATION XV

**Chlorure de 2,6-dichloro-4-méthoxybenzènesulfonyle et chlorure de 2,4-dichloro-6-méthoxybenzènesulfonyle**

On prépare une solution de 15 g (84,7 mM) de 3,5-dichloroanisole dans 8 ml de chlorure de thionyle que l'on refroidit à - 10˚C, puis on ajoute goutte à goutte 6 ml (90 mM) d'acide chlorosulfonique. Le mélange réactionnel est ensuite agité pendant 3 heures à température ambiante, puis versé sur un mélange de glace et d'acétate d'éthyle. La phase organique est séparée, séchée sur sulfate de magnésium et concentrée sous pression réduite. Le résidu d'évaporation est utilisé sans autre purification dans l'étape suivante.

## PREPARATION XVIa

**2,6-dichloro-N-(2-hydroxyéthyl)-4-méthoxy-N-méthyl-benzènesulfonamide**

## PREPARATION XVIb

**2,4-dichloro-N-(2-hydroxyéthyl)-6-méthoxy-N-méthyl-benzènesulfonamide**

En opérant de façon analogue à la préparation I, au départ des chlorures de sulfonyle obtenus selon la préparation XV, on obtient, après séparation des composés et purification par chromatographie sur gel de silice, en éluant à l'aide d'un mélange toluène/isopropanol (95/5 ;v/v), les produits attendus sous forme de solides blancs.

**PREPARATION XVIa (rendement = 13%) : F = 47°C**

**PREPARATION XVIb (rendement = 48 %) : F =100°C**

**PREPARATION XVII**

**Acide [2-[[(2,6-dichloro-4-méthoxyqlhényl)sulfonyl]méthylamino]éthoxy] acétique,1,1-diméthyléthyl ester**

En opérant de façon analogue à la préparation II, au départ du composé obtenu selon la préparation XVIa, on obtient le produit attendu sous forme d'une huile incolore (rendement = 52%).

RMN $^1$H (300 MHz, DMSO) δ : 7,24 (s, 2H) ; 3,94 (s, 2H) ; 3,87 (s, 3H) ; 3,60 (t, 2H) ; 3,39 t, 2H) ; 2,90 (s, 3H) ; 1,41 (s, 9H).

**PREPARATION XVIII**

**Acide [2-[[(2,6-dichloro-4-méthoxyphényl)sulfonyl]méthylamino]éthoxy] acétique**

En opérant de façon analogue à la préparation III, au départ du composé obtenu selon la préparation XVII, on obtient le produit attendu sous forme d'un solide blanc (rendement = 94%).
F = 95°C

**PREPARATION XIX**

**Acide [2-[[(2,4-dichloro-6-méthoxyphényl)sulfonyl]méthylamino]éthoxy] acétique, 1,1-diméthyléthyl ester**

En opérant de façon analogue à la préparation II, au départ du composé obtenu selon la préparation XVIb, on obtient le produit attendu sous forme d'une huile utilisée sans autre purification dans l'étape suivante.

**PREPARATION XX**

**Acide [2-[[(2,4-dichloro-6-méthoxyphényl)sulfonyl]méthylamino] éthoxy] acétique**

En opérant de façon analogue à la préparation III, au départ du composé obtenu selon la préparation XIX, on obtient le produit attendu sous forme d'un solide blanc (rendement = 76%).
F = 74°C

**PREPARATION XXI**

**2,4-dichloro-N-(2-hydroxyéthyl)-3,N-diméthyl-benzènesulfonamide**

En opérant de façon analogue à la préparation I, au départ du chlorure de 2,4-dichloro-3-méthylbenzènesulfonyle, on obtient le produit attendu sous forme d'une huile incolore.

**PREPARATION XXII**

**Acide [2-[[(2,4-dichloro-3-méthylphényl)sulfonyl]méthylamino]éthoxy] acétique, 1,1-diméthyléthyl ester**

En opérant de façon analogue à la préparation II, au départ du composé obtenu selon la préparation XXI, on obtient le produit attendu sous forme d'une huile incolore (rendement = 87%).

RMN $^1$H (300 MHz, DMSO) δ : 7,83 (d, 1H) ; 7,63 (d, 1H) ; 3,93 (s, 2H) ; 3,59 (t, 2H) ; 3,39 (t, 2H) ; 2,91 (s, 3H) ; 2,49 (s, 3H) ; 1,41 (s, 9H).

**PREPARATION XXIII**

**Acide [2-[[(2,4-dichloro-3-méthylphényl)sulfonyl]méthylamino] éthoxy]acétique**

En opérant de façon analogue à la préparation III, au départ du composé obtenu selon la préparation XXII, on obtient le produit attendu sous forme d'une huile incolore (rendement = 100%).

RMN $^1H$ (300 MHz, DMSO) δ : 12,50 (m large, 1H) ; 7,84 (d, 1H) ; 7,63 (d, 1H) ; 4,02 (s, 2H) ; 3,61 (t, 2H) ; 3,40 (t, 2H) ; 2,91 (s, 3H) ; 2,51 (s, 3H).

**PREPARATION XXIV**

**N-(2-hydroxyéthyl)-N-méthyl-2-(trifluorométhyl)benzènesulfonamide**

En opérant de façon analogue à la préparation I, au départ du chlorure de 2-(trifluorométhyl)benzènesulfonyle, on obtient le produit attendu sous forme d'une huile jaune (rendement = 99%).
RMN $^1H$ (250 MHz, DMSO) δ: 8,02 (m, 2H) ; 7,88 (m, 2H) ; 4,84 (t, 1H) ; 3,55 (q, 2H) ; 3,30 (t, 2H) ; 2,93 (s, 3H).

**PREPARATION XXV**

**Acide [2-[[[2-(trifluorométhyl)phényl]sulfonyl]méthylamino]éthoxy]acétique, 1,1-diméthyléthyl ester**

En opérant de façon analogue à la préparation II, au départ du composé obtenu selon la préparation XXIV, on obtient le produit attendu sous forme d'une huile jaune (rendement = 67%).
RMN $^1H$ (250 MHz, DMSO) δ: 8,02 (m, 2H) ; 7,98 (m, 2H) ; 3,97 (s, 2H) ; 3,63 (t, 2H) ; 3,44 (t, 2H) ; 2,95 (s, 3H) ; 1,42 (s, 9H).

**PREPARTION XXVI**

**Acide [2-[[[2-(trifluorométhyl)phényl]sulfonyl]méthylamino]éthoxy]acétique**

En opérant de façon analogue à la préparation III, au départ du composé obtenu selon la préparation XXV, on obtient le produit attendu sous forme d'une huile incolore (rendement = 78%).
RMN $^1H$ (250 MHz, DMSO) δ: 8,02 (m, 2H) ; 7,86 (m, 2H) ; 4,01 (s, 2H) ; 3,65 (t, 2H) ; 3,45 (t, 2H) ; 2,95 (s, 3H).

**PREPARATION XXVII**

**Acide [2-[[[4-méthoxy-2-(trifluorométhyl)phényl]sulfonyl]méthylamino] éthoxy] acétique**

On prépare une suspension de 8g (60 mM) d'acide 2-(2-méthylaminoéthoxy)acétique dans 100 ml de chloroforme et 20 ml d'acétonitrile, et on ajoute, à température ambiante, 17 ml (120 mM) de triéthylamine, puis, goutte à goutte, 7,63 ml (60 mM) de chlorotriméthylsilane. Le mélange est agité à 60°C pendant 2 heures puis refroidi à température ambiante et on ajoute doucement 17 ml (120 mM) de triéthylamine puis 16,48 g (60 mM) de chlorure de 4-méthoxy-2-(trifluorométhyl)benzènesulfonyle en solution dans 60 ml de chloroforme. Le milieu réactionnel est maintenu sous agitation à 5 °C pendant 16 heures, puis concentré sous pression réduite. Le résidu est repris dans 150 ml de dichlorométhane et traité par 40 ml d'une solution d'acide chlorhydrique N. La phase organique est séparée, lavée à l'eau, puis extraite par 2 fois 120 ml de soude N. La phase aqueuse basique est séparée, lavée par 100 ml de dichlorométhane puis acidifiée par une solution d'acide chlorhydrique 5N. Le précipité formé est extrait par 2 fois 80 ml de dichlorométhane ; la phase organique obtenue est lavée à l'eau puis séchée sur sulfate de magnésium et concentrée sous pression réduite. Le résidu obtenu est cristallisé dans l'éther isopropylique, séparé et séché. On obtient ainsi 6,32 g du composé attendu sous forme d'un solide blanc (rendement = 28%).
F = 60°C

**PREPARATION XXVIII**

**Acide 4-(3-quinuclidinyl)pipérazine-1-carboxylique, 1,1-diméthyléthyl ester**

On mélange 1 g (8 mM) de quinuclidinone, 1,63 g (8,75 mM) d'ester t-butylique de l'acide 1-pipérazinecarboxylique (1-Boc-pipérazine) et 2,71 ml (9,1 mM) d'isopropoxyde de titane et on maintient ce mélange sous agitation pendant 1 heure. On ajoute ensuite 5 ml d'éthanol puis 460 mg (7,3 mM) de cyanoborohydrure de sodium et on agite ce mélange pendant 24 h à température ambiante. On ajoute 25 ml d'eau et agite pendant 15 minutes. Le précipité formé est séparé par filtration et le filtrat est concentré sous pression réduite. Le résidu est purifié par chromatographie sur gel de silice $NH_2$ en éluant à l'aide d'un mélange toluène/isopropanol (95/5 ; v/v). On obtient ainsi 1 g du composé attendu sous forme d'un solide blanc (rendement = 42 %).
F=174°C

## PREPARATION XXIX

**(3RS)-3-(1-pipérazinyl)-1-azabicyclo [2.2.2] octane ou : (3(RS)-3-(1-pipérazinyl)quinuclidine).**

On prépare une solution de 990 mg (3,35 mM) du composé obtenu selon la préparation XXVIII dans 10 ml d'acide trifluoroacétique et on agite le mélange pendant 30 minutes à 10 °C. On ajoute ensuite 30 ml de toluène et on concentre sous pression réduite. Le résidu est repris dans 25 ml de méthanol et la solution est agitée avec 20 g de résine Amberlite IRA 400 (OH-) pendant 1 heure à température ambiante. La résine est séparée par filtration, rincée avec 15 ml de méthanol et les filtrats rassemblés sont concentrés sous pression réduite. On obtient ainsi 530 mg du produit attendu sous forme d'une pâte blanche (rendement = 80 %).

RMN $^1$H (300 MHz, DMSO) δ : 2,79 (m, 1H) ; 2,67 (m, 7H) ; 2,50 (m, 2H) ; 2,22 (m, 4H) ; 1,91 (m, 1H) ; 1,85 (m, 1H) ; 1,58 (m, 2H) ; 1,32 (m, 1H) ; 1,22 (m, 1H).

## Exemple 2

**N-[2-[2-[4-[(3RS)-1-azabicyclo[2.2.2]oct-3-yl]-1-pipérazinyl]-2-oxo-éthoxy]éthyl]-N,2,4,6-tétraméthylbenzènesulfonamide, bis trifluoroacétate**

On conditionne pendant 15 mn 250 mg (0,32 mM) de résine polystyrène greffée TFP (tétrafluorophénol) dans 4 ml de DMF, puis on ajoute 4 mg (0,03 mM) de 4-(diméthylamino)pyridine, 103 mg (0,32 mM) d'acide obtenu selon la préparation V et 76 µl (0,48 mM) de diisopropylcarbodiimide. Le mélange est maintenu sous agitation pendant 18 heures, puis la résine est séparée par filtration, rincée 2 fois par 3 ml de DMF et mise en réaction avec 44 mg (0,225 mM) d'amine obtenue selon la préparation XXIX, dans 3 ml de DMF. Le mélange est agité pendant 1 heure à température ambiante et la résine est séparée par filtration et rincée par 2 fois 3 ml de DMF. Les filtrats sont rassemblés et traités par 20 mg de résine Amberlite IRA 400 (OH-), puis par 20 mg de résine greffée isocyanate, puis concentrée sous pression réduite. Le résidu est purifié par chromatographie semi-préparative (conditions analogues à celles de l'exemple 1). On obtient ainsi 16 mg du composé attendu sous forme d'un solide blanc (rendement = 8 %).
F = 78-80 °C

## Exemple 3

**N-[2-[2-[4-[(3RS)-1-azabicyclo [2.2.2] oct-3-yl]-1-pipérazinyl]-2-oxo-éthoxy] éthyl]-4-méthoxy-N,2,6-triméthyl-benzènesulfonamide**

On mélange 500 mg (1,51 mM) d'acide obtenu selon la préparation III, 319 mg (1,65 mM) de EDCI, 2,26 mg (1,65 mg) de HOAT et 233 µl de triéthylamine dans 10 ml de DMF et on maintient ce mélange réactionnel pendant 30 mn sous agitation à température ambiante. On ajoute ensuite 334 mg d'amine obtenue selon la préparation XXIX et on agite pendant 20 heures à température ambiante. Le mélange réactionnel est versé sur de l'eau glacée et extrait par le DCM. La phase organique est séchée sur sulfate de magnésium et concentrée sous pression réduite. Le résidu est purifié par chromatographie sur gel de silice greffée $NH_2$ en éluant avec un mélange toluène/isopropanol (95/5 ; v/v). On obtient ainsi 140 mg du produit attendu sous forme d'une huile incolore (rendement = 18 %).

RMN $^1$H (300 MHz, DMSO) δ : 6,80 (s, 2H) ; 4,05 (s, 2H) ; 3,80 (s, 3H) ; 3,53 (t, 2H) ; 3,41 (m, 2H) ; 3,31 (m, 2H) ; 3,22 (t, 2H) ; 2,85 (m, 1H) ; 2,70 (s, 3H) ; 2,57 (m, 5H) ; 2,53 (s, 6H) ; 2,27 (m, 4H) ; 1,90 (m, 2H) ; 1,60 (m, 2H) ; 1,35 (m, 1H) ; 1,20 (m, 1H).

## Exemple 4

**N-[2-[2-[4-[(3RS)-1-azabicydo[2.2.2]oct-3-yl]-1-pipérazinyl]-2-oxo-éthoxy]éthyl]-4-méthoxy-N,2,6-triméthyl-benzènesulfonamide, difumarate**

On prépare une solution de 137 mg (0,269 mM) du composé obtenu selon l'exemple 3 dans 3 ml de méthanol et on ajoute 62,5 mg (0,538 mM) d'acide fumarique. Le mélange est agité pendant 30 mn puis concentré sous pression réduite. Le résidu est repris dans 5 ml d'eau et lyophilisé. On obtient ainsi 200 mg du sel attendu sous forme d'une poudre blanche (rendement quantitatif). F = 86-90 °C

## Exemple 5

**N-[2-[2-[4-[(3S)-1-azabicyclo[2.2.2]oct-3-yl]-1-pipérazinyl]-2-oxo-éthoxy]éthyl]-4-méthoxy-N,2,6-triméthylbenzènesulfonamide**

On mélange 840 mg (2,53 mM) d'acide obtenu selon la préparation III dans 8 ml de toluène anhydre et 0,1 ml de DMF. On ajoute lentement 0,245 ml (2,81 mM) de chlorure d'oxalyle. Le milieu réactionnel est agité pendant une heure à température ambiante puis concentré sous pression réduite. Le résidu est repris dans 10 ml de toluène et ajouté goutte à goutte à une solution de 0,5 g (2,56 mM) de 3(S)-(1-pipérazinyl)quinuclidine et 0,39 ml (2,81 mM) de triéthylamine dans 10 ml de toluène. Le mélange réactionnel est agité pendant une heure à température ambiante puis on ajoute 2 ml d'éthanol et 10 g de gel de silice pour chromatographie. Les solvants sont chassés sous pression réduite et le produit adsorbé sur la silice est purifié par chromatographie sur gel de silice en éluant à l'aide d'un mélange acétate d'éthyle/éthanol/ammoniaque (6/3/1 ; v/v/v). Les fractions pures sont concentrées sous pression réduite, reprises en solution dans l'acétate d'éthyle, séchées sur sulfate de magnésium et concentrées. On obtient ainsi le composé attendu sous forme d'une huile incolore (rendement = 71 %).

RMN $^1$H (300 MHz, DMSO) $\delta$ : 6,80 (s, 2H) ; 4,06 (t, 2H) ; 3,80 (s, 3H) ; 3,53 (t, 2H) ; 3,41 (m, 2H) ; 3,31 (m, 2H) ; 3,22 (t, 2H) ; 2,85 (m, 1H) ; 2,69 (s, 3H) ; 2,57 (m, 5H) ; 2,53 (s, 6H) ; 2,27 (m, 4H) ; 1,90 (m, 2H) ; 1,60 (m, 2H) ; 1,35 (m, 1H) ; 1,20 (m, 1H).

## Exemple 6

**N-[2-[2-[4-[(3S)-1-azabicyclo[2.2.2]oct-3-yl]-1-pipérazinyl]-2-oxo-éthoxyléthyl]-4-méthoxy-N,2,6-triméthylbenzènesulfonamide, fumarate**

On dissout 0,84 g (1,65 mM) du composé obtenu selon l'exemple 5 dans 50 ml de méthanol et on ajoute 0,192 g (1,64 mM) d'acide fumarique. Le mélange est agité jusqu'à dissolution complète et concentré sous pression réduite. Le résidu est repris en solution dans 40 ml d'eau, la solution est filtrée et lyophilisée. On obtient ainsi 1 g du sel attendu sous forme d'une poudre blanche (rendement = 97 %).

F = 86 °C

$[\alpha]^{25}_D$ = -6,5 ° (C = 0,31 ; $CH_3OH$)

## Exemple 7

**N-[2-[2-[4-[(3R)-1-azabicyclo[2.2.2]oct-3-yl]-1-pipérazinyl]-2-oxo-éthoxy]éthyl]-4-méthoxy-N,2,6-triméthylbenzènesulfonamide, fumarate**

Le mélange racémique obtenu selon l'exemple 3 est séparé par chromatographie sur phase chirale (colonne Chiralpack AD) en éluant avec un mélange hexane/éthanol/isopropanol/acide trifluoroacétique (60/25/15/0,05). Au départ de 500 mg du mélange racémique, on obtient 260 mg d'énantiomère S et 100 mg d'énantiomère R. Chaque énantiomère est mis en solution dans le méthanol et traité par 1 g de résine Amberlite IRA 400 (OH'). La solution traitée est filtrée et concentrée sous pression réduite et les composés basiques obtenus sont salifiés par l'acide fumarique, de façon analogue à l'opération décrite pour l'exemple 6. On obtient ainsi 143 mg de l'énantiomère S et 68 mg de l'énantiomère R.

F = 84 °C

$[\alpha]^{27}_D$ = 7,2 ° (C = 0,31 ; $CH_3OH$)

## PREPARATION XXX

**Acide 4-(3-méthyl-à-azabicyclo[3.2.1]oct-3-yl)-1-pipérazine-carboxylique, 1,1-diméthyléthyl ester**

En opérant de façon analogue à la préparation XXVIII, au départ de 8-méthyl-8-azabicyclo[3.2.1]octan-3-one (tropinone), on obtient l'ester attendu sous forme d'un solide blanc (rendement = 53 %)

RMN $^1$H (300 MHz, DMSO) $\delta$ : 3,25 (m, 4H) ; 3,15 (m, 2H) ; 2,51 (m, 2H) ; 2,35 (m, 4H) ; 2,20 (s, 3H) ; 1,92 (m, 3H) ; 1,53 (m, 4H) ; 1,38 (s, 9H).

## PREPARATION XXXI

**8-méthyl-3-(1-pipérazinyl)-8-azabicyclo[3.2.1]octane**

En opérant de façon analogue à la préparation XXIX, au départ du composé obtenu selon la préparation XXX, on obtient le composé attendu sous forme d'une huile jaune (rendement = 99 %).
RMN $^1$H (300 MHz, DMSO) δ : 3,70 (m, 2H) ; 3,03 (m, 4H) ; 2,75 (m, 1H) ; 2,61 (m, 4H) ; 2,53 (s, 3H) ; 2,10 (m, 2H) ; 1,75 (m, 6H).

## PREPARATION XXXII

**Acide 4-(1-azabicyclo[2.2.2]oct-3-yl)hexahydro-1*H*-1,4-diazépine-1-carboxylique,1,1-diméthyléthyl ester**

En opérant de façon analogue à la préparation XXVIII, au départ d'ester t-butylique de l'acide 1-homopipérazinecarboxylique, on obtient le composé attendu sous forme d'une pâte incolore (rendement = 67 %).
RMN $^1$H (300 MHz, DMSO) δ : 3,34 (m, 4H) ; 2,81-2,35 (m, 11H ; 1,88 (m, 1H) ; 1,65 (m, 4H) ; 1,39 (s, 9H) ; 1,34 (m, 1H) ; 1,18 (m, 1H).

## PREPARATION XXXIII

**3-(hexahydro-1*H*-1,4-diazépine-1-yl)-1-azabicyclo[2.2.2] octane**

En opérant de façon analogue à la préparation XXIX, au départ du composé obtenu selon la préparation XXXII, on obtient le composé attendu sous forme d'une huile jaune (rendement = 98 %).
RMN $^1$H (250 MHz, DMSO) δ : 3,41 (m, 1H) ; 3,11 (m, 8H) ; 2,96 (m, 1H) ; 2,78 (m, 4H) ; 2,52 (m, 1H) ; 2,24 (m, 1H) ; 1,89 (m, 4H) ; 1,85 (m, 1H) ; 1,62 (m, 1H).
En opérant de façon analogue à l'exemple 1, au départ de dérivés de la pipérazine connus, décrits dans la littérature ou décrits ci-dessus, on prépare les exemples suivants :

## Exemple 8

**1-[[2-[[(4-méthoxy-2,6-diméthylphényl)sulfonyl]méthylamino]éthoxy]acétyl]-4-[3-(1-pyrrolidinyl)propyl]pipérazine, bis-trifluoroacétate**

Rendement = 90 % (pâte incolore).
RMN $^1$H (300 MHz, $CD_3CN$) δ : 6,74 (s, 2H) ; 4,12 (s, 2H) ; 3,81 (s, 3H) ; 3,78 (m, 4H) ; 3,62 (t, 2H) ; 3,34 (t, 2H) ; 3,27 (m, 12H) ; 2,73 (s, 3H) ; 2,57 (s, 6H) ; 2,22 (m, 2H) ; 2,03 (m,4H).

## Exemple 9

**1-[[2-[[(4-méthoxy-2,6-diméthylphényl)sulfonyl]méthylamino]-éthoxy] acétyl]-4-[2-(4-morpholinyl)éthyl]pipérazine, bis-trifluoroacétate**

Rendement = 51 % (pâte incolore)
RMN $^1$H (300 MHz, $CD_3CN$) δ : 6,75 (s, 2H) ; 4,11 (s, 2H) ; 3,92 (m, 4H) ; 3,81 (s, 3H) ; 3,76 (m, 4H) ; 3,61 (t, 2H) ; 3,48 (m, 4H) ; 3,35 (t, 2H) ; 3,27 (m, 8H) ; 2,72 (s, 3H) ; 2,57 (s, 6H).

## Exemple 10

**1-[[2-[[(4-méthoxy-2,6-diméthylphényl)sulfonyl]méthylamino]-éthoxy]acétyl]-4-[2-(1-pipéridinyl)éthyl]pipérazine, bis-trifluoroacétate**

Rendement = 92 % (pâte incolore)
RMN $^1$H (300 MHz, $CD_3CN$) δ : 6,74 (s, 2H) ; 4,10 (s, 2H) ; 3,81 (s, 3H) ; 3,81 (m, 4H) ; 3,61 (t, 2H) ; 3,40 (m, 2H) ; 3,34 (m, 4H) ; 3,24 (m, 4H) ; 3,09 (m, 4H) ; 2,74 (s, 3H) ; 2,57 (s, 6H) ; 1,90 (m, 4H) ; 1,64 (m, 2H).

**Exemple 11**

**1-[[2-[[(4-méthoxy-2,6-diméthylphényl)sulfonyl]méthylamino]-éthoxy]acétyl]-4-[3-(1-pipéridinyl)propyl]pipérazine, bis-trifluoroacétate**

Rendement = 85 % (pâte incolore).

RMN $^1H$ (300 MHz, $CD_3CN$) δ : 6,74 (s, 2H) ; 4,10 (s, 2H) ; 3,96 (s, 3H) ; 3,80 (m, 2H) ; 3,60 (t, 2H) ; 3,50 (m, 4H) ; 3,30 (t, 2H) ; 3,15 (m, 2H) ; 3,10 (m, 4H) ; 2,80 (m, 4H) ; 2,75 (s; 3H) ; 2,55 (s, 6H) ; 2,20 (m, 2H) ; 1,93 (m, 5H) ; 1,37 (m, 1H).

**Exemple 12**

**1-[[2-[[(4-méthoxy-2,6-diméthylphényl)sulfonyl]méthylamino] éthoxy]acétyl]-4-[3-(diméthylamino)propyl]pipérazine, bis-trifluoroacétate**

Rendement = 74% (pâte incolore)

RMN $^1H$ (300 MHz, CD3CN) δ : 6,74 (s, 2H) ; 4,11 (s, 2H) ; 3,81 (s, 3H) ; 3,78 (m, 4H) ; 3,63 (t, 2H) ; 3,31 (t, 2H) ; 3,22 (m, 4H) ; 3,15 (m, 4H) ; 2,81 (s, 6H) ; 2,74 (s, 3H) ; 2,57 (s, 6H) ; 2,23 (m, 2H).

En opérant de façon analogue aux exemples 3 et 4, au départ de dérivés de pipérazine ou d'homopipérazine décrits ci-dessus, on prépare les exemples suivants :

**Exemple 13**

**4-méthoxy-N,2,6-triméthyl-N-[2-[2-[4-(1-méthyl-4-pipéridinyl)-1-pipérazinyl]-2-oxoéthoxy] éthyl]benzènesulfonamide, bis trifluoroacétate**

Rendement = 74 % (pâte incolore)

RMN $^1H$ (300 MHz, $CD_3CN$) δ : 6,74 (s, 2H) ; 4,11 (s, 2H) ; 3,81 (s, 3H) ; 3,80 (m, 4H) ; 3,77 (d, 2H) ; 3,64 (m, 2H) ; 3,55 (m, 1H) ; 3,34 (t, 2H) ; 3,26 (m, 4H) ; 3,01 (m, 2H) ; 2,79 (s, 3H) ; 2,74 (s, 3H) ; 2,57 (s, 6H) ; 2,29 (m, 4H).

**Exemple 14**

**4-méthoxy-N,2,6-triméthyl-N-[2-[2-[4-(8-méthyl-8-azabicyclo[3.2.1]-oct-3-yl)-1-pipérazinyl]-2-oxoéthoxy]éthyl] benzènesulfonamide, fumarate**

Rendement = 62 % (solide blanc)
F = 88-90 °C

**Exemple 15**

**1-(1-azabicyclo [2.2.2] oct-3-yl)hexahydro-4-[[2-[[(4-méthoxy-2,6-diméthylphényl)sulfonyl]méthylamino] éthoxylacét-yl]-1*H*-1,4-diazépine, fumarate**

Rendement = 47 % (solide blanc)
F=90°C

**PREPARATION XXXIV**

**Acide 4-[1-(1,1-diméthyléthyl)-4-pipéridinyl]-1-pipérazinecarboxylique, phénylméthyl ester**

En opérant de façon analogue à la préparation XXVIII, au départ de 1-(1,1-diméthyléthyl)-4-pipéridinone et de l'ester benzylique de l'acide 1-pipérazinecarboxylique, on obtient le produit attendu sous forme d'un solide blanc (rendement = 56%).
F = 70-72 °C

## PREPARATION XXXV

### 1-[1-(1,1-diméthyléthyl)-4-pipéridinyl]pipérazine

On prépare une solution de 570 mg (1,59 mM) du composé obtenu selon la préparation XXXIV dans 20 ml de méthanol et on ajoute 114 mg de charbon palladié à 10%. Le mélange est agité sous atmosphère d'hydrogène pendant 2 heures à température ambiante et à pression atmosphérique. Le catalyseur est éliminé par filtration et le filtrat est concentré sous pression réduite. Le produit brut est purifié par chromatographie sur gel de silice en éluant à l'aide d'un mélange dichlorométhane/méthanol/ammoniaque (90/10/1 ; v/v/v). On obtient ainsi 270 mg du composé attendu sous forme d'une poudre blanche (rendement = 75%).
F = 106˚C

## PREPARATION XXXVI

### Acide 4-(9-méthyl-9-azabicyclo[3.3.1]non-3-yl)-1-pipérazinecarboxylique, phénylméthyl ester

En opérant de façon analogue à la préparation XXVIII, au départ de 9-méthyl-9-azabicyclo[3.3.1]nonan-3-one et de l'ester benzylique de l'acide 1-pipérazinecarboxylique, on obtient le produit attendu sous forme d'un solide jaune pâle (rendement = 18%).
F= 74 ˚C

## PREPARATION XXXVII

### 1-(9-méthyl-9-azabicyclo[3.3.1]non-3-yl)pipérazine

En opérant de façon analogue à la préparation XXXV, au départ du composé obtenu selon la préparation XXXVI, on obtient le produit attendu sous forme d'une huile jaune (rendement = 100%).

RMN $^1$H (300 MHz, D2O) δ : 3,74 (m, 2H) ; 3,59 (m, 1H) ; 3,43 (m, 4H) ; 3,15 (m, 4H) ; 3,01 et 2,97 (2s, 3H) ; 2,46 (dd, 1H) 2,30 (m, 2H) ; 2,10 (m, 4H) ; 1,83 (m, 3H).

## PREPARATION XXXVIII

### Acide 4-(1,2,2,6,6-pentaméthyl-4-pipéridinyl)-1-pipérazinecarboxylique, phénylméthyl ester

En opérant de façon analogue à la préparation XXVIII, au départ de 1,2,2,6,6-pentaméthyl-4-pipéridinone et de l'ester benzylique de l'acide 1-pipérazinecarboxylique, on obtient le produit attendu sous forme d'une huile incolore (rendement = 52%).

RMN $^1$H (300 MHz, DMSO) δ: 7,36 (m, 5H) ; 5,07 (s, 2H) ; 3,37 (m, 4H) ; 2,85 (m, 1H) ; 2,50 (m, 7H) ; 1,80 (m, 2H) ; 1,48 (m, 2H) ; 1,28 (s, 6H) ; 1,21 (s, 6H).

## PREPARATION XXXIX

### 1-(1,2,2,6,6-pentaméthyl-4-pipéridinyl)pipérazine

En opérant de façon analogue à la préparation XXXV, au départ du composé obtenu selon la préparation XXXVIII, on obtient le produit attendu sous forme d'un solide blanc (rendement = 35%).
F = 65˚C

## PREPARATION XL

### Acide 4-[1-(1-méthyléthyl)-4-pipéridinyl]-1-pipérazinecarboxylique, 1,1-diméthyléthyl ester

En opérant de façon analogue à la préparation XXVIII, au départ de 1-(1-méthyléthyl)-4-pipéridinone, on obtient le produit attendu sous forme d'un solide jaune (rendement = 31 %).
F = 53 ˚C

**PREPARATION XLI**

**1-[1-(1-méthylétlxyl)-4-pipéridinyl]pipérazine, trichlorlaydrate**

On prépare une solution de 247 mg (0,79 mM) du composé obtenu selon la préparation XL dans 1 ml de méthanol, et on ajoute 15 ml d'une solution 2,3 N de chlorure d'hydrogène dans l'acétate d'éthyle. Le mélange est agité pendant 4 heures à température ambiante. Le mélange réactionnel est concentré sous pression réduite. On obtient ainsi 239 mg du composé attendu sous forme d'une poudre blanche.(rendement = 75%).
F = 262˚C

**PREPARATION XLII**

**Acide 4-(1-cyclopropyl-4-pipéridinyl)-1-pipérazinecarboxylique, phénylméthyl ester**

En opérant de façon analogue à la préparation XXXIV, au départ de 1-cyclopropyl-4-pipéridinone, on obtient le produit attendu sous forme d'un solide blanc (rendement = 67%).
F = 88 ˚C

**PREPARATION XLIII**

**1-(1-cyclopropyl-4-pipéridinyl)pipérazine**

En opérant de façon analogue à la préparation XXXV, au départ du composé obtenu selon la préparation XLII, on obtient le produit attendu sous forme d'un solide blanc (rendement = 92%).
F = 58˚C

**PREPARATION XLIV**

**Acide 4-(1-méthyl-4-pipéridinyl)hexahydro-1*H*-1,4-diazépine-1-carboxylique, 1,1-diméthyléthyl ester**

En opérant de façon analogue à la préparation XXXII, au départ de 1-méthyl-4-pipéridinone, on obtient le produit attendu sous forme d'une huile jaune (rendement = 78%).

RMN $^1$H (250 MHz, CDCl3) δ : 3,43 (m, 4H) ; 3,10 (dm, 2H) ; 2,70 (m, 4H) ; 2,61 (m, 1H) ; 2,44 (s, 3H) ; 2,28 (m, 2H) ; 1,86 (m, 6H) ; 1,45 (s, 9H).

**PREPARATION XLV**

**1-(1-méthyl-4-pipéridinyl)hexahydro-1*H*-1,4-diazépine, trichlorhydrate**

En opérant de façon analogue à la préparation XLI, au départ du composé obtenu selon la préparation XLIV, on obtient le produit attendu sous forme d'un solide beige (rendement = 99%).
F = 186˚C

**PREPARATION XLVI**

**Acide 4-(8-cyclopropyl-8-azabicyclo[3.2.1]oct-3-yl)-1-pipérazinecarboxylique, 1,1-diméthyléthyl ester**

En opérant de façon analogue à la préparation XXVIII, au départ de 8-cyclopropyl-8-azabicyclo[3.2.1]octan-3-one, on obtient l'ester attendu sous forme d'une pâte blanche (rendement = 45 %).

RMN $^1$H (300 MHz, DMSO) δ : 3,24 (m, 6H) ; 2,50 (m, 5H) ; 2,34 (t, 4 H) ; 1,87 (m, 3H) ; 1,48 (dd, 6H) ; 1,38 (s, 9H) ; 0,36 (m, 2H) ; 0,25 (m, 2H).

**PREPARATION XLVII**

**8-cyclopropyl-3-(1-pipérazinyl)-8-azabicyclo [3.2.1] octane**

En opérant de façon analogue à la préparation XXIX, au départ du composé obtenu selon la préparation XLVI, on obtient le composé attendu sous forme d'un solide blanc (rendement = 95 %).

RMN $^1H$ (250 MHz, CDCl3) δ : 3,37 (m, 2H) ; 2,94 (t, 2H) ; 2,58 (m, 4H) ; 2,51 (m, 1H) ; 1,96 (m, 3H) ; 1,74 (t, 2H) ; 1,54 (m, 4H) ; 0,43 (d, 4H).

**PREPARATION XLVIII**

**Acide 4-[1-[(1,1-diméthyléthoxy)carbonyl)-4-pipéridinyl]-1-pipérazinecarboxylique, phénylméthyl ester**

En opérant de façon analogue à la préparation XXXIV, au départ de l'ester t-butylique de l'acide 4-oxo-1-pipéridinecarboxylique, on obtient le produit attendu sous forme d'une huile jaune (rendement = 30%).

RMN $^1H$ (250 MHz, DMSO) δ : 7,35 (m, 5H) ; 5,06 (s, 2H) ; 3,93 (d, 2H) ; 3,36 (m, 4H) ; 2,68 (t, 2H) ; 2,44 (t, 4H) ; 2,37 (m, 1H) ; 1,66 (m, 2H) ; 1,38 (s, 9H) ; 1,26 (m, 2H).

**PREPARATION IL**

**Acide 4-(4-pipéridinyl)-1-pipérazinecarboxylique, phénylméthyl ester**

En opérant de façon analogue à la préparation XXIX, au départ du composé obtenu selon la préparation XLVIII, on obtient le produit attendu sous forme d'une huile jaune (rendement = 100%).

RMN $^1H$ (250 MHz, DMSO) δ : 8,91 (m large, h) ; 8,63 (m large, 1H) ; 7,36 (m, 5H) ; 5,11 (s, 2H) ; 3,80 (m, 2H) ; 3,45 (m, 4H) ; 3,22 (m, 5H) ; 2,92 (q, 2H) ; 2,20 (d, 2H) ; 1,82 (dq, 2H).

**PREPARATION L**

**Acide 4-(1-éthyl-4-pipéridinyl)-1-pipérazinecarboxylique, phénylméthyl ester**

En opérant de façon analogue à la préparation XXXIV, au départ d'acétaldéhyde et du composé obtenu selon la préparation IL, on obtient le produit attendu sous forme d'une huile incolore (rendement = 72%).

RMN $^1H$ (250 MHz, DMSO) δ : 7,34 (m, 5H) ; 5,07 (s, 2H) ; 3,90 (m, 7H) ; 3,02 (q, 2H) ; 2,84 (m, 2H) ; 2,45 (t, 4H) ; 1,91 (m, 2H) ; 1,63 (m, 2H) ; 1,19 (t, 3H).

**PREPARATION LI**

**1-(1-éthyl-4-pipéridinyl)pipérazine**

En opérant de façon analogue à la préparation XXXV, au départ du composé obtenu selon la préparation L, on obtient le produit attendu sous forme d'une huile jaune (rendement = 71%).

RMN $^1H$ (250 MHz, DMSO) δ : 2,90 (m, 6H) ; 2,52 (m, 4H) ; 2,30 (q, 2H) ; 2,17 (tt, 1H) ; 1,85 (dt, 2H) ; 1,69 (m, 2H) ; 1,38 (dq, 2H) ; 0,97 (t, 3H).

**PREPARATION LII**

**Acide 4-[8-[(1,1-diméthyléthoxy)carbonyl]-8-azabicyclo[3.2.1]oct-3-yl]-1-pipérazinecarboxylique, phénylméthyl ester**

En opérant de façon analogue à la préparation XXVIII, au départ de l'ester t-butylique de l'acide 3-oxo-8-azabicyclo[3.2.1]octane-8-carboxylique, on obtient l'ester attendu sous forme d'un solide blanc (rendement = 40 %).

RMN $^1H$ (250 MHz, $CD_3CN$) δ : 7,33 (m, 5H) ; 5,07 (s, 2H) ; 4,13 (m, 2H) ; 3,37 (t, 4H) ; 2,80 (hep, 1H) ; 2,43 (t, 4H) ; 1,87 (m, 2H) ; 1,62 (m, 6H) ; 1,42 (s, 9H).

**PREPARATION LIII**

**Acide 4-(8-azabicyclo[3.2.1]oct-3-yl)-1-pipérazinecarboxylique, phénylméthyl ester, chlorhydrate**

En opérant de façon analogue à la préparation XLI, au départ du composé obtenu selon la préparation LII, on obtient le produit attendu sous forme d'une huile incolore (rendement = 96 %).

RMN $^1H$ (250 MHz, DMSO) δ : 11,78 (m large, 1H) ; 9,45 (s, 2H) ; 7,38 (m, 5H) ; 5,11 (s, 3H) ; 4,09 (m, 4H) ; 3,95 (m, 1H) ; 3,43 (m, 4H) ; 3,07 (m, 2H) ; 2,22 (m, 4H) ; 1,92 (m, 4H).

**PREPARATION LIV**

**Acide 4-(8-éthyl-8-azabicyclo[3.2.1]oct-3-yl)-1-pipérazinecarboxylique, phénylméthyl ester**

En opérant de façon analogue à la préparation XXXIV, au départ d'acétaldéhyde et du composé obtenu selon la préparation LIII, on obtient le produit attendu sous forme d'une huile incolore (rendement = 99 %).

RMN $^1$H (250 MHz, CD3CN) δ : 7,33 (m, 5H) ; 5,07 (s, 2H) ; 3,39 (m, 6H) ; 2,63 (m, 1H) ; 2,56 (q, 2H) ; 2,42 (t, 4H) ; 1,93 (m, 2H) ; 1,61 (m, 6H) ; 1,06 (t, 3H).

**PREPARATION LV**

**1-(8-éthyl-8-azabicyclo[3.2.1]oct-3-yl)pipérazine**

En opérant de façon analogue à la préparation XXXV, au départ du composé obtenu selon la préparation LIV, on obtient le produit attendu sous forme d'une huile incolore (rendement = 78 %).

RMN $^1$H (300 MHz, CDC13) δ : 3,57 (m, 2H) ; 2,94 (t, 2H) ; 2,71 (q, 2H) ; 2,59 (m, 4H) ; 2,52 (m, 1H) ; 1,99 (m, 4H) ; 1,64 (m, 4H).

**PREPARATION LVI**

**Acide 4-[8-(1-méthyléthyl)-8-azabicyclo[3.2.1]oct-3-yl]-1-pipérazinecarboxylique, phénylméthyl ester**

En opérant de façon analogue à la préparation XXXIV, au départ d'acétone et du composé obtenu selon la préparation LIII, on obtient le produit attendu sous forme d'une huile incolore (rendement = 77 %).

RMN $^1$H (250 MHz, CDCl3) δ : 7,32 (m, 5H) ; 5,12 (s, 2H) ; 3,64 (m, 2H) ; 3,49 (t, 4.H) ; 2,92 (quin, 1H) ; 2,59 (hep, 1H) ; 2,47 (m, 4H) ; 1,99 (m, 2H) ; 1,82 (dt, 2H) ; 1,63 (m, 2H) ; 1,48 (m, 2H) ; 1,14 (d, 6H).

**PREPARATION LVII**

**1-[8-(1-méthyléthyl)-8-azabicyclo[3.2.1]oct-3-yl]pipérazine**

En opérant de façon analogue à la préparation XXXV, au départ du composé obtenu selon la préparation LVI, on obtient le produit attendu sous forme d'une huile incolore (rendement = 82 %).

RMN $^1$H (250 MHz, CDC13) δ : 3,79 (m, 2H) ; 3,05 (quin, 1H) ; 2,98 (m, 4H) ; 2,71 (hep, 1H) ; 2,61 (m, 4H) ; 2,13 (m, 2H) ; 1,95(m, 2H) ; 1,65 (m, 4H) ; 1,29 (d, 6H).

**PREPARATION LVIII**

**Hexahydro-1-méthyl-4-(1-oxo-2-propényl)-1*H*-1,4-diazépine**

On prépare une solution de 20 g (175 mM) de N-méthylhomopipérazine dans 100 ml de dichlorométhane, à laquelle on ajoute goutte à goutte, à 0 °C, une solution de 15,85 g (175 mM) de chlorure d'acryloyle dans 20 ml de dichlorométhane. Le milieu réactionnel est agité pendant 1 heure à 0°C, puis pendant 2 heures à température ambiante, puis hydrolysé par une solution de 12 g de carbonate de sodium dans 20 ml d'eau. Le mélange est décanté, la phase organique est lavée une fois à l'eau puis séchée sur sulfate de magnésium et concentrée sous pression réduite. On obtient ainsi 23 g du composé attendu, sous forme d'une huile orangée, utilisée sans autre purification à l'étape suivante.

**PREPARATION LIX**

**Acide 4-[3-(hexahydro-4-méthyl-1*H*-1,4-diazépin-1-yl)-3-oxopropyl]-1-pipérazinecarboxylique, phénylméthyl ester**

On prépare une solution de 7g (41 mM) du composé obtenu selon la préparation LVIII dans 100 ml de toluène, à laquelle on ajoute 11 g (50 mM) d'ester benzylique de l'acide 1-pipérazinecarboxylique. Le milieu réactionnel est agité pendant 16 heures à reflux du solvant, puis concentré sous pression réduite. Le résidu est repris dans de l'acétate d'éthyle et la phase organique est lavée une fois à l'eau puis séchée sur sulfate de magnésium et concentrée sous pression réduite. Le produit brut est purifié par chromatographie sur gel de silice en éluant à l'aide d'un mélange toluène/isopropanol/ammoniaque (80/20/1 ; v/v/v). On obtient ainsi 4,1 g du composé attendu, sous forme d'une huile jaune

(rendement = 26 %).

RMN $^1H$ (250 MHz, DMSO) δ : 7,36 (m, 5H) ; 5,06 (s, 2H) ; 3,47 (m, 4H) ; 3,37 (m, 4H) ; 2,50 (m, 8H) ; 2,35 (m, 4H) ; 2,24 et 2,21 (2s, 3H) ; 1,75 (m, 2H).

**PREPARATION LX**

**1-[3-(hexahydro-4-méthyl-1*H*-1,4-diazépin-1-yl)-3-oxopropyl]pipérazine**

En opérant de façon analogue à la préparation XXXV, au départ du composé obtenu selon la préparation LIX, on obtient le produit attendu sous forme d'une huile incolore (rendement = 65 %).

RMN $^1H$ (250 MHz, DMSO) δ : 3,48 (m, 4H) ; 2,67 (t, 4H) ; 2,48 (m, 8H) ; 2,30 (t, 4H) ; 2,25 et 2,22 (2s, 3H) ; 1,73 (m, 2H).

**PREPARATION LXI**

**1-[3-(hexahydro-4-méthyl-1*H*-1,4-diazépin-1-yl)propyl]pipérazine**

On prépare une suspension de 120 mg (3,1 mM) d'hydrure de lithium-aluminium dans 3 ml de tétrahydrofurane (THF) et on ajoute une solution de 800 mg (3,1 mM) du composé obtenu selon la préparation LX dans 10 ml de THF. Le mélange réactionnel est chauffé à doux reflux du solvant pendant 4 heures, puis refroidi à température ambiante. On ajoute 200 mg de sel de Glauber au milieu réactionnel puis, après environ 15 min, 50 ml d'acétate d'éthyle. La suspension obtenue est filtrée et le filtrat est concentré sous pression réduite. Le produit brut est purifié par chromatographie sur gel de silice en éluant à l'aide d'un mélange dichlorométhane/méthanol/ammoniaque (80/20/10 ; v/v/v). On obtient ainsi le produit attendu sous forme d'une huile incolore (rendement = 34 %).

RMN $^1H$ (250 MHz, DMSO) S : 2,64 (t, 4H) ; 2,57 (m, 4H) ; 2,49 (m, 4H) ; 2,38 (t, 2H) ; 2,23 (m, 6H) ; 2,21 (s, 3H) ; 1,66 (quin, 2H) ; 1,52 (quin, 2H).

**PREPARATION LXII**

**Acide 4-[2-(hexahydro-4-méthyl-1*H*-1,4-diazépin-1-yl)-2-oxoéthyl]-1-piperazinecarboxylique, phénylméthyl ester**

On prépare une solution de 10 g (87,5 mM) de N-méthylhomopipérazine dans 200 ml de dichlorométhane, à laquelle on ajoute 36,6 ml de triéthylamine. Le mélange est refroidi à -78 °C et on ajoute goutte à goutte 6,97 ml de chlorure de chloracétyle. Le milieu réactionnel est agité pendant 1,5 heure à -78 °C, puis on ajoute une solution de 19,3 g (87,6 mM) d'ester benzylique de l'acide 1-pipérazinecarboxylique dans 10 ml de dichlorométhane. On laisse ensuite le mélange revenir à température ambiante, et on agite pendant 15 heures. Le milieu est ensuite hydrolysé par une solution de carbonate de sodium. Le mélange est décanté, la phase organique est lavée une fois à l'eau puis séchée sur sulfate de magnésium et concentrée sous pression réduite. Le produit brut est purifié par chromatographie sur gel de silice en éluant à l'aide d'un mélange dichlorométhane/méthanol/ammoniaque (90/10/5 ; v/v/v). On obtient ainsi le produit attendu sous forme d'une huile jaune (rendement = 43 %).

RMN $^1H$ (250 MHz, DMSO) δ : 7,36 (m, 5H) ; 5,07 (s, 2H) ; 3,55 (m, 2H) ; 3,44 (m, 2H) ; 3,38 (m, 4H) ; 3,16 (d, 2H) ; 2,60 (m, 2H) ; 2,44 (m, 6H) ; 2,24 et 2,22 (2s, 3H) ; 1,76 (m, 2H).

**PREPARATION LXIII**

**1-[2-(hexahydro-4-méthyl-1*H*-1,4-diazépin-1-yl)-2-oxoéthyl]pipérazine**

En opérant de façon analogue à la préparation XXXV, au départ du composé obtenu selon la préparation LXII, on obtient le produit attendu sous forme d'une huile incolore (rendement = 60 %).

RMN $^1H$ (250 MHz, DMSO) δ : 3,56 (m, 2H) ; 3,44 (m, 2H) ; 3,10 (d, 2H) ; 2,74 (dd, 4H) ; 2,61 (m, 1H2.44 (m, 4H) ; 2,39 (m, 3H) ; 2,25 et 2,22 (2s, 3H) ; 1,78 (m, 2H).

**PREPARATION LXIV**

**1-[2-(hexahydro-4-méthyl-1*H*-1,4-diazépin-1-yl)éthyl]pipérazine**

En opérant de façon analogue à la préparation LXI, au départ du composé obtenu selon la préparation LXIII,

on obtient le produit attendu sous forme d'une huile jaune (rendement = 21 %).

RMN $^1H$ (300 MHz, DMSO) $\delta$ : 2,63 (m, 8H) ; 2,49 (m, 5H) ; 2,28 (m, 7H) ; 2,21 (s, 3H) ; 1,66 (quin, 2H).

**PREPARATION LXV**

**1-(1-oxo-2-propényl)azétidine**

En opérant de façon analogue à la préparation LVIII, au départ d'azétidine, on obtient le produit attendu sous forme d'une huile jaune qui est remise en réaction dans la préparation suivante sans purification.

**PREPARATION LXVI**

**Acide 4-[3-(1-azétidinyl)-3-oxopropyl]-1-pipérazinecarboxylique, phénylméthyl ester**

En opérant de façon analogue à la préparation LIX, au départ du composé obtenu selon la préparation LXV, on obtient le produit attendu sous forme d'une huile jaune (rendement = 14 %).

RMN $^1H$ (250 MHz, DMSO) $\delta$ : 7,34 (m, 5H) ; 5,06 (s, 2H) ; 4,09 (t, 2H) ; 3,80 (t, 2H) ; 3,35 (m, 4H) ; 2,48 (t, 2H) ; 2,33 (t, 4H) ; 2,16 (m, 4H).

**PREPARATION LXVII**

**1-[3-(1-azétidinyl)-3-oxopropyl]pipérazine**

En opérant de façon analogue à la préparation XXXV, au départ du composé obtenu selon la préparation LXVI, on obtient le produit attendu sous forme d'un solide jaune (rendement = 100 %).
F = 50˚C

**PREPARATION LXVIII**

**1-[3-(1-azétidinyl)propyl]pipérazine**

En opérant de façon analogue à la préparation LXI, au départ du composé obtenu selon la préparation LXVII, on obtient le produit attendu sous forme d'une huile jaune (rendement = 30 %).

RMN $^1H$ (300 MHz, DMSO) $\delta$ : 3,19 (m large, 1H) ; 3,02 (t, 4H) ; 2,66 (t, 4H) ; 2,25 (m, 8H) ; 1,93 (quin, 2H) ; 1,36 (quin, 2H).

**PREPARATION LXIX**

**$\alpha$-[(diméthylamino)méthyl]-4-pipéridineméthanol**

On dissout 20 g (83,6 mM) de dichlorhydrate de 2-[(diméthylamino)méthyl]-4-pyridineméthanol dans 200 ml de méthanol à 50 ˚C. On ajoute ensuite, sous atmosphère d'azote, 2 g d'oxyde de platine et on agite le mélange sous atmosphère d'hydrogène, à 50 ˚C, sous une pression de 300 hPa (3 bars), pendant 7 heures. Ce catalyseur est ensuite éliminé par filtration et le filtrat est concentré sous pression réduite. Le résidu solide est repris avec 10 ml de soude 10N et 150 ml de chloroforme. La phase organique est séparée et lavée avec une solution de chlorure de sodium, séchée sur sulfate de magnésium et concentrée sous pression réduite. On obtient ainsi le produit attendu sous forme d'une huile qui cristallise (rendement = 87 %).
F = 98 ˚C

**PREPARATION LXX**

**4-(1-pyrrolidinylméthyl)pipéridine**

En opérant de façon analogue à la préparation LXIX, au départ de 4-(1-pyrrolidinylméthyl)pyridine, on obtient le produit attendu sous forme d'une huile jaune.

**PREPARATION LXXI**

**Acide 4-[(4-(1-méthyléthyl)-1-pipérazinyl]-1-pipéridinecarboxylique, phénylméthyl ester**

On prépare un mélange de 827 mg (3,54 mM) d'ester benzylique de l'acide 4-oxo-1-pipéridinecarboxylique, 500 mg (3,9 mM) de N-isopropylpipérazine et 1,2 g (4,25 mM) d'isopropylate de titane dans 10 ml de méthanol et on ajoute sous agitation et à température ambiante, 148 mg de borohydrure de sodium. Le mélange réactionnel est maintenu sous agitation pendant 16 heures, puis dilué avec 20 ml d'eau. La suspension obtenue est filtrée et le précipité est lavé avec 30 ml d'acétate d'éthyle que l'on utilise ensuite pour extraire le filtrat. La phase organique obtenue est lavée à l'eau puis séchée sur sulfate de magnésium et concentrée sous pression réduite. L'huile jaune obtenue est purifiée par chromatographie sur gel de silice en éluant à l'aide d'un mélange dichlorométhane/méthanol (9/1 ; v/v). On obtient ainsi 235 mg du produit attendu sous forme d'un solide blanc (rendement = 19%).
F = 55°C

**PREPARATION LXXII**

**4-[4-(1-méthyléthyl)-1-pipérazinyl]pipéridine**

En opérant de façon analogue à la préparation XXXV, au départ de l'ester obtenu suivant la préparation LXXI, on obtient le produit attendu sous forme d'un solide blanc (rendement = 98%).
F=65°C

**PREPARATION LXXIII**

**Acide 4-[(4-méthyl-1-pipérazinyl)méthyl]-1-pipéridinecarboxylique, phénylméthyl ester**

En opérant de façon analogue à la préparation LXXI, au départ de N-méthylpipérazine et de l'ester benzylique de l'acide 4-formyl-1-pipéridinecarboxylique, on obtient le produit attendu sous forme d'une huile incolore (rendement = 52%)

RMN $^1$H (250 MHz, DMSO) δ : 7,34 (m, 5H) ; 5,05 (s, 2H) ; 3,98 (m, 2H) ; 2,77 (m, 2H) ; 2,29 (m, 8H) ; 2,19 (s, 3H) ; 2,08 (d, 2H) ; 1,67 (m, 3H) ; 0,95 (m, 2H).

**PREPARATION LXXIV**

**4-[(4-méthyl-1-piperazinyl)méthyl]pipéridine**

En opérant de façon analogue à la préparation XXXV, au départ de l'ester obtenu suivant la préparation LXXIII, on obtient le produit attendu sous forme d'une huile incolore (rendement = 77%).

RMN $^1$H (250 MHz, DMSO) δ : 2,89 (m, 2H) ; 2,51 (m, 2H) ; 2,28 (m, 8H) ; 2,12 (s, 3H) ; 2,05 (d, 2H) ; 1,55 (m, 3H) ; 1,02 (m, 2H).

**PREPARATION LXXV**

**Acide 4-[(1-azétidinyl)méthyl]-1-pipéridinecarboxylique, phénylméthyl ester**

En opérant de façon analogue à la préparation LXXIII, au départ d'azétidine, on obtient le produit attendu sous forme d'une huile incolore (rendement = 69%).

RMN $^1$H (300 MHz, DMSO) δ : 7,34 (m, 5H) ; 5,04 (s, 2H) ; 3,95 (m, 2H) ; 3,06 (t, 4H) ; 2,75 (m, 2H) ; 2,18 (d, 2H) ; 1,94 (quin, 2H) ; 1,61 (m, 2H) ; 1,38 (m, 1H) ; 0,94 (m, 2H).

**PREPARATION LXXVI**

**4-[(1-azétidinyl)méthyl]pipéridine**

En opérant de façon analogue à la préparation XXXV, au départ de l'ester obtenu suivant la préparation LXXV, on obtient le produit attendu sous forme d'une huile incolore (rendement = 93%).

RMN $^1$H (250 MHz, DMSO) δ : 3,06 (t, 4H) ; 2,92 (m, 2H) ; 2,50 (m, 2H) ; 2,15 (d, 2H) ; 1,91 (quin, 2H) ; 1,59 (m, 2H) ; 1,30 (m, 1H) ; 1,00 (m, 2H).

## PREPARATION LXXVII

### Acide 4-[(diméthylamino)méthyl]-1-pipéridinecarboxylique, phénylméthyl ester

En opérant de façon analogue à la préparation LXXIII, au départ de diméthylamine, on obtient le produit attendu sous forme d'une huile incolore (rendement = 78%).

RMN $^1$H (300 MHz, DMSO) δ : 7,35 (m, 5H) ; 5,05 (s, 2H) ; 3,97 (m, 2H) ; 2,79 (m, 2H) ; 2,09 (s, 6H) ; 2,02 (d, 2H) ; 1,68 (d, 2H) ; 1,61 (m, 1H) ; 0,96 (m, 2H).

## PREPARATION LXXVIII

### 4-[(diméthylamino)méthyl]pipéridine

En opérant de façon analogue à la préparation XXXV, au départ de l'ester obtenu suivant la préparation LXXVII, on obtient le produit attendu sous forme d'une huile incolore (rendement = 90%).

RMN $^1$H (300 MHz, DMSO) δ : 2,91 (m, 2H) ; 2,43 (m, 2H) ; 2,08 (s, 6H) ; 2,01 (d, 2H) ; 1,59 (m, 2H) ; 1,49 (m, 1H) ; 0,96 (dq, 2H).

## PREPARATION LXXIX

### Acide 4-[(4-éthyl-1-pipérazinyl)méthyl]-1-pipéridinecarboxylique, 1,1-diméthyléthyl ester

En opérant de façon analogue à la préparation LXXIII, au départ de N-éthylpipérazine et de l'ester t-butylique de l'acide 4-formyl-1-pipéridinecarboxylique, on obtient le produit attendu sous forme d'une huile incolore (rendement = 78%).

RMN $^1$H (300 MHz, DMSO) δ : 3,90 (m, 2H) ; 2,67 (m, 2H) ; 2,29 (m, 10H) ; 2,09 (d, 2H) ; 1,63 (m, 3H) ; 1,38 (s, 9H) ; 0,96 (t, 3H) ; 0,85 (m, 2H).

## PREPARATION LXXX

### 4-[(4-éthyl-1-pipérazinyl)méthyl]pipéridine

En opérant de façon analogue à la préparation XXIX, au départ de l'ester obtenu suivant la préparation LXXIX, on obtient le produit attendu sous forme d'une huile incolore (rendement = 76%).

RMN $^1$H (250 MHz, DMSO) δ : 2,95 (m, 2H) ; 2,51 (m, 2H) ; 2,42 (m, 10H) ; 2,05 (d, 2H) ; 1,54 (m, 3H) ; 1,03 (m, 2H) ; 0,99 (t, 3H).

## PREPARATION LXXXI

### Acide 4-[(hexahydro-4-méthyl-1*H*-1,4-diazépin-1-yl)méthyl]-1-pipéridinecarboxylique, 1,1-diméthyléthyl ester

En opérant de façon analogue à la préparation LXXIX, au départ de N-méthyl-homopipérazine, on obtient le produit attendu sous forme d'une huile incolore (rendement = 83%).

RMN $^1$H (300 MHz, DMSO) δ : pas de RMN $^1$H

## PREPARATION LXXXII

### 4-[(hexahydro-4-méthyl-1*H*-1,4-diazépin-1-yl)méthyl]pipéridine

En opérant de façon analogue à la préparation XXIX, au départ de l'ester obtenu suivant la préparation LXXXI, on obtient le produit attendu sous forme d'une huile incolore (rendement = 23%).

RMN $^1$H (250 MHz, DMSO) δ: 3,03 (m, 2H) ; 2,57 (m, 5H) ; 2,50 (m, 5H) ; 2,25 (s, 2H) ; 2,22 (s, 3H) ; 1,68 (m, 4H) ; 1,63 (m, 1H) ; 1,08 (m, 2H).

**PREPARATION LXXXIII**

**Acide 4-(hexahydro-4-méthyl-1*H*-1,4-diazépin-1-yl)-1-pipéridinecarboxylique, 1,1-diméthyléthyl ester**

En opérant de façon analogue à la préparation LXXI, au départ de N-méthyl-homopipérazine et de l'ester t-butylique de l'acide 4-oxo-1-pipéridinecarboxylique, on obtient le produit attendu sous forme d'une huile jaune (rendement = 36%).

RMN $^1$H (250 MHz, CDCl3) δ : 4,15 (m, 2H) ; 2,82 (m, 4H) ; 2,70 (m, 7H) ; 2,42 (s, 3H) ; 1,87 (m, 2H) ; 1,98 (m, 2H) ; 1,45 (s, 9H) ; 1,40 (m, 2H).

**PREPARATION LXXXIV**

**4-(hexahydro-4-méthyl-1*H*-1,4-diazépin-1-yl)pipéridine, trichlorhydrate**

On dissout 409 mg (1,37 mM) du composé obtenu selon la préparation LXXXIII dans 1 ml de méthanol et on ajoute 27 ml d'une solution 2,3 N de chlorure d'hydrogène dans l'acétate d'éthyle. Le mélange est agité à température ambiante pendant 12 heures puis concentré sous pression réduite. On obtient ainsi le produit attendu sous forme d'une mousse beige (rendement = 99%).

RMN $^1$H (250 MHz, DMSO) δ: 3,88 (m, 4H) ; 3,62 (m, 1H) ; 3,43 (m, 6H) ; 2,94 (m, 2H) ; 2,82 (s, 3H) ; 2,31 (m, 4H) ; 2,10 (m, 2H).

**PREPARATION LXXXV**

**Acide 4-(4-cyclopropyl-1-pipérazinyl)-1-pipéridinecarboxylique, phénylméthyl ester**

En opérant de façon analogue à la préparation LXXI, au départ de N-cyclopropylpipérazine, on obtient le produit attendu sous forme d'un solide blanc (rendement = 62%).
F = 64°C

**PREPARATION LXXXVI**

**4-(4-cyclopropyl-1-pipérazinyl)pipéridine**

En opérant de façon analogue à la préparation LXXII, au départ de l'ester obtenu suivant la préparation LXXXV, on obtient le produit attendu sous forme d'un solide blanc (rendement = 90%).
F =107°C

**PREPARATION LXXXVII**

**Acide 4-[4-(1,1-diméthyléthyl)-1-pipérazinyl]-1-pipéridinecarboxylique, phénylméthyl ester**

En opérant de façon analogue à la préparation LXXI, au départ de N-*t*-butylpipérazine, on obtient le produit attendu sous forme d'un solide blanc (rendement = 50%).
F = 84°C

**PREPARATION LXXXVIII**

**4-[4-(1,1-diméthyléthyl)-1-pipérazinyl]pipéridine**

En opérant de façon analogue à la préparation LXXII, au départ de l'ester obtenu suivant la préparation LXXXVII, on obtient le produit attendu sous forme d'un solide blanc (rendement = 75%).
F = 82°C

**PREPARATION LXXXIX**

**Acide 4-(4-pipéridinyl)-1-pipéridinecarboxylique, 1,1-diméthyléthyl ester**

On prépare une solution de 41 g (0,17 M) de dichlorhydrate de 4,4'-bipipéridine dans 250 ml d'éthanol et 250

ml de soude 2N et on ajoute lentement, à 0 ˚C, une solution de 18,5 g (0,085 M) de dicarbonate de t-butyle dans 100 ml d'éthanol. Le mélange réactionnel est agité pendant 1 heure à 10 ˚C puis l'éthanol est chassé à l'évaporateur, sous pression réduite. La phase aqueuse résiduelle est saturée par du chlorure de sodium et extraite par l'acétate d'éthyle. La phase organique obtenue est séchée sur sulfate de magnésium et concentrée sous pression réduite. Le produit brut est purifié par chromatographie sur gel de silice en éluant à l'aide d'un mélange dichlorométhane/méthanol/ammoniaque (8/2/0,4 ; v/v/v). On obtient ainsi 16,5 g du composé attendu sous forme d'un solide blanc (rendement = 72 %).
F=70-71˚C

**PREPARATION XC**

**Acide 4-(1-éthyl-4-pipéridinyl)-1-pipéridinecarboxylique, 1,1-diméthyléthyl ester**

En opérant de façon analogue à la préparation LXXI, au départ de l'ester obtenu suivant la préparation LXXXIX et d'acétaldéhyde, on obtient le produit attendu sous forme d'un solide blanc (rendement = 87%).
F = 68-70 ˚C

**PREPARATION XCI**

**4-(1-éthyl-4-pipéridinyl)pipéridine, dichlorhydrate**

En opérant de façon analogue à la préparation LXXXIV, au départ de l'ester obtenu suivant la préparation XC, on obtient le produit attendu sous forme d'un solide blanc (rendement = 100%).
F = 280 ˚C

**PREPARATION XCII**

**Acide 4-[1-(1-méthyléthyl)-4-pipéridinyl]-1-pipéridinecarboxylique, 1,1-diméthyléthyl ester**

En opérant de façon analogue à la préparation XC, au départ de l'ester obtenu suivant la préparation LXXXIX et d'acétone, on obtient le produit attendu sous forme d'une huile incolore (rendement = 69%).
RMN $^1$H (250 MHz, CDCl3) δ: 4,10 (m, 2H) ; 3,52 (m, 1H) ; 3,47 (m, 2H) ; 2,55 (m, 4H) ; 1,88 (m, 4H) ; 1,85 (m, 4H) ; 1,45 (s, 9H) ; 1,30 (d, 6H) ; 1,12 (m, 2H).

**PREPARATION XCIII**

**4-[1-(1-méthyléthyl)-4-pipéridinyl]pipéridine, dichlorhydrate**

En opérant de façon analogue à la préparation LXXXIV, au départ de l'ester obtenu suivant la préparation XCII, on obtient le produit attendu sous forme d'un solide blanc (rendement = 89%).
F = 250 ˚C

**PREPARATION XCIV**

**Acide 4-(1-cyclopropyl-4-pipéridinyl)-1-pipéridinecarboxylique, 1,1-diméthyléthyl ester**

On dissout 150 mg (0,56 mM) du composé obtenu selon la préparation LXXXIX dans 5 ml de méthanol et on ajoute 320 µl d'acide acétique et 200 mg de tamis moléculaire 3 Å, puis 562 µl (2,79 mM) de 1-éthoxy-1-(triméthylsilyloxy) cyclopropane et 141 mg (2,23 mM) de borohydrure de sodium. Le milieu réactionnel est chauffé à doux reflux pendant 20 heures, puis refroidi et filtré. Le filtrat est concentré sous pression réduite et le résidu d'évaporation est repris dans 20 ml d'acétate d'éthyle. Cette phase organique est lavée avec une solution de soude 2N, puis avec une solution de chlorure de sodium saturée, séchée sur sulfate de magnésium et concentrée sous pression réduite. Le produit brut est purifié par chromatographie sur gel de silice en éluant à l'aide d'un mélange dichlorométhane/méthanol (95/5; v/v). On obtient ainsi 110 mg du composé attendu sous forme d'une poudre blanche (rendement = 64 %).
RMN $^1$H (250 MHz, CDCl3) δ : 4,10 (m, 2H) ; 3,05 (m, 4H) ; 2,65 (t, 2H) ; 2,09 (t, 2H) ; 1,67 (m, 4H) ; 1,55 (m, 1H) ; 1,45 (s, 9H) ; 1,22 (m, 6H) ; 0,47 (m, 4H).

## PREPARATION XCV

### 4-(1-cyclopropyl-4-pipéridinyl)pipéridine,dichlorhydrate

En opérant de façon analogue à la préparation LXXXIV, au départ de l'ester obtenu suivant la préparation XCIV, on obtient le produit attendu sous forme d'une pâte blanche (rendement = 99%).
RMN $^1$H (250 MHz, DMSO) δ : 10,50 (m large, 1H) ; 8,80 (m large, 2H) ; 3,45 (m, 2H) ; 3,16 (m, 2H) ; 2,81 (m, 2H) ; 2,73 (m, 3H) ; 1,90 (m, 4H) ; 1,60 (m, 2H) ; 1,35 (m, 4H) ; 1,10 (m, 2H) ; 0,78 (m, 2H).

## PREPARATION XCVI

### Acide 4-[2-(4-pipéridinyl)éthyl]-1-pipéridinecarboxylique, 1,1-diméthyléthyl ester

En opérant de façon analogue à la préparation LXXXIX, au départ du chlorhydrate de 4,4'-éthylènedipipéridine, on obtient le produit attendu sous forme d'un solide blanc (rendement = 39%).
F = 92-94°C

## PREPARATION XCVII

### 4-[2-(1-méthyl-4-pipéridinyl)éthyl]pipéridine

On prépare une solution de 307 mg (1,04 mM) de l'ester obtenu suivant la préparation XCVI dans 20 ml de tétrahydrofurane (THF) anhydre et on ajoute par portions 157 mg (4,14 mM) d'hydrure de lithium-aluminium. Le mélange réactionnel est agité pendant 10 heures à 70 °C puis refroidi et dilué avec 30 ml de THF. On ajoute 200 mg de sel de Glauber et le mélange est laissé sous agitation à température ambiante pendant une nuit, puis filtré. Le filtrat est concentré sous pression réduite pour donner 235 mg du produit attendu sous forme d'une pâte blanche (rendement = 99%).
RMN $^1$H (300 MHz, DMSO) δ : 3,16 (s, 2H) ; 2,80 (m, 2H) ; 2,70 (m, 2H) ; 2,38 (t, 2H) ; 2,10 (s, 3H) ; 1,76 (m, 2H) ; 1,58 (m, 3H) ; 1,42 (m, 1H) ; 1,11 (m, 8H) ; 0,94 (m, 2H).

## PREPARATION XCVIII

### 4-[2-(4-méthyl-1-pipérazinyl)éthyl]pyridine

On mélange, dans un tube, 2 ml (19 mM) de 4-vinylpyridine, 3,16 ml (28,5 mM) de N-méthylpiperazine, 200 µl d'acide acétique dans 12 ml d'éthanol. Le tube est fermé et chauffé pendant 10 min à 160°C dans un four micro-ondes. Après refroidissement, on ajoute doucement 20 ml de soude 0,5 N et le mélange est extrait par du dichlorométhane. La phase organique séparée est séchée sur sulfate de magnésium et concentrée sous pression réduite. On obtient ainsi le produit attendu sous forme d'une huile jaune (rendement = 86%).
RMN $^1$H (250 MHz, DMSO) δ : 8,43 (dd, 2H) ; 7,25 (dd, 2H) ; 2,73 (t, 2H) ; 2,52 (m, 2H) ; 2,48 (m, 4H) ; 2,30 (m, 4H) ; 2,13 (s, 3H).
En opérant de façon analogue à la préparation XCVIII, on obtient les dérivés de pyridine suivants :

## PREPARATION IC

### 4-[2-(1-pipéridinyl)éthyl]pyridine

huile jaune (rendement = 52%)
RMN $^1$H (250 MHz, DMSO) δ : 8,43 (dd, 2H) ; 7,24 (dd, 2H) ; 2,73 (t, 2H) ; 2,49 (m, 2H) ; 2,37 (t, 2H) ; 1,43 (m, 6H).

## PREPARATION C

### 4-[2-[(méthyl)(1-méthyléthyl)amino]éthyl]pyridine

huile jaune (rendement = 10%).
RMN $^1$H (250 MHz, DMSO) δ : 8,49 (d, 2H) ; 7,16 (d, 2H) ; 2,76 (hep, 1H) ; 2,65 (t, 2H) ; 2,56 (d, 2H) ; 2,15 (s, 3H) ; 0,90 (d, 6H).

## PREPARATION CI

### 4-[2-(4-morpholinyl)éthyl]pyridine

huile jaune (rendement = 72%).
RMN $^1$H (2500 MHz, DMSO) δ : 8,44 (dd, 2H) ; 7,26 (dd, 2H) ; 3,05 (dd, 4H) ; 2,75 (t, 2H) ; 2,53 (m, 2H) ; 2,41 (dd, 2H).

## PREPARATION CII

### 4-[2-(1-azétidinyl)éthyl]pyridine

huile jaune (rendement = 62%).
RMN $^1$H (300 MHz, DMSO) δ : 8,43 (d, 2H) ; 7,22 (d, 2H) ; 3,07 (t, 4H) ; 2,53 (m, 4H) ; 1,91 (quin, 2H).

## PREPARATION CIII

### Acide (méthyl)[2-(4-pyridinyl)éthyl]carbamique, 1,1-diméthyléthyl ester

huile jaune (rendement = 83%).
RMN $^1$H (300 MHz, DMSO) δ : 8,45 (d, 2H) ; 7,21 (d, 2H) ; 3,42 (t, 2H) ; 2,76 (t, 2H) ; 2,75 (s, 3H) ; 1,23 (s, 9H).

## PREPARATION CIV

### 4-[2-[(méthyl)(éthyl)amino]éthyl]pyridine

huile incolore (rendement = 51%).
RMN $^1$H (300 MHz, DMSO) δ : 8,50 (d, 2H) ; 7,25 (d, 2H) ; 2,73 (t, 2H) ; 2,55 (t, 2H) ; 2,43 (q, 2H) ; 2,23 (s, 3H) ; 0,90 (t; 3H).

## PREPARATION CV

### 4-[2-(diéthylamino)éthyl]pyridine

huile jaune (rendement = 30%).
RMN $^1$H (250 MHz, DMSO) δ : 8,43 (dd, 2H) ; 7,24 (dd, 2H) ; 2,65 (m, 4H) ; 2,40 (q, 4H) ; 0,93 (t, 6H).

## PREPARATION CVI

### 4-[2-(hexahydro-4-méthyl-1*H*-1,4-diazépin-1-yl)éthyl]pyridine

huile jaune (rendement = 52%)
RMN $^1$H (250 MHz, DMSO) δ : 8,43 (d, 2H) ; 7,25 (d, 2H) ; 2,71 (m, 8H) ; 2,50 (m, 4H) ; 2,22 (s, 3H) ; 1,68 (m, 2H).

## PREPARATION CVII

### 4-[2-(4-méthyl-1-pipérazinyl)éthyl]pipéridine

En opérant de façon analogue à la préparation LXIX, au départ du composé obtenu selon la préparation XCVIII, on obtient le produit attendu sous forme d'un solide jaune (rendement = 80%).
F = 111-112 °C

## PREPARATION CVIII

### 4-[2-(diméthylamino)éthyl]pipéridine

En opérant de façon analogue à la préparation CVII, au départ de 4-[2-(diméthylamino)éthyl]pyridine, on obtient le produit attendu sous forme d'une huile incolore (rendement = 96%).

RMN $^1H$ (300 MHz, DMSO) δ : 2,86 (2H) ; 2,39 (dt, 2H) ; 2,18 (dd, 2H) ; 2,08 (s, 6H) ; 1,54 (dm, 2H) ; 1,27 (m, 3H) ; 0,96 (m, 2H).

## PREPARATION CIX

### 4-[2-(1-pyrrolidinyl)éthyl]pipéridine

En opérant de façon analogue à la préparation CVII, au départ de 4-[2-(1-pyrrolidinyl)éthyl]pyridine, on obtient le produit attendu sous forme d'une huile incolore (rendement = 88%).
RMN $^1H$ (300 MHz, DMSO) δ: 2,86 (dm, 2H) ; 2,38 (m, 8H) ; 1,64 (m, 4H) ; 1,54 (dm, 2H) ; 1,32 (m, 3H) ; 0,96 (dq, 2H).
En opérant de façon analogue à la préparation CVII, au départ des composés obtenus selon les préparations IC à CVI, on obtient les dérivés de pipéridine suivants :

## PREPARATION CX

### 4-[2-(1-pipéridinyl)éthyl]pipéridine

huile jaune (rendement = 92%).
RMN $^1H$ (300 MHz, DMSO) δ : 3,10 (dm, 2H) ; 2,67 (dt, 2H) ; 2,27 (m, 6H) ; 1,70 (m, 2H) ; 1,47 (m, 5H) ; 1,35 (m, 4H) ; 1,23 (m, 2H).

## PREPARATION CXI

### 4-[2-[(méthyl)(1-méthyléthyl)amino]éthyl]pipéridine

huile jaune (rendement = 96%).
RMN $^1H$ (250 MHz, DMSO) δ : 3,10 (d, 2H) ; 2,77 (m, 1H) ; 2,60 (m, 2H) ; 2,35 (t, 2H) ; 2,10 (s, 3H) ; 1,72 (m, 2H) ; 1,34 (m, 1H) ; 1,29 (t, 2H) ; 1,23 (m, 2H) ; 0,91 (d, 6H).

## PREPARATION CXII

### 4-[2-(4-morpholinyl)éthyl]pipéridine

huile incolore (rendement = 98%).
RMN $^1H$ (300 MHz, DMSO) δ : 3,54 (t, 4H) ; 2,86 (dt, 2H) ; 2,39 (dt, 2H) ; 2,28 (m, 4H) ; 2,25 (t, 2H) ; 1,54 (dm, 2H) ; 1,31 (m, 3H) ; 0,97 (m, 2H).

## PREPARATION CXIII

### 4-[2-(1-azétidinyl)éthyl]pipéridine

huile incolore (rendement = 74%).
RMN $^1H$ (250 MHz, DMSO) δ : 3,16 (t, 4H) ; 3,12 (m, 2H) ; 2,65 (m, 2H) ; 2,35 (t, 2H) ; 1,94 (quin, 2H) ; 1,85 (m, 2H) ; 1,35 (m, 1H) ; 1,23 (t, 2H) ; 1,15 (m, 2H).

## PREPARATION CXIV

### Acide (méthyl)[2-(4-pipéridinyl)éthyl]carbamique, 1,1-diméthyléthyl ester

huile incolore(rendement = 64%).
RMN $^1H$ (300 MHz, DMSO) δ: 3,16 (t, 2H) ; 2,89 (dm, 2H) ; 2,73 (s, 3H) ; 2,40 (t, 2H) ; 1,38 (s, 9H) ; 1,35 (m, 2H) ; 0,96 (m, 2H).

## PREPARATION CXV

### 4-[2-[(méthyl)(éthyl)amino]éthyl]pipéridine

huile incolore (rendement = 98%).

RMN $^1$H (300 MHz, DMSO) δ : 3,13 (m, 2H) ; 2,68 (m, 2H) ; 2,32 (m, 4H) ; 2,11 (s, 3H) ; 1,85 (m, 2H) ; 1,51 (m, 1H) ; 1,34 (t, 2H) ; 1,24 (m, 2H) ; 0,96 (t, 3H).

## PREPARATION CXVI

### 4-[2-(diéthylamino)éthyl]pipéridine

huile jaune (rendement = 87%).

RMN $^1$H (250 MHz, DMSO) δ : 2,87 (dm, 2H) ; 2,40 (m, 8H) ; 1,54 (dm, 2H) ; 1,26 (m, 3H) ; 0,98 (m, 2H) ; 0,91 (t, 6H).

## PREPARATION CXVII

### 4-[2-(hexahydro-4-méthyl-1*H*-1,4-diazépin-1-yl)éthyl]pipéridine

huile jaune (rendement = 79%).

RMN $^1$H (300 MHz, DMSO) δ: 2,89 (d, 2H) ; 3,05 (m, 2H) ; 2,58 (m, 4H) ; 2,49 (m, 4H) ; 2,46 (m, 4H) ; 2,21 (s, 3H) ; 1,66 (m, 2H) ; 1,64 (m, 2H) ; 1,28 (m, 3H) ; 0,99 (m, 2H).

## PREPARATION CXVIII

### Acide 4-(2-hydroxy-1,1-diméthyléthyl)-1-pipéridinecarboxylique, 1,1-diméthyléthyl ester

En opérant de façon analogue à la préparation LXXXIX, au départ de β,β-diméthyl-4-pipéridineéthanol, on obtient le produit attendu sous forme d'une huile incolore (rendement = 96%).

RMN $^1$H (300 MHz, DMSO) δ : 4,41 (t, 1H) ; 3,98 (d, 2H) ; 3,13 (d, 2H) ; 2,51 (m, 2H) ; 1,50 (d, 2H) ; 1,38 (s, 9H) ; 1,33 (m, 1H) ; 1,04 (m, 2H) ; 0,93 (s, 6H.

## PREPARATION CXIX

### Acide 4-(1,1-diméthyl-2-oxoéthyl)-1-pipéridinecarboxylique, 1,1-diméthyléthyl ester

On prépare une solution de 68 µl (0,77 mM) de chlorure d'oxalyle dans 6 ml de dichlorométhane anhydre. On ajoute, à -70°C, 82,5 µl de diméthylsulfoxyde, puis après 15 min, 100 mg (0,39 mM) de l'alcool obtenu selon la préparation CXVIII en solution dans 4ml de dichlorométhane, puis, après 5 min, 270 µl de triéthylamine. Le mélange est agité 5 min à - 70°C, puis laissé revenir à température ambiante. Après addition de 25 ml d'acétate d'éthyle, cette phase organique est lavée par une solution à 10 % de bicarbonate de sodium, séchée sur sulfate de magnésium et concentrée sous pression réduite. Le produit brut est purifié par chromatographie sur gel de silice en éluant à l'aide d'un mélange dichlorométhane/acétate d'éthyle (85/15 ; v/v). On obtient ainsi 80 mg du composé attendu sous forme d'une huile incolore (rendement = 80 %).

RMN $^1$H (250 MHz, DMSO) δ : 9,44 (s, 1H) ; 3,96 (d, 2H) ; 2,59 (m, 2H) ; 1,69 (m, 1H) ; 1,50 (m, 2H) ; 1,38 (s, 9H) ; 1,07 (m, 2H) ; 0,93 (s, 6H).

## PREPARATION CXX

### Acide 4-[2-(1-azétidinyl)-1,1-diméthyléthyl]-1-pipéridinecarboxylique, 1,1-diméthyléthyl ester

En opérant de façon analogue à la préparation LXXI, au départ du composé obtenu selon la préparation CXIX et d'azétidine, on obtient le produit attendu sous forme d'une huile incolore (rendement = 65%).

RMN $^1$H (300 MHz, DMSO) δ : 3,96 (d, 2H) ; 3,13 (t, 4H) ; 2,51 (m, 2H) ; 2,16 (s, 2H) ; 1,92 (quin, 2H) ; 1,55 (d, 2H) ; 1,39 (s, 9H) ; 1,35 (m, 1H) ; 1,05 (m, 2H) ; 0,70 (s, 6H).

**PREPARATION CXXI**

**4-[2-(1-azétidinyl)-1,1-diméthyléthyl]pipéridine, bis(trifluoroacétate)**

520 mg (1,75 mM) du composé obtenu selon la préparation CXX sont mélangés à 3 ml d'acide trifluoroacétique et 380 µl d'anisole dans 3 ml de dichlorométhane. Le mélange est agité une nuit à température ambiante puis concentré sous pression réduite. Le résidu est repris dans 20 ml de toluène et à nouveau concentré sous pression réduite. Le produit brut est trituré dans 4 ml d'éther éthylique pour donner un solide qui est séparé par filtration et séché. On obtient ainsi 683 mg du composé attendu (rendement = 92 %).
F = 138-140 °C

**PREPARATION CXXII**

**Acide 4-[2-(4-morpholinyl)-1,1-diméthyléthyl]-1-pipéridinecarboxylique, 1,1-diméthyléthyl ester**

En opérant de façon analogue à la préparation CXX, au départ de morpholine, on obtient le produit attendu sous forme d'une huile incolore (rendement = 12%).
RMN $^1$H (300 MHz, DMSO) δ : 3,97 (d, 2H) ; 3,53 (t, 4H) ; 2,57 (m, 2H) ; 2,41 (t, 4H) ; 2,10 (s, 2H) ; 1,60 (d, 2H) ; 1,38 (s, 9H) ; 1,29 (m, 1H) ; 1,05 (m, 2H) ; 0,77 (s, 6H).

**PREPARATION CXXIII**

**4-[2-(4-morpholinyl)-1,1-diméthyléthyl]pipéridine, bis(trifluoroacétate)**

En opérant de façon analogue à la préparation CXXI, au départ du composé obtenu selon la préparation CXXII, on obtient le produit attendu sous forme d'un solide jaune pâle (rendement = 69 %).
F =158-160 °C

**PREPARATION CXXIV**

**4-[2-(1-pipéridinyl)-1,1-diméthyléthyl]pyridine**

On porte à reflux pendant 48 heures une suspension de 70 g (0,58 M) de 4-isopropylpyridine, 250 g (2 M) de chlorhydrate de pipéridine et 85 g de paraformaldéhyde dans 600 ml d'éthanol à 95 %. Le mélange réactionnel est ensuite concentré sous pression réduite et le résidu est repris par 650 ml de soude 3 N et extrait par 3 fois 250 ml d'acétate d'éthyle. Les phases organiques rassemblées sont lavées par une solution de chlorure de sodium, séchées et concentrées sous pression réduite. Le produit brut est distillé sous un vide de 1 mm Hg et la fraction recueillie entre 80 et 125 °C est purifiée par chromatographie sur gel de silice en éluant à l'aide d'un mélange toluène/isopropanol (9/1 ; v/v). On obtient ainsi 21,4 g du composé attendu sous forme d'une huile jaune claire (rendement = 17%).
RMN $^1$H (300 MHz, CDCl3) δ : 8,49 (d, 2H) ; 7,32 (d, 2H) ; 2,53 (s, 2H) ; 2,20 (t, 4H) ; 1,39 (m, 4H) ; 1,36 (m, 2H) ; 1,29 (s, 6H),

**PREPARATION CXXV**

**4-[2-(1-pipéridinyl)-1,1-diméthyléthyl]pipéridine**

En opérant de façon analogue à la préparation CVII, au départ du composé obtenu selon la préparation CXXIV, on obtient le produit attendu sous forme d'une huile jaune (rendement = 33 %).
RMN $^1$H (300 MHz, DMSO) δ : 2,94 (dm, 2H) ; 2,37 (m, 6H) ; 2,03 (s, 2H) ; 1,45 (m, 6H) ; 1,32 (m, 2H) ; 1,18 (m, 1H) ; 1,05 (m, 2H) ; 0,74 (s, 6H).
En opérant de façon analogue aux préparations CXXIV et CXXV, on obtient les deux composés suivants :

**PREPARATION CXXVI**

**4-[2-(1-pyrrolidinyl)-1,1-diméthyléthyl]pipéridine**

huile jaune (rendement = 72 %).
RMN $^1$H (250 MHz, DMSO) δ : 2,97 (d, 2H) ; 2,55 (m, 4H) ; 2,48 (m, 2H) ; 2,26 (s, 2H) ; 1,66 (m, 4H) ; 1,62 (d,

2H) ; 1,29 (m, 1H) ; 1,03 (m, 2H) ; 0,77 (s, 6H).

## PREPARATION CXXVII

### 4-[2-(diéthylamino)-1,1-diméthyléthyl]pipéridine

huile incolore(rendement = 79 %).
RMN $^1$H (300 MHz, DMSO) δ : pas de RMN $^1$H

## PREPARATION CXXVIII

### N,1-diméthyl-N-[1-(phénylméthyl)-4-pipéridinyl]-4-pipéridinamine

En opérant de façon analogue à la préparation LXXI, au départ de N-méthyl-4-pipéridinone et de 1-benzyl-4-(méthylamino)pipéridine, on obtient le produit attendu sous forme d'une huile jaune (rendement = 66 %).
RMN $^1$H (300 MHz, DMSO) δ : 7,28 (m, 5H) ; 3,41 (s, 2H) ; 2,76 (m, 4H) ; 2,40 (m, 2H) ; 2,12 (s, 3H) ; 2,10 (s, 3H) ; 1,90 (dt, 2H) ; 1,81 (dt, 2H) ; 1,57 (dm, 4H) ; 1,44 (m, 4H).

## PREPARATION CXXIV

### N,1-diméthyl-N-(4-piperidinyl)-4-pipéridinamine

On prépare une solution de 980 mg (3,25 mM) du composé obtenu selon la préparation CXXVIII dans 60 ml de méthanol et on ajoute 150 mg de charbon palladié à 10 %. Le mélange est agité sous atmosphère d'hydrogène, sous une pression de 50 PSI (3,5 bars ou 3450 hPa), à température ambiante pendant 10 heures. Le catalyseur est éliminé par filtration et le filtrat est concentré sous pression réduite. Le produit brut obtenu est purifié par chromatographie sur gel de silice en éluant à l'aide d'un mélange dichlorométhane/ méthanol/ammoniaque (97/3/0,3 ; v/v/v). On obtient le produit attendu sous forme d'une huile jaune (rendement = 29 %).
RMN $^1$H (250 MHz, DMSO) δ: 2,94 (d, 2H) ; 2,76 (d, 2H) ; 2,43 (m, 4H) ; 2,12 (s, 3H) ; 2,10 (s, 3H) ; 1,81 (m, 2H) ; 1,76 (m, 4H) ; 1,45 (m, 2H) ; 1,30 (m, 2H).

## PREPARATION CXXX

### 4-[2-oxo-2-(4-méthyl-1-piperazinyl)éthyl]pyridine

On prépare une solution de 3 g (17,3 mM) d'acide (4-pyridinyl)acétique dans 50 ml de tétrahydrofurane (THF) et on ajoute goutte à goutte, à température ambiante, une solution de 3,4 g (20,7 mM) de carbonyldiimidazole en solution dans 50 ml de THF. Le mélange réactionnel est agité pendant 8 heures, puis on ajoute une solution de 1,73 g (17,3 mM) de N-méthylpipérazine dans 20 ml de THF. Le milieu réactionnel est chauffé à reflux du solvant pendant 2 heures, puis concentré sous pression réduite. Le résidu d'évaporation est repris par 80 ml de soude 3 N et extrait par l'acétate d'éthyle. La phase organique est séchée sur sulfate de magnésium et concentrée sous pression réduite. Le produit brut est purifié par chromatographie sur gel de silice en éluant à l'aide d'un mélange dichlorométhane/méthanol (99/1 ; v/v). On obtient le produit attendu sous forme d'une huile incolore (rendement = 28 %).
RMN $^1$H (250 MHz, DMSO) δ: 8,47 (dd, 2H) ; 7,23 (dd, 2H) ; 3,75 (s, 2H) ; 3,46 (t, 2H) ; 2,23 (t, 4H) ; 2,15 (3H),

## PREPARATION CXXXI

### 4-[2-oxo-2-(4-méthyl-1-piperazinyl)éthyl]piperidine

En opérant de façon analogue à la préparation CVII, au départ du composé obtenu selon la préparation CXXX, on obtient le produit attendu sous forme d'une huile incolore (rendement = 59 %).
RMN $^1$H (250 MHz, DMSO) δ : 3,42 (t, 4H) ; 3,28 (m, 2H) ; 2,87 (dt, 2H) ; 2,41 (dt, 2H) ; 2,24 (m, 6H) ; 2,16 (s, 3H) ; 1,71 (m, 1H) ; 1,54 (dm, 2H) ; 1,06 (dq, 2H).
En opérant de façon analogue aux exemples 3 et 4, au départ des acides et amines obtenus ci-dessus (ou d'amines connues de la littérature), on obtient les composés selon l'invention suivants :

**Exemple 16**

**N-[2-[2-[4-[3-(1-azétidinyl)propyl]-1-pipérazinyl]-2-oxo-éthoxy]éthyl]-4-méthoxy-N,2,6-triméthylbenzènesulfonamide, difumarate**

Solide beige (rendement = 34 %).
F = 82 °C

**Exemple 17**

**N-[2-[2-[4-(1-méthyl-3-pipéridinyl)-1-pipérazinyl]-2-oxo-éthoxy] éthyl]-4-méthoxy-N,2,6-triméthylbenzènesulfonamide, difumarate**

Solide blanc (rendement = 36 %).
F = 60-65 °C

**Exemple 18**

**N-[2-[2-[4-(1-méthyl-4-pipéridinyl)-1-pipérazinyl]-2-oxo-éthoxy]éthyl]-4-méthoxy-N-cyclopropyl-2,6-diméthylbenzènesulfonamide, difumarate**

Solide blanc (rendement = 51 %).
F = 185 °C

**Exemple 19**

**N-[2-[2-[4-[2-(1-pyrrolidinyl)éthyl]-1-pipérazinyl]-2-oxo-éthoxy]éthyl]-4-méthoxy-N-cyclopropyl-2,6-diméthylbenzènesulfonamide, difumarate**

Solide blanc (rendement = 42%).
F=141°C

**Exemple 20**

**N-[2-[2-[4-[(1-méthyl-2-imidazolyl)méthyl]-1-pipérazinyl]-2-oxo-éthoxy]éthyl]-4-méthoxy-N,2,6-triméthylbenzènesulfonamide, difumarate**

Solide blanc (rendement = 45 %).
F = 60-65 °C

**Exemple 21**

**N-[2-[2-[4-(1-méthyl-4-pipéridinyl)-1-pipérazinyl]-2-oxo-éthoxy]éthyl]-4-méthoxy-N-éthyl-2,6-diméthylbenzènesulfonamide, difumarate**

Solide blanc (rendement = 48 %).
F = 206 °C

**Exemple 22**

**N-[2-[2-[4-[3-(diméthylamino)propyl]-1-pipérazinyl]-2-oxo-éthoxy]éthyl]-4-méthoxy-N-éthyl-2,6-diméthylbenzènesulfonamide, difumarate**

Solide blanc (rendement = 26 %).
F = 60°C

**Exemple 23**

**N-[2-[2-[4-(9-méthyl-9-azabicyclo[3.3.1]non-3-yl)-1-pipérazinyl]-2-oxo-éthoxy]éthyl]-4-méthoxy-N,2,6-triméthylbenzènesulfonamide, fumarate**

Solide blanc (rendement = 73 %).
F=96°C

**Exemple 24**

**N-[2-[2-[*4-[3-(1-pyrrolidinyl)propyl]-1-pipérazinyl]-2-oxo-éthoxy]éthyl]-4-méthoxy-N-éthyl-2,6-diméthylbenzènesulfonamide, difumarate**

Solide blanc (rendement = 63 %).
F=65°C

**Exemple 25**

**N-[2-[2-[4-[3-(1-pyrrolidinyl)propyl]-1-pipérazinyl]-2-oxo-éthoxy]éthyl]-4-méthoxy-N-cyclopropyl-2,6-diméthylbenzènesulfonamide, difumarate**

Solide écru (rendement = 61 %).
F = 55°C

**Exemple 26**

**N-[2-[2-[4-(8-cyclopropyl-8-azabicyclo[3.2.1]oct-3-yl)-1-pipérazinyl]-2-oxo-éthoxy]éthyl]-4-méthoxy-N,2,6-triméthylbenzènesulfonamide, fumarate**

Solide blanc (rendement = 81 %).
F = 75°C

**Exemple 27**

**N-[2-[2-[4-(8-méthyl-8-azabicyclo[3.2.1]oct-3-yl)-1-pipérazinyl]-2-oxo-éthoxy]éthyl]-4-méthoxy-N-éthyl-2,6-diméthylbenzènesulfonamide, difumarate**

Solide blanc (rendement = 64%).
F = 185°C

**Exemple 28**

**N-[2-[2-[4-(8-méthyl-8-azabicyclo[3.2.1]oct-3-yl)-1-pipérazinyl]-2-oxo-éthoxy]éthyl]-4-méthoxy-N-cyclopropyl-2,6-diméthylbenzènesulfonamide, difumarate**

Solide blanc (rendement = 51%).
F =160°C

**Exemple 29**

**N-[2-[2-[4-(1-cyclopropyl-4-pipéridinyl)-1-pipérazinyl]-2-oxo-éthoxy]éthyl]-4-méthoxy-N,2,6-triméthylbenzènesulfonamide, difumarate**

Solide blanc (rendement = 60%).
F=112-114°C

**Exemple 30**

**N-[2-[2-[4-(8-méthyl-8-azabicyclo[3.2.1]oct-3-yl)-1-pipérazinyl]-2-oxo-éthoxy]éthyl]-4-méthoxy-N-(1-méthyléthyl)-2,6-diméthylbenzènesulfonamide, difumarate**

Solide blanc (rendement = 60%).
F = 168°C

**Exemple 31**

**N-[2-[2-[4-(1-éthy!-4-pipéridinyl)-1-pipérazinyl]-2-oxo-éthoxytéthyl]-4-méthoxy-N,2,6-triméthylbenzènesulfonamide, fumarate**

Solide beige (rendement = 48%).

RMN $^1H$ (250 MHz, DMSO) δ : 6,80 (s, 2H) ; 6,53 (s, 2H) ; 4,05 (s, 2H) ; 3,80 (s, 3H) ; 3,53 (t, 2H) ; 3,39 (m, 2H) ; 3,29 (m, 2H) ; 3,22 (t, 2H) ; 3,12 (m, 2H) ; 2,70 (s, 3H) ; 2,62 (q, 2H) ; 2,53 (s, 6H) ; 2,48 (m, 4H) ; 2,29 (m, 3H) ; 1,77 (m, 2H) ; 1,52 (m, 2H) ; 1,07 (t, 2H).

**Exemple 32**

**N-[2-[2-[4-[1-(1,1-diméthyléthyl)-4-pipéridinyl]-1-pipérazinyl]-2-oxo-éthoxy]éthyl]-4-méthoxy-N,2,6-triméthylbenzènesulfonamide, difumarate**

Solide blanc (rendement =41%).
F = 230°C

**Exemple 33**

**N-[2-[2-[4-[(1-méthyl-4-pipéridinyl)méthyl]-1-pipérazinyl]-2-oxo-éthoxy]éthyl]-4-méthoxy-N,2,6-triméthylbenzènesulfonamide, fumarate**

Solide jaune (rendement = 62%).
F = 50°C

**Exemple 34**

**N-[2-[2-[4-[3-(diméthylamino)propyl]-1-pipérazinyl]-2-oxo-éthoxy]éthyl]-2,6-dichloro-4-méthoxy-N-méthylbenzènesulfonamide, difumarate**

Solide blanc (rendement = 78 %).
F = 60°C

**Exemple 35**

**N-[2-[2-[4-(1-méthyl-4-pipéridinyl)-1-pipérazinyl]-2-oxo-éthoxy]éthyl]-2,6-dichloro-4-méthoxy-N-méthyl-benzènesulfonamide, difumarate**

Solide blanc (rendement = 69 %).
F = 125°C

**Exemple 36**

**N-[2-[2-[4-[2-(1-méthyl-4-pipéridinyl)éthyl]-1-pipérazinyl]-2-oxo-éthoxy]éthyl]-4-méthoxy-N,2,6-triméthylbenzènesulfonamide, difumarate**

Solide écru (rendement = 62%).
F = 90°C

**Exemple 37**

**N-[2-[2-[4-(1-méthyl-4-pipéridinyl)hexahydro-1*H*-1,4-diazépin-1-yl]-2-oxo-éthoxy]éthyl]-4-méthoxy-N,2,6-triméthylbenzènesulfonamide, fumarate**

Solide blanc (rendement = 56%).
F=53˚C

**Exemple 38**

**N-[2-[2-[4-(1-méthyl-4-pipéridinyl)-1-pipérazinyl]-2-oxo-éthoxy]éthyl]-4-méthoxy-N-(1-méthyléthyl)-2,6-diméthylbenzènesulfonamide, difumarate**

Solide blanc (rendement = 41 %).
F=170˚C

**Exemple 39**

**N-[2-[2-[4-[1-(1-méthyléthyl)-4-pipéridinyl]-1-pipérazinyl]-2-oxo-éthoxy]éthyl]-4-méthoxy-N,2,6-triméthylbenzènesulfonamide, fumarate**

Solide blanc (rendement = 81 %).
F=130˚C

**Exemple 40**

**N-[2-[2-[4-[3-(1-pipéridinyl)propyl]-1-pipérazinyl]-2-oxo-éthoxy]éthyl]-4-méthoxy-N-éthyl-2,6-diméthylbenzènesulfonamide, difumarate**

Solide écru (rendement = 60%).
F = 65˚C

**Exemple 41**

**N-[2-[2-[4-(1-méthyl-4-pipéridinyl)-1-pipérazinyl]-2-oxo-éthoxy]éthyl]-2,4-dichloro-6-méthoxy-N-méthyl-benzènesulfonamide, difumarate**

Solide blanc (rendement = 46 %).
F = 202 ˚C

**Exemple 42**

**N-[2-[2-[4-(1-éthyl-4-pipéridinyl)-1-pipérazinyl]-2-oxo-éthoxy]éthyl]-2,6-dichloro-4-méthoxy-N-méthyl-benzènesulfonamide, fumarate**

Solide blanc (rendement = 68 %).
F = 96-98 ˚C

**Exemple 43**

**N-[2-[2-[4-((3S)-1-azabicyclo[2.2.2]oct-3-yl)-1-pipérazinyl]-2-oxo-éthoxy]éthyl]-4-méthoxy-N-méthyl-2,6-dichlorobenzènesulfonamide, fumarate**

Solide blanc (rendement = 36 %).
F = 75-79 ˚C

En opérant de façon analogue à l'exemple 2, au départ des acides et amines obtenus ci-dessus (ou d'amines connues de la littérature), et en utilisant l'acide adapté pour la salification du composé basique purifié, on obtient les composés selon l'invention suivants :

**Exemple 44**

**N-[2-[2-[4-(1,2,2,6,6-pentaméthyl-4-pipéridinyl)-1-pipérazinyl]-2-oxo-éthoxy]éthyl]-4-méthoxy-N,2,6-triméthylbenzènesulfonamide, bis(trifluoroacétate)**

Huile incolore (rendement = 37%).

RMN $^1$H (300 MHz, CD3CN) δ : 8,70 (m large, 1H) ; 6,75 (s, 2H) ; 4,10 (s, 2H) ; 3,80 (s, 3H) ; 3,76 (m, 4H) ; 3,70 (t, 1H) ; 3,61 (t, 2H) ; 3,28 (m, 4H) ; 2,27 (s, 6H) ; 2,57 (s, 6H) ; 2,28 (dm, 4H) ; 1,47 (s, 6H) ; 1,38 (s, 6H).

**Exemple 45**

**N-[2-[2-[4-[3-(4-méthyl-1-pipérazinyl)propyl]-1-pipérazinyl]-2-oxo-éthoxy]éthyl]-4-méthoxy-N,2,6-triméthylbenzènesulfonamide, difumarate**

Solide blanc (rendement = 30%).
F = 204 ˚C

**Exemple 46**

**N-[2-[2-[4-(8-éthyl-8-azabicyclo[3.2.1]oct-3-yl)-1-pipérazinyl]-2-oxo-éthoxy]éthyl]-4-méthoxy-N,2,6-triméthylbenzènesulfonamide, difumarate**

Solide blanc (rendement = 27%).
F=132˚C

**Exemple 47**

**N-[2-[2-[4-[3-(4-méthyl-hexahydro-*1H*-1,4-diazépin-1-yl)propyl]-1-pipérazinyl]-2-oxo-éthoxy]éthyl]-4-méthoxy-N,2,6-triméthylbenzènesulfonamide, difumarate**

Solide blanc (rendement = 45%).
F = 169 ˚C

**Exemple 48**

**N-[2-[2-[4-(8-(1-méthyléthyl)-8-azabicyclo [3.2.1]oct-3-yl]-1-pipérazinyl]-2-oxo-éthoxy]éthyl]-4-méthoxy-N,2,6-triméthylbenzènesulfonamide, difumarate**

Solide blanc (rendement = 35%).
F = 132˚C

**Exemple 49**

**N-[2-[2-[4-[3-(4-méthyl-hexahydro-1*H*-1,4-diazépin-1-yl)-3-oxo-propyl]-1-pipérazinyl]-2-oxo-éthoxy]éthyl]-4-méthoxy-N,2,6-triméthylbenzènesulfonamide, bis(trifluoroacétate)**

Huile incolore (rendement = 35%).

RMN $^1$H (300 MHz, CD3CN) δ : 6,76 (s, 2H) ; 4,15 (s, 2H) ; 3,84 (s, 3H) ; 3,82 (m, 6H) ; 3,65 (t, 2H) ; 3,62 (m, 2H) ; 3,44 (t, 2H) ; 3,36 (m, 10H) ; 2,90 (t, 2H) ; 2,87 (s, 3H) ; 2,77 (s, 3H) ; 2,61 (s, 6H) ; 2,18 (m, 2H).

**Exemple 50**

**N-[2-[2-[4-[2-(4-méthyl-hexahydro-*1H*-1,4-diazépin-1-yl)éthyl]-1-pipérazinyl]-2-oxo-éthoxy]éthyl]-4-méthoxy-N,2,6-triméthylbenzènesulfonamide, trifumarate**

Solide blanc (rendement = 8 %).
F=176˚C

En opérant de façon analogue aux exemples 5 et 4, au départ des acides obtenus ci-dessus, on obtient les

deux composés selon l'invention suivants :

**Exemple 51**

**N-[2-[2-[4-((3S)-1-azabicyclo[2.2.2]oct-3-yl)-1-pipérazinyl]-2-oxo-éthoxy]éthyl]-4-méthoxy-N-cyclopropyl-2,6-diméthylbenzènesulfonamide, difumarate**

Solide blanc (rendement = 37 %).
F=72°C
$[\alpha_D^{25}]$ = -11,4 ° (c = 0,5 ; $CH_3OH$)

**Exemple 52**

**N-[2-[2-[4-((3S)-1-azabicyclo[2.2.2]oct-3-yl)-1-pipérazinyl]-2-oxo-éthoxy]éthyl]-4-méthoxy-N-éthyl-2,6-diméthylbenzènesulfonamide, difumarate**

Solide blanc (rendement = 32 %).
F = 70 °C
$[\alpha_D^{25}]$ = -11 ° (c = 0,5 ; $CH_3OH$)

En opérant de façon analogue à l'exemple 1, au départ de l'acide obtenu selon la préparation III et de 1-[2-(diéthylamino)éthyl]pipérazine, on obtient le composé selon l'invention suivant :

**Exemple 53**

**N-[2-[2-[4-[2-(diéthylamino)éthyl]-1-pipérazinyl]-2-oxo-éthoxy]éthyl]-4-méthoxy-N,2,6-triméthylbenzènesulfonamide, fumarate**

Solide pâteux blanc (rendement = 77 %).

RMN $^1H$ (300 MHz, CD3CN) $\delta$ : 6,74 (s, 2H) ; 6,60 (s, 2H) ; 4,02 (s, 2H) ; 3,80 (s, 3H) ; 3,54 (t, 2H) ; 3,46 (m, 2H) ; 3,32 (m, 2H) ; 3,26 (t, 2H) ; 2,96 (m, 6H) ; 2,74 (s, 3H) ; 2,62 (t, 2H) ; 2,57 (s, 6H) ; 2,41 (m, 4H) ; 1,16 (t, 6H).

**PREPARATION CXXXII**

**Acide [2-[méthyl(phénylméthyl)amino]éthoxy]acétique, 1,1-diméthyléthyl ester**

En opérant de façon analogue à la préparation II, au départ de 2-[méthyl(phénylméthyl)amino]éthanol, on obtient le produit attendu sous forme d'une huile incolore (rendement = 35 %).

RMN $^1H$ (250 MHz, DMSO) $\delta$ : 7,26 (m, 5H) ; 3,96 (s, 2H) ; 3,57 (t, 2H) ; 3,49 (s, 2H) ; 2,49 (m, 2H) ; 2,14 (s, 3H) ; 1,40 (s, 9H).

**PREPARATION CXXXIII**

**Acide [2-[méthyl(phénylméthyl)amino]éthoxy]acétique, sel de lithium**

Une solution de 1g (3,58 mM) du composé obtenu selon la préparation CXXXII dans 10 ml de tétrahydrofurane est chauffée au reflux du solvant en présence de 165 mg de lithine et 3 ml d'eau, pendant 8 heures. Le mélange réactionnel est ensuite concentré sous pression réduite pour donner le produit attendu sous forme d'une mousse incolore (rendement = 99 %).

RMN $^1H$ (250 MHz, DMSO) $\delta$ : 7,26 (m, 5H) ; 3,59 (s, 2H) ; 3,53 (t, 2H) ; 3,48 (s, 2H) ; 2,50 (t, 2H) ; 2,12 (s, 3H).

**PREPARATION CXXXIV**

***N*-méthyl-*N*-[2-[2-[4-(1-méthyl-4-pipéridinyl)-1-pipérazinyl]-2-oxoéthoxy]éthyl]-benzèneméthanamine**

En opérant de façon analogue à l'exemple 5, au départ du sel obtenu selon la préparation CXXXIII et de 1-(1-méthyl-4-pipéridinyl)pipérazine, on obtient le produit attendu sous forme d'une huile jaune (rendement = 24 %).

RMN $^1H$ (250 MHz, DMSO) $\delta$: 7,26 (m, 5H) ; 4,09 (s, 2H) ; 3,54 (t, 2H) ; 3,49 (s, 2H) ; 3,37 (m, 4H) ; 2,78 (m, 2H) ; 2,53 (t, 2H) ; 2,43 (m, 4H) ; 2,41 (s, 3H) ; 2,11 (s, 3H) ; 2,07 (m, 1H) ; 1,80 (dt, 2H) ; 1,65 (dm, 2H) ; 1,39 (dq, 2H).

## PREPARATION CXXXIV

### *N*-méthyl-2-[2-[4-(1-méthyl-4-pipéridinyl)-1-pipérazinyl]-2-oxoéthoxy]éthanamine

En opérant de façon analogue à la préparation XXXV, au départ du composé obtenu selon la préparation CXXXIV, on obtient le produit attendu sous forme d'une huile jaune (rendement = 80 %).
RMN $^1$H (300 MHz, DMSO) δ: 4,16 (s, 2H) ; 3,55 (t, 2H) ; 3,49 (m, 1H) ; 3,39 (m, 2H) ; 3,33 (m, 2H) ; 2,77 (t, 4H) ; 2,43 (m, 2H) ; 2,38 (s, 3H) ; 2,16 (m, 2H) ; 2,12 (s, 3H) ; 1,82 (dt, 2H) ; 1,67 (dm, 2H) ; 1,38 (dq, 2H).

## PREPARATION CXXXVI

### Chlorure de 2,6-dichloro-4-fluorobenzènesulfonyle

On ajoute, à -15°C, une solution de 4,75g (25 mM) de 2,6-dichloro-4-fluoroaniline dans 50 ml de dichlorométhane à 4,75 ml d'éthérate de trifluorure de bore. On ajoute 5 ml de tétrahydrofurane pour dissoudre le précipité formé, puis, doucement, 3,6 ml de nitrite de t-butyle en solution dans 25 ml de dichlorométhane. Le mélange réactionnel est agité 10 min à -15 °C puis 20 min à +5°C. On ajoute 200 ml de pentane, on maintient sous agitation à 0°C pendant 30 min puis filtre le précipité. Après séchage on obtient 7,2 g du sel de diazonium. Ce sel est dissout dans 30 ml d'acétonitrile et ajouté, à 10 °C, à un mélange d'une solution d'anhydride sulfureux dans 90 ml d'acide acétique à laquelle on a ajouté 1,4 g de chlorure cuivrique anhydre et 23 ml d'acide chlorhydrique concentré. Le mélange réactionnel est agité 30 min à température ambiante puis concentré sous pression réduite. Le résidu d'évaporation est repris dans 60 ml de dichlorométhane et les sels insolubles sont éliminés par filtration. Le filtrat est concentré sous pression réduite pour donner 4,71 g du produit attendu sous forme de cristaux orange (rendement = 71 %).
F = 57°C

## Exemple 54

### N-[2-[2-[4-(1-méthyl-4-pipéridinyl)-1-pipérazinyl]-2-oxo-éthoxy]éthyl]-2,6-dichloro-4-fluoro-N-méthyl-benzènesulfonamide

En opérant de façon analogue à la préparation I, au départ des composés obtenus selon les préparations CXXXV et CXXXVI, on obtient le produit attendu sous forme d'une huile jaune pâle (rendement = 73 %).
RMN $^1$H (250 MHz, DMSO) δ : 7,73 (d, 2H) ; 4,08 (s, 2H) ; 3,58 (t, 2H) ; 3,45 (t, 2H) ; 3,40 (m, 2H) ; 3,30 (m, 2H) ; 2,93 (s, 3H) ; 2,78 (m, 2H) ; 2,41 (m, 4H) ; 2,13 (m, 1H) ; 2,11 (s, 3H) ; 1,81 (t, 2H) ; 1,75 (m, 2H) ; 1,40 (m, 2H).

## Exemple 55

### N-[2-[2-[4-(1-méthyl-4-pipéridinyl)-1-pipérazinyl]-2-oxo-éthoxy]éthyl]-2,6-dichloro-4-fluoro-N-méthyl-benzènesulfonamide, difumarate

En opérant de façon analogue à l'exemple 4, au départ du composé obtenu selon l'exemple 54, on obtient le produit attendu sous forme d'un solide blanc (rendement = 76 %).
F = 152-155 °C
En opérant de façon analogue aux exemples 54 et 55, au départ de chlorures de benzènesulfonyle diversement substitués, on obtient les composés selon l'invention suivants :

## Exemple 56

### N-[2-[2-[4-(1-méthyl-4-pipéridinyl)-1-pipérazinyl]-2-oxo-éthoxy]éthyl]-4-bromo-2,6-dichloro-N-méthyl-benzènesulfonamide

Huile incolore (rendement = 37 %).
RMN $^1$H (300 MHz, DMSO) δ : 7,97 (s, 2H) ; 4,07 (s, 2H) ; 3,58 (t, 2H) ; 3,45 (t, 2H) ; 3,43 (m, 2H) ; 2,99 (s, 3H) ; 2,74 (m, 2H) ; 2,42 (m, 4H) ; 2,14 (m, 1H) ; 2,13 (s, 3H) ; 1,81 (t, 2H) ; 1,76 (m, 2H) ; 1,44 (m, 2H).

**Exemple 57**

**N-[2-[2-[4-(1-méthyl-4-pipéridinyl)-1-pipérazinyl]-2-oxo-éthoxy]éthyl]-4-bromo-2,6-dichloro-N-méthyl-benzènesulfonamide, difumarate**

Solide blanc (rendement = 87 %).
F=194˚C

**Exemple 58**

**N-[2-[2-[4-(1-méthyl-4-pipéridinyl)-1-pipérazinyl]-2-oxo-éthoxy]éthyl]-2,4,6-trichloro-N-méthyl-benzènesulfonamide**

Huile incolore (rendement = 64%).
RMN $^1$H (300 MHz, DMSO) δ : 7,88 (s, 2H) ; 4,08 (s, 2H) ; 3,58 (t, 2H) ; 3,44 (t, 2H) ; 3,38 (m, 4H) ; 2,94 (s, 3H) ; 2,78 (m, 2H) ; 2,42 (m, 4H) ; 2,17 (m, 1H) ; 2,11 (s, 3H) ; 1,81 (t, 2H) ; 1,76 (m, 2H) ; 1,44 (m, 2H).

**Exemple 59**

**N-[2-[2-[4-(1-méthyl-4-pipéridinyl)-1-pipérazinyl]-2-oxo-éthoxy]éthyl]-2,4,6-trichloro-N-méthyl-benzènezulfonamide, difumarate**

Solide blanc (rendement = 82 %).
F=194˚C

**Exemple 60**

**N-[2-[2-[4-(1-méthyl-4-pipéridinyl)-1-pipérazinyl]-2-oxo-éthoxy]éthyl]-2,4-dichloro-6-méthyl-N-méthyl-benzènesulfonamide**

Huile incolore (rendement = 61 %).
RMN $^1$H (300 MHz, DMSO) δ : 7,70 (d, 1H) ; 7,56 (d, 1H) ; 4,07 (s, 2H) ; 3,57 (t, 2H) ; 3,37 (m, 2H) ; 3,30 (s, 4H) ; 2,85 (s, 3H) ; 2,74 (m, 2H) ; 2,62 (s, 3H) ; 2,42 (m, 4H) ; 2,13 (m, 1H) ; 2,11 (s, 3H) ; 1,81 (t, 2H) ; 1,77 (m, 2H) ; 1,43 (m, 2H).

**Exemple 61**

**N-[2-[2-[4-(1-méthyl-4-pipéridinyl)-1-pipérazinyl]-2-oxo-éthoxy]éthyl]-2,4-dichloro-6-méthyl-N-méthyl-benzènesulfonamide, difumarate**

Solide blanc (rendement = 87 %).
F=190˚C

**Exemple 62**

**N-[2-[2-[4-(1-méthyl-4-pipéridinyl)-1-pipérazinyl]-2-oxo-éthoxy]éthyl]-4-méthoxy-2,3,6-triméthyl-N-méthyl-benzènesulfonamide**

Huile incolore (rendement = 34 %).
RMN $^1$H (250 MHz, DMSO) δ : 6,83 (s, 1H) ; 4,03 (s, 2H) ; 3,84 (s, 3H) ; 3,52 (t, 2H) ; 3,35 (m, 2H) ; 3,35 (m, 2H) ; 3,18 (t, 2H) ; 2,73 (m, 2H) ; 2,69 (s, 3H) ; 2,57 (s, 3H) ; 2,42 (s, 3H) ; 2,41 (t, 4H) ; 2,12 (s, 3H) ; 2,10 (m, 1H) ; 2,08 (s, 3H) ; 1,81 (m, 2H) ; 1,66 (m, 2H) ; 1,38 (m, 2H).

**Exemple 63**

**4-méthoxy-N,2,3,6-tétraméthyl-N-[2-[2-[4-(1-méthyl-4-pipéridinyl)-1-pipérazinyl]-2-oxo-éthoxy]éthyl]benzènesulfonamide, difumarate**

Solide blanc (rendement = 71 %).
F=180˚C

**PREPARATION CXXXVII**

**Acide 4-[[2-[[(4-méthoxy-2,6-diméthylphényl)sulfonyl] méthylamino]éthoxy]acétyl]-1-pipérazinecarboxylique 1,1-diméthyléthyl ester**

En opérant de façon analogue à l'exemple 5, au départ de N-Boc-pipérazine, on obtient le produit attendu qui est engagé dans l'étape suivante sans purification particulière.

**PREPARATION CXXXVIII**

**4-méthoxy-*N*,2,6-triméthyl-*N*-[2-[2-oxo-2-(1-pipérazinyl)éthoxy]éthyl] benzènesulfonamide**

En opérant de façon analogue à la préparation XXIX, au départ du composé obtenu suivant la préparation CXXXVII, on obtient le produit attendu sous forme d'une huile jaune (rendement = 98 %).
RMN $^1$H (250 MHz, DMSO) δ : 6,80 (s, 2H) ; 4,04 (s, 2H) ; 3,80 (s, 3H) ; 3,53 (t, 2H) ; 3,30 (m, 2H) ; 3,23 (t, 4H) ; 2,70 (s, 3H) ; 2,60 (t, 4H) ; 2,53 (s, 6H).

**PREPARATION CXXXIX**

**Acide 4-(3-oxopropyl)-pipéridinecarboxylique, phénylméthyl ester**

On refroidit à -50˚C 0,81 ml de diméthylsulfoxyde en solution dans 10 ml de dichlorométhane (DCM) et on ajoute 0,42 ml (4,8 mM) de chlorure d'oxalyle en solution dans 1,5 ml de DCM. Le mélange est agité pendant 10 min à -60 ˚C puis on ajoute goutte à goutte, à cette température, 1,21g (4,4 mM) d'ester benzylique de l'acide 4-(3-hydroxypropyl)pipéridinecarboxylique en solution dans 6 ml de DCM. Le milieu réactionnel est agité pendant 30 min à - 50˚C puis on ajoute goutte à goutte 3 ml de triéthylamine et on laisse remonter la température jusqu'à la température ambiante en 2 heures. Le mélange est hydrolysé sur 25 ml d'acide chlorhydrique N et extrait par du DCM. La phase organique est lavée à l'eau, séchée sur sulfate de magnésium et concentrée sous pression réduite. Le produit brut est purifié par chromatographie sur gel de silice en éluant à l'aide d'un mélange cyclohexane/acétate d'éthyle (75/25 ; v/v). On obtient ainsi le produit attendu sous forme d'une huile jaune (rendement = 83%).
RMN $^1$H (300 MHz, DMSO) δ : 9,66 (s, 1H) ; 7,35 (m, 5H) ; 5,05 (s, 2H) ; 3,98 (m, 2H) ; 2,75 (m, 2H) ; 2,44 (m, 2H) ; 1,64 (q, 2H) ; 1,63 (m, 2H) ; 1,37 (m, 1H) ; 1,02 (m, 2H).

**PREPARATION CXL**

**Acide 4-[3-[4-[[2-[[(4-méthoxy-2,6-diméthylphényl)sulfonyl]méthyl amino]éthoxy]acétyl-1-pipérazinyl]propyl]-1-pipéridinecarboxylique, phénylméthyl ester**

En opérant de façon analogue à la préparation XXVIII, au départ des composés obtenus suivant les préparations CXXXVIII et CXXXIX, on obtient le produit attendu sous forme d'une huile incolore (rendement = 51 %).
RMN $^1$H (300 MHz, DMSO) δ : 7,40 (m, 5H) ; 6,81 (s, 2H) ; 5,05 (s, 2H) ; 4,33 (s, 2H) ; 3,96 (m, 2H) ; 3,79 (s, 3H) ; 3,55 (t, 2H) ; 3,37 (m, 2H) ; 3,30 (m, 2H) ; 3,19 (t, 2H) ; 2,71 (m, 2H) ; 2,69 (s, 3H) ; 2,52 (s, 6H) ; 2,25 (m, 6H) ; 1,65 (m, 2H) ; 1,42 (m, 3H) ; 1,20 (m, 2H) ; 1,00 (m, 2H).

**Exemple 64**

**4-méthoxy-*N*,2,6-triméthyl-*N*-[2-[2-oxo-2-[4-[3-(4-pipéridinyl)propyl]-1-pipérazinyl]ethoxy]éthyl]benzènesulfonamide**

En opérant de façon analogue à la préparation XXXV, au départ du composé obtenu suivant la préparation

CXL, on obtient le produit attendu sous forme d'une huile incolore (rendement = 62 %).

RMN $^1H$ (250 MHz, DMSO) δ : 6,80 (s, 2H) ; 4,05 (s, 2H) ; 3,80 (s, 3H) ; 3,53 (t, 2H) ; 3,30 (m, 4H) ; 3,19 (t, 2H) ; 2,88 (m, 2H) ; 2,69 (s, 3H) ; 2,50 (s, 6H) ; 2,44 (m, 2H) ; 2,27 (m, 6H) ; 1,57 (m, 2H) ; 1,41 (m, 3H) ; 1,17 (m, 2H) ; 0,97 (m, 2H).

## Exemple 65

### 4-méthoxy-*N*,2,6-triméthyl-*N*-[2-[2-oxo-2-[4-[3-(1-méthyl-4-pipéridinyl)propyl]-1-pipérazinyl]éthoxy]éthyl]benzènesulfonamide

En opérant de façon analogue à la préparation XXVIII, au départ du composé obtenu suivant l'exemple 64, on obtient le produit attendu sous forme d'une huile incolore (rendement = 90 %).

RMN $^1H$ (250 MHz, DMSO) δ : 6,80 (s, 2H) ; 4,05 (s, 2H) ; 3,80 (s, 3H) ; 3,53 (t, 2H) ; 3,38 (m, 2H) ; 3,24 (t, 2H) ; 3,18 (m, 2H) ; 2,72 (m, 2H) ; 2,69 (s, 3H) ; 2,50 (s, 6H) ; 2,28 (m, 6H) ; 2,11 (s, 2H) ; 1,78 (m, 2H) ; 1,60 (m, 2H) ; 1,45 (m, 2H) ; 1,15 (m, 5H).

## Exemple 66

### 4-méthoxy-*N*,2,6-triméthyl-*N*-[2-[2-oxo-2-[4-[3-(1-méthyl-4-pipéridinyl)propyl]-1-pipérazinyl]éthoxy]éthyl]benzènesulfonamide, difumarate

En opérant de façon analogue à l'exemple 4, au départ du composé obtenu suivant l'exemple 65, on obtient le produit attendu sous forme d'un solide blanc (rendement = 98 %).
F = 50˚C

## PREPARATION CXLI

### Acide 4-[4-(phénylméthyl)-1-pipérazinyl]-1-pipéridinecarboxylique, 1,1-diméthyléthyl ester

En opérant de façon analogue à la préparation XXVIII, au départ de N-benzylpipérazine et de N-Boc-4-pipéridinone, on obtient le produit attendu sous forme d'une huile incolore (rendement = 60 %).

RMN $^1H$ (300 MHz, CDCl3) δ : 7,27 (m, 5H) ; 4,15 (m, 2H) ; 3,52 (s, 2H) ; 2,61 (m, 11H) ; 1,81 (m, 2H) ; 1,44 (s, 9H) ; 1,41 (m, 2H).

## PREPARATION CXLII

### Acide 4-(1-pipérazinyl)-pipéridinecarboxylique, 1,1-diméthyléthyl ester

On prépare une solution de 15,5 g (43,1 mM) du composé obtenu selon la préparation CXLI dans 46 ml d'éthanol et on ajoute 3,1 g d'hydroxyde de palladium, puis après inertage du réacteur, 17,7 g (215 mM) de cyclohexène. Le mélange réactionnel est agité à 50 ˚C pendant 6 heures puis refroidi et filtré. Le filtrat est concentré sous pression réduite et l'on obtient 10,02 g du produit attendu sous forme d'une huile incolore(rendement = 86 %).

RMN $^1H$ (300 MHz, CDCL3) δ : 4,12 (m, 2H) ; 2,92 (m, 4H) ; 2,69 (m, 2H) ; 2,55 (t, 4H) ; 2,36 (m, 1H) ; 1,79 (m, 2H) ; 1,45 (s, 9H) ; 1,38 (m, 2H).

## PREPARATION CXLIII

### Acide 4-[4-[[2-[[(4-méthoxy-2,6-diméthylphényl)sulfonyl]méthylamino éthoxy]acétyl]-1-pipérazinyl]-1-pipéridinecarboxylique, 1,1-diméthyléthyl ester

En opérant de façon analogue à l'exemple 3, au départ des composés obtenus suivant les préparations III et CXLII, on obtient le produit attendu sous forme d'une huile incolore (rendement = 32 %).

RMN $^1H$ (300 MHz, CD3CN) δ : 6,74 (s, 2H) ; 4,07 (m, 2H) ; 4,00 (s, 2H) ; 3,80 (s, 3H) ; 3,54 (t, 2H) ; 3,43 (m, 2H) ; 3,26 (m, 4H) ; 2,74 (s, 3H) ; 2,69 (m, 2H) ; 2,57 (s, 6H) ; 2,44 (m, 4H) ; 2,34 (m, 1H) ; 1,71 (m, 2H) ; 1,41 (s, 9H) ; 1,29 (m, 2H).

**Exemple 67**

**4-méthoxy-*N*,2,6-triméthyl-*N*-[2-[2-oxo-2-[4-(4-pipéridinyl)-1-pipérazinyl]éthoxy]éthyl]benzènesulfonamide, bis(trifluoroacétate)**

On prépare un mélange de 140 mg (0,24 mM) du composé obtenu selon la préparation CXLIII dans 1 ml de dichlorométhane et 0,7 ml d'acide trifluoroacétique. Le mélange réactionnel est agité à température ambiante pendant 2 heures puis concentré sous pression réduite. Le résidu d'évaporation est repris dans 3 ml d'eau et lyophilysé. On obtient ainsi 160 mg du produit attendu sous forme d'un solide blanc (rendement = 95 %).
F = 62°C

**PREPARATION CXLIV**

**Acide 4-(6-nitro-3-pyridinyl)-1-pipérazinecarboxylique, 1,1-diméthyléthyl ester**

On prépare une solution de 1 g (4,9 mM) de 2-nitro-5-bromopyridine et 917 mg (4,9 mM) de N-Boc-pipérazine dans 10 ml de diméthylformamide et on ajoute 1,02 g (7,4 mM) de carbonate de potassium. Le mélange réactionnel est agité à 120 °C pendant 36 heures puis refroidi. On ajoute 50 ml d'eau et extrait par l'acétate d'éthyle. La phase organique obtenue est lavée à l'eau, séchée et concentrée sous pression réduite. Le produit brut est purifié par chromatographie sur gel de silice en éluant à l'aide d'un mélange toluène/isopropanol (98/2 ; v/v). On obtient ainsi le produit attendu sous forme d'un solide jaune (rendement = 33%).
RMN $^1H$ (250 MHz, DMSO) δ : 8,24 (d, 1H) ; 8,17 (d, 1H) ; 7,46 (dd, 1H) ; 3,50 (m, 8H) ; 1,42 (s, 9H).

**PREPARATION CXLV**

**1-(6-nitro-3-pyridinyl)pipérazine, bis(trifluoroacétate)**

En opérant de façon analogue à l'exemple 67, au départ du composé obtenu suivant la préparation CXLIV, on obtient le produit attendu sous forme d'un solide jaune (rendement = 97 %).
F = 188-190 °C

**PREPARATION CXLVI**

**4-méthoxy-*N*,2,6-triméthyl-*N*-[2-[2-oxo-2-[4-(6-nitro-3-pyridinyl)-1-pipérazinyl]éthoxy]éthyl]benzènesulfonamide**

En opérant de façon analogue à l'exemple 3, au départ des composés obtenus suivant les préparations III et CXLV, on obtient le produit attendu sous forme d'un solide jaune (rendement = 82 %).
F = 118-120 °C

**Exemple 68**

**4-méthoxy-*N*,2,6-triméthyl-*N*-[2-[2-oxo-2-[4-(6-amino-3-pyridinyl)-1-pipérazinyl]éthoxy]éthyl]benzènesulfonamide**

On prépare une suspension de 580 mg (1,11 mM) du composé obtenu suivant la préparation CXLVI dans 20 ml de méthanol et on ajoute 58 mg de charbon palladié à 10 %. Le mélange réactionnel est agité à température ambiante pendant 6 heures sous atmosphère d'hydrogène à pression atmosphérique puis filtré et concentré sous pression réduite. Le produit brut est purifié par chromatographie sur gel de silice en éluant à l'aide d'un mélange dichlorométhane/méthanol (97/3 ; v/v). On obtient ainsi le produit attendu sous forme d'un solide jaune pâteux (rendement = 81%).
RMN $^1H$ (300 MHz, DMSO) δ : 7,61 (d, 1H) ; 7,17 (dd, 1H) ; 6,80 (s, 2H) ; 6,40 (dd, 1H) ; 5,43 (s, 2H) ; 4,11 (s, 2H) ; 3,79 (s, 3H) ; 3,55 (m, 4H) ; 3,44 (m, 2H) ; 3,24 (t, 2H) ; 2,87 (m, 4H) ; 2,70 (s, 3H) ; 2,53 (s, 6H).

## Exemple 69

**4-méthoxy-*N*,2,6-triméthyl-*N*-[2-[2-oxo-2-[4-(6-amino-3-pyridinyl)-1-pipérazinyl]éthoxyléthyl]benzènesulfonamide, fumarate**

En opérant de façon analogue à l'exemple 4, au départ du composé obtenu suivant l'exemple 68, on obtient le produit attendu sous forme d'un solide beige (rendement = 97 %).
F = 194-196 °C

## Exemple 70

**N-[2-[2-[4-[2-(diméthylamino)-1,1-diméthyléthyl]-1-pipéridinyl]-2-oxoéthoxyl éthyl]-4-méthoxy-N,2,6-triméthylbenzènesulfonamide, trifluoroacétate**

On conditionne pendant 20 min, 460 mg de résine polystyrène greffée par une fonction cyclohexylcarbodiimide dans 5 ml de DCM. Le solvant est éliminé par filtration, puis on ajoute 100 mg (0,31 mM) d'acide [2-[[(4-méthoxy-2,6-diméthylphényl)sulfonyl]méthylamino]éthoxy]acétique en solution dans 3 ml de DCM, 37 mg (0,20 mM) de 4-[2-(diméthylamino)-1,1-diméthyléthyl]pipéridine et 2 mg de HOAT(1-hydroxy-7-azabenzotriazole). Le mélange est agité pendant 4 heures puis la résine est séparée par filtration et rincée par 4 ml de DCM. Les phases organiques rassemblées sont traitées par 50 mg de résine Amberlite IRA 400 ($OH^-$) pendant 3 heures, puis par 100 mg de résine polystyrène greffé isocyanate pendant 1 heure. La résine est éliminée par filtration et le filtrat est concentré sous pression réduite. Le produit obtenu est repris dans 0,5 ml d'acétonitrile et on ajoute 6 ml d'une solution à 1 % d'acide trifluoroacétique dans l'eau. Le mélange est filtré et lyophilisé. On obtient ainsi 59 mg du composé attendu sous forme d'un solide amorphe (rendement = 48 %).
F = 60°C

RMN $^1H$ (250 MHz, $CD_3CN$) δ : 6,74 (s, 2H) ; 4,50 (m, 1H) ; 4,06 (m, 2H) ; 3,80 (s, 3H) ; 3,78 (m, 1H) ; 3,57 (t, 2H) ; 3,31 (t, 2H) ; 3,04 (s, 2H) ; 2,85 (s, 6H) ; 2,83 (m, 1H) ; 2,73 (s, 3H) ; 2,56 (s, 6H) ; 2,55 (m, 1H) ; 1,65 (m, 2H) ; 1,45 (m, 1 H) , 1,20 (m, 2H) ; 0,99 (s, 6H).

## Exemple 71

**N-[2-[2-[4-[2-(diméthylamino)-1-hydroxyéthyl]-1-pipéridinyl]-2-ozoéthozy]éthyl]-4-méthoxy-N,2,6-triméthylbenzènesulfonamide, trifluoroacétate**

En opérant de façon analogue à l'exemple 70, au départ du composé obtenu selon la préparation LXIX, on obtient le produit attendu sous forme d'une pâte incolore (rendement = 45 %).

RMN $^1H$ (250 MHz, $CD_3CN$) δ : 6,74 (s, 2H) ; 4,45 (m, 1H) ; 4,05 (m, 2H) ; 3,80 (s, 3H) ; 3,75 (m, 2H) ; 3,59 (t, 2H) ; 3,28 (t, 2H) ; 3,06 (m, 2H) ; 2,90 (m, 1H) ; 2,84 (s, 3H) ; 2,80 (s, 3H) ; 2,77 (s, 3H) ; 2,56 (s, 6H) ; 2,50 (m, 1H) ; 1,80 (m, 1H) ; 1,60 (m, 2H) ; 1,20 (m, 2H).

## Exemple 72

**4-méthoxy-N,2,6-triméthyl-N-[2-[2-[4-(4-méthyl-1-pipérazinyl)-1-pipéridinyl]-2-oxoéthoxy]éthyl]benzènesulfonamide**

On prépare une suspension de 350 mg (1,06 mM) d'acide obtenu selon la préparation III dans 3 ml de DCM et on ajoute 243 mg (1,27 mM) d'EDCI (chlorhydrate de 1-(3-diméthylaminopropyl)-3-éthylcarbodiimide) et 173 mg (1,27 mM) de HOAT. Le mélange est agité pendant 30 min à température ambiante, puis on ajoute 232 mg (1,27 mM) de 1-méthyl-4-(4-pipéridinyl)pipérazine. Le milieu réactionnel est agité pendant 18 heures à température ambiante puis versé sur 10 ml d'eau et extrait par du DCM. La phase organique est lavée à l'eau, séchée et concentrée sous pression réduite. Le résidu est purifié par chromatographie sur gel de silice en éluant à l'aide d'un mélange DCM/méthanol (90/10 ; v/v). On obtient ainsi 449 mg du produit attendu sous forme d'une huile jaune (rendement = 86 %).

RMN $^1H$ (300 MHz, $CD_3CN$) δ : 6,74 (s, 2H) ; 4,35 (d, 1H) ; 4,01 (q, 2H) ; 3,81 (s, 3H) ; 3,69 (d, 1H) ; 3,56 (t, 2H) ; 3,26 (t, 2H) ; 2,89 (t, 1H) ; 2,75 (s, 3H) ; 2,58 (s, 6H) ; 2,40 (m, 10H) ; 2,17 (s, 3H) ; 1,75 (m, 2H) ; 1,27 (m, 2H).

**Exemple 73**

**4-méthoxy-N,2,6-triméthyl-N-[2-[2-[4-(4-méthyl-1-pipérazinyl)-1-pipéridinyl]-2-oxoéthoxy]éthyl]benzènesulfonamide, fumarate**

On dissout 404 mg (0,814 mM) du composé obtenu selon l'exemple 72 dans 5 ml de méthanol et on ajoute 95 mg (0,815 mM) d'acide fumarique. Le mélange est agité pendant 10 min puis concentré sous pression réduite. Le résidu est repris dans 10 ml d'eau et la solution est lyophilisée. On obtient ainsi le sel attendu (474 mg) sous forme d'une poudre blanche (rendement = 95 %).
F = 90 °C

En opérant de façon analogue aux exemples 72 et 73, au départ des acides et des dérivés de piperidine décrits précédemment ou connus de la littérature, on obtient les composés selon l'invention suivants :

**Exemple 74**

**4-méthoxy-N,2,6-triméthyl-N-[2-[2-[4-(1-méthyl-4-pipéridinyl)-1-pipéridinyl]-2-oxoéthoxy] éthyl]benzènesulfonamide, fumarate**

Solide blanc (rendement = 44 %).
F = 88-89 °C

**Exemple 75**

**N-cyclopropyl-4-méthoxy-2,6-diméthyl-N-[2-[2-[4-(4-méthyl-1-pipérazinyl)-1-pipéridinyl]-2-oxoéthoxy]éthyl] benzènesulfonamide, difumarate**

Solide blanc (rendement = 76 %).
F = 148 °C

**Exemple 76**

**4-méthoxy-N,2,6-triméthyl-N-[2-[2-[4-[2-(1-pyrrolidinyl)éthyl]-1-pipéridinyl]-2-oxoéthoxy]éthyl]benzènesulfonamide, fumarate**

Solide blanc (rendement = 87 %).
F = 55 °C

RMN $^1$H (300 MHz, CD3CN) δ : 8,50 (m large, 1H) ; 6,74 (s, 2H) ; 4,38 (m, 1H) ; 4,10 (d, 2H) ; 3,80 (s, 3H) ; 3,75 (m, 1H) ; 3,57 (m, 4H) ; 3,27 (t, 2H) ; 3,13 (m, 2H) ; 2,95 (m, 3H) ; 2,74 (s, 3H) ; 2,57 (s, 6H) ; 2,50 (m, 1H) ; 2,06 (m, 4H) ; 1,65 (m, 5H) ; 1,07 (m, 2H).

**Exemple 77**

**4-méthoxy-N,2,6-triméthyl-N-[2-[2-[4-[4-(1-méthyléthyl)-1-pipérazinyl]-1-pipéridinyl]-2-oxoéthoxy]éthyl]benzènesulfonamide, fumarate**

Solide blanc (rendement = 92 %).
F = 145 °C

**Exemple 78**

**N-éthyl-4-méthoxy-2,6-diméthyl-N-[2-[2-[4-[2-(1-pyrrolidinyl)éthyl]-1-pipéridinyl]-2-oxoéthoxy]éthyl]benzènesulfonamide, fumarate**

Solide blanc (rendement = 50 %).
F=50°C

**Exemple 79**

**N-cyclopropyl-4-méthoxy-2,6-diméthyl-N-[2-[2-[4-(2-(1-pyrrolidinyl)éthyl]-1-pipéridinyl]-2-oxoéthoxy] éthyl] benzènesulfonamide, fumarate**

Solide blanc (rendement = 96 %).
F = 50 °C

**Exemple 80**

***N*-[2-[2-[4-[2-(hexahydro-4-méthyl-1*H*-1,4-diazépin-1-yl)éthyl]-1-pipéridinyl]-2-oxoéthoxy]éthyl]-4-méthoxy-*N*, 2,6-triméthylbenzènesulfonamide, difumarate**

Solide blanc (rendement = 54 %).
F = 60 °C

**Exemple 81**

**4-méthoxy-*N*,2,6-triméthyl-*N*-[2-[2-[4-[2-[méthyl(1-méthyléthyl)amino]éthyl]-1-pipéridinyl]-2-oxoéthoxy]éthyl] benzènesulfonamide, fumarate**

Solide blanc (rendement = 64 %).
F = 60 °C

**Exemple 82**

**4-méthoxy-*N*,2,6-triméthyl-*N*-[2-[2-[4-[méthyl(1-méthyl-4-pipéridinyl) amino]-1-pipéridinyl]-2-oxoéthoxy]éthyl] benzènesulfonamide, fumarate**

Solide blanc (rendement = 66 %).
F = 72 °C

**Exemple 83**

**4-méthozy-*N*,2,6-triméthyl-*N*-[2-[2-[4-[1-(1-méthyléthyl)-4-pipéridinyl]-1-pipéridinyl]-2-oxoéthoxy]éthyl]benzènesulfonamide, fumarate**

Solide blanc (rendement = 84 %).
F = 62-64 °C

**Exemple 34**

**4-méthoxy-*N*,2,6-triméthyl-*N*-[2-[2-[4-(1-éthyl-4-pipéridinyl)-1-pipéridinyl]-2-oxoéthoxy]éthyl]benzènesulfonamide, fumarate**

Huile incolore (rendement = 35 %).
RMN $^{1}H$ (300 MHz, DMSO) δ : 6,80 (s, 2H) ; 6,51 (s, 2H) ; 4,32 (m, 1H) ; 4,06 (q, 2H) ; 3,80 (s, 3H) ; 3,67 (m, 1H) ; 3,53 (m, 2H) ; 3,22 (m, 2H) ; 3,15 (d, 2H) ; 2,86 (m, 1H) ; 2,69 (s, 3H) ; 2,61 (q, 2H) ; 2,53 (s, 6H) ; 2,45 (m, 1H) ; 2,27 (t, 2H) ; 1,66 (m, 4H) ; 1,30 (m, 3H) ; 1,07 (t, 3H) ; 0,96 (m, 3H).

**Exemple 85**

**4-méthoxy-*N*,2,6-triméthyl-*N*-[2-[2-[4-(1-cyclopropyl-4-pipéridinyl)-1-pipéridinyl]-2-oxoéthoxy]éthyl]benzènesulfonamide,** fumarate

Huile incolore (rendement = 63 %).
RMN $^{1}H$ (300 MHz, DMSO) δ : 6,80 (s, 2H) ; 6,66 (s, 2H) ; 4,34 (m, 1H) ; 4,01 (q, 2H) ; 3,79 (s, 3H) ; 3,66 (m, 1H) ; 3,54 (t, 2H) ; 3,21 (t, 2H) ; 2,98 (d, 2H) ; 2,82 (m, 1H) ; 2,69 (s, 3H) ; 2,53 (s, 6H) ; 2,43 (m, 1H) ; 2,10 (t, 2H) ; 1,60

(m, 5H) ; 1,23 (m, 1H) ; 1,03 (m, 5H) ; 0,38 (m, 2H) ; 0,29 (m, 2H).

**Exemple 86**

**4-méthoxy-*N*,2,6-triméthyl-*N*-[2-[2-[4-[2-(4-morpholinyl)éthyl]-1-pipéridinyl]-2-oxoéthoxy]éthyl]benzènesulfonamide, fumarate**

Solide blanc (rendement = 73 %).
F = 50 °C

**Exemple 87**

**4-méthoxy-*N*,2,6-triméthyl-*N*-[2-[2-[4-[1,1-diméthyl-2-(1-azétidinyl)éthyl]-1-pipéridinyl]-2-oxoéthoxyléthyl]benzènesulfonamide, fumarate**

Solide blanc (rendement = 67 %).
F = 60-62 °C

**Exemple 88**

***N*-éthyl-4-méthoxy-2,6-diméthyl-*N*-[2-[2-[4-(1-méthyl-4-pipéridinyl)-1-pipéridinyl]-2-oxoéthoxy]éthyl]enzènesulfonamide, fumarate**

Solide blanc (rendement = 67 %).
F = 65°C

**Exemple 39**

***N*-[2-[2-[4-(hexahydro-4-méthyl-1*H*-1,4-diazépin-1-yl)-1-pipéridinyl]-2-oxoéthoxy]éthyl]-4-méthoxy-N,2,6-triméthyl-benzènesulfonamide, fumarate**

Huile jaune (rendement = 61 %).
RMN $^1$H (300 MHz, DMSO) δ : 6,80 (s, 2H) ; 6,50 (s, 2H) ; 4,36 (m, 1H) ; 4,11 (m, 2H) ; 3,80 (s, 3H) ; 3,73 (m, 1H) ; 3,55 (t, 2H) ; 3,24 (t, 2H) ; 2,87 (m, 2H) ; 2,85 (m, 6H) ; 2,69 (s, 3H) ; 2,67 (m, 1H) ; 2,53 (s, 6H) ; 2,46 (s, 3H) ; 2,42 (m, 1H) ; 1,81 (m, 2H) ; 1,69 (m, 2H) ; 1,21 (m, 2H).

**Exemple 90**

***N*-cyclopropyl-4-méthoxy-2,6-diméthyl-*N*-[2-[2-[4-(1-méthyl-4-pipéridinyl)-1-pipéridinyl]-2-oxoéthoxy]éthyl] benzènesulfonamide, fumarate**

Solide blanc (rendement = 60 %).
F = 80°C

**Exemple 91**

**2,4-dichloro-*N*,3-diméthyl-*N*-[2-[2-[4-(1-méthyl-4-pipéridinyl)-1-pipéridinyl]-2-oxoéthoxy]éthyl]benzènesulfonamide, fumarate**

Solide blanc (rendement = 39 %).
F = 86-88°C

**Exemple 92**

**4-méthoxy-*N*,2,6-triméthyl-*N*-[2-[2-[4-[2-(1-azétidinyl)éthyl]-1-pipéridinyl]-2-oxoéthoxy]éthyl]benzènesulfonamide,** fumarate

Solide blanc (rendement = 40 %).

RMN $^1H$ (300 MHz, DMSO) δ : 6,80 (s, 2H) ; 6,52 (s, 2H) ; 4,28 (m, 1H) ; 4,05 (m, 2H) ; 3,80 (s, 3H) ; 3,67 (m, 1H) ; 3,57 (m, 6H) ; 3,20 (t, 2H) ; 2,85 (m, 1H) ; 2,78 (t, 2H) ; 2,69 (s, 3H) ; 2,53 (s, 6H) ; 2,49 (m, 1H) ; 2,17 (quin, 2H) ; 1,59 (m, 2H) ; 1,50 (m, 1H) ; 1,30 (m, 2H) ; 0,93 (m, 2H).

## Exemple 93

**2,6-dichloro-4-méthoxy-*N*-méthyl-*N*-[2-[2-[4-[2-(diméthylamino)éthyl]-1-pipéridinyl]-2-oxoéthoxy]éthyl]benzènesulfonamide, fumarate**

Solide blanc (rendement = 39 %).
F = 70°C

## Exemple 94

**2,6-dichloro-4-méthoxy-*N*-méthyl-*N*-[2-[2-[4-(1-méthyl-4-pipéridinyl)-1-pipéridinyl]-2-oxoéthoxy]éthyl]benzènesulfonamide, fumarate**

Solide blanc (rendement = 70 %).
F = 67°C

## Exemple 95

**4-méthoxy-*N*,2,6-triméthyl-*N*-[2-[2-[4-[(1-pyrrolidinyl)méthyl]-1-pipéridinyl]-2-oxoéthoxy]éthyl]benzènesulfonamide, fumarate**

Huile incolore (rendement = 30 %).

RMN $^1H$ (250 MHz, DMSO) δ: 6,80 (s, 2H) ; 6,54 (s, 2H) ; 4,29 (m, 1H) ; 4,15 (m, 2H) ; 3,80 (s, 3H) ; 3,63 (m, 1 H) ; 3,53 (t, 2H) ; 3,22 (t, 2H) ; 2,89 (m, 1H) ; 2,80 (m, 4H) ; 2,70 (s, 3H) ; 2,56 (m, 1H) ; 2,53 (s, 6H) ; 2,51 (m, 2H) ; 1,81 (m, 7H) ; 0,91 (m, 2H).

## Exemple 96

**4-méthoxy-*N*,2,6-triméthyl-*N*-[2-[2-[4-[(4-éthyl-1-pipérazinyl)méthyl]-1-pipéridinyl]-2-oxoéthoxy]éthyl]benzènesulfonamide, fumarate**

Solide blanc (rendement = 31 %).
F =120°C

## Exemple 97

***N*-cyclopropyl-4-méthoxy-2,6-diméthyl-*N*-[2-[2-[4-[(4-méthyl-1-pipérazinyl)méthyl]-1-pipéridinyl]-2-oxoéthoxy]éthyl]benzènesulfonamide, difumarate**

Solide blanc (rendement = 44 %).
F =160°C

## Exemple 98

***N*-éthyl-4-méthoxy-2,6-diméthyl-*N*-[2-[2-[4-[(4-méthyl-1-pipérazinyl)méthyl]-1-pipéridinyl]-2-oxoéthoxy]éthyl] benzènesulfonamide, difumarate**

Solide blanc (rendement = 49 %).
F = 98°C

**Exemple 99**

***N*-méthyl-4-méthoxy-2,6-dichloro-*N*-[2-[2-[4-[(4-méthyl-1-pipérazinyl)méthyl]-1-pipéridinyl]-2-oxoéthoxy] éthyl]benzènesulfonamide, difumarate**

Solide blanc (rendement = 50 %).
F =103˚C

**Exemple 100**

**4-méthoxy-*N*,2,6-triméthyl-*N*-[2-[2-[4-[1,1-diméthyl-2-(1-pipéridinyl)éthyl]-1-pipéridinyl]-2-oxoéthoxy]éthyl] benzènesulfonamide, trifluoroacétate**

Huile incolore (rendement = 47 %).
RMN $^1H$ (300 MHz, $CD_3CN$) δ : 7,50 (m large, 1H) ; 6,74 (s, 2H) ; 4,49 (m, 1H) ; 4,01 (q, 2H) ; 3,81 (s, 3H) ; 3,78 (m, 1H) ; 3,57 (t, 2H) ; 3,43 (m, 2H) ; 3,28 (t, 2H) ; 3,01 (m, 2H) ; 2,98 (s, 2H) ; 2,90 (m, 1H) ; 2,74 (s, 3H) ; 2,63 (s, 6H) ; 2,45 (m, 1H) ; 1,85 (m, 3H) ; 1,66 (m, 3H) ; 1,51 (m, 2H) ; 1,19 (m, 3H) ; 1,00 (s, 6H).

**Exemple 101**

**4-méthoxy-*N*,2,6-triméthyl-*N*-[2-[2-[4-[1,1-diméthyl-2-(1-pyrrolidinyl)éthyl]-1-pipéridinyl]-2-oxoéthoxyléthyl] benzènesulfonamide, fumarate**

Solide blanc (rendement = 61 %).
F = 50 ˚C

**Exemple 102**

***N*-[2-[2-[4-[2-(éthylméthylamino)éthyl]-1-pipéridinyl]-2-oxoéthoxyléthyl]-4-méthoxy-*N*,2,6-triméthylbenzènesulfonamide, fumarate**

Solide blanc (rendement = 46 %).
RMN $^1H$ (300 MHz, DMSO) δ: 6,80 (s, 2H) ; 6,51 (s, 2H) ; 4,27 (m, 1H) ; 4,03 (q, 2H) ; 3,80 (s, 3H) ; 3,55 (m, 1H) ; 3,24 (t, 2H) ; 3,22 (t, 2H) ; 2,86 (m, 1H) ; 2,70 (s, 3H) ; 2,62 (m, 4H) ; 2,53 (s, 6H) ; 2,48 (m; 1H) ; 2,46 (s, 3H) ; 1,66 (m, 2H) ; 1,52 (m, 1H) ; 1,45 (m, 2H) ; 1,38 (t, 3H) ; 0,97 (m, 2H).

**Exemple 103**

***N*-[2-[2-[4-[2-(diéthylamino)éthyl]-1-pipéridinyl]-2-oxoéthoxy]éthyl]-4-méthoxy-*N*,2,6-triméthylbenzènesulfonamide, fumarate**

Solide blanc (rendement = 52 %).
F = 60˚C

**Exemple 104**

**4-méthoxy-*N*-(1-méthyléthyl)-2,6-diméthyl-*N*-[2-[2-[4-[2-(1-pyrrolidinyl) éthyl]-1-pipéridinyl]-2-oxoéthoxyléthyl]benzènesulfonamide, fumarate**

Solide blanc (rendement = 61 %).
F = 56˚C

**Exemple 105**

**4-méthoxy-*N*,2,6-triméthyl-*N*-[2-[2-[4-[1,1-diméthyl-2-(4-morpholinyl)éthyl]-1-pipéridinyl]-2-oxoéthoxy]éthyl] benzènesulfonamide, fumarate**

Pâte blanche (rendement = 50 %).

RMN $^1H$ (300 MHz, DMSO) δ : 6,80 (s, 2H) ; 6,62 (s, 2H) ; 4,38 (m, 1H) ; 4,04 (q, 2H) ; 3,79 (s, 3H) ; 3,55 (m, 1H) ; 3,52 (m, 6H) ; 3,21 (m, 2H) ; 2,81 (m, 1H) ; 2,70 (s, 3H) ; 2,53 (s, 6H) ; 2,49 (m, 4H) ; 2,47 (m, 1H) ; 2,09 (s, 2H) ; 1,60 (m, 2H) ; 1,43 (m, 1H) ; 1,10 (m, 2H) ; 0,76 (s, 6H).

## Exemple 106

***N*-[2-[2-[4-[2-(hexahydro-4-méthyl-1*H*-1,4-diazépin-1-yl)éthyl]-1-pipéridinyl]-2-oxoéthoxyléthyl]-4-méthoxy-*N*,2,6-triméthylbenzènesulfonamide, difumarate**

Solide blanc (rendement = 36 %).
F = 125 °C

## Exemple 107

***N*-[2-[2-[4-[(hexahydro-4-méthyl-1*H*-1,4-diazépin-1-yl)méthyl]-1-pipéridinyl]-2-oxoéthoxy]éthyl]-4-méthoxy-*N*,2,6-triméthylbenzènesulfonamide, difumarate**

Solide blanc (rendement = 31 %).
F = 100-102 °C

## Exemple 108

**4-méthoxy-*N*-[2-[2-[4-[2-(1-méthyl-4-pipéridinyl)éthyl]-1-pipéridinyl]-2-oxoéthoxy]éthyl]-*N*,2,6-triméthylbenzènesulfonamide, fumarate**

Solide blanc (rendement = 24%).
F = 70°C

En opérant de façon analogue à l'exemple 70, au départ des acides et des dérivés de pipéridine décrits précédemment ou connus de la littérature, on obtient les composés selon l'invention suivants :

## Exemple 109

**4-méthoxy-*N*-[2-[2-[4-[2-(1-pipéridinyl)éthyl]-1-pipéridinyl]-2-oxoéthoxy]éthyl]-*N*,2,6-triméthylbenzènesulfonamide, fumarate**

Solide blanc (rendement = 69%).
F = 50°C

## Exemple 110

**4-méthoxy-*N*-[2-[2-[4-[2-(1-pyrrolidinyl)éthyl]-1-pipéridinyl]-2-oxoéthoxyl éthyl]-*N*- méthyl-2-trifluorométhylbenzènesulfonamide, trifluoroacétate**

Huile incolore (rendement = 99%).

RMN $^1H$ (300 MHz, CD3CN) δ : 8,20 (m large, 1H) ; 7,95 (d, 1H) ; 7,41 (d, 1H) ; 7,25 (dd, 1H) ; 4,43 (m, 1H) ; 4,10 (q, 2H) ; 3,91 (s, 3H) ; 3,63 (m, 1H) ; 3,63 (m, 4H) ; 3,43 (t, 2H) ; 3,13 (m, 2H) ; 2,96 (m, 2H) ; 2,90 (s, 3H) ; 2,60 (t, 1H) ; 2,06 (m, 6H) ; 1,71 (m, 4H) ; 1,60 (m, 2H).

## Exemple 111

**4-méthoxy-*N*-[2-[2-[4-[2-(1-méthyl-4-pipérazinyl)-2-oxoéthyl]-1-pipéridinyl]-2-oxoéthoxy]éthyl]-*N*,2,6-triméthylbenzènesulfonamide, fumarate**

Solide blanc (rendement = 33%).
F = 60°C

## Exemple 112

**4-méthoxy-*N*,2,6-triméthyl-*N*-[2-[2-[4-[1,1-diméthyl-2-(diéthylamino)éthyl]-1-pipéridinyl]-2-oxoéthoxy]éthyl] benzènesulfonamide, trifluoroacétate**

Huile incolore (rendement = 26 %).

RMN $^1$H (300 MHz, DMSO) δ : 8,12 (m large, 1H) ; 6,81 (s, 2H) ; 4,40 (m, 1H) ; 4,09 (q, 2H) ; 3,80 (s, 3H) ; 3,78 (m, 1H) ; 3,54 (t, 2H) ; 3,24 (t, 2H) ; 3,15 (m, 4H) ; 2,97 (d, 2H) ; 2,84 (t, 1H) ; 2,70 (s, 3H) ; 2,53 (s, 6H) ; 2,41 (m, 1H) ; 1,64 (m, 2H) ; 1,42 (m, 1H) ; 1,25 (m, 2H) ; 1,22 (t, 6H) ; 0,96 (s, 6H).

## Exemple 113

**4-méthoxy-*N*,2,6-triméthyl-*N*-[2-[2-[4-[(diméthylamino)méthyl]-1-pipéridinyl]-2-oxoéthoxy]éthyl]benzènesulfonamide, fumarate**

Solide blanc (rendement = 63 %).
F = 65°C

## Exemple 114

**4-méthoxy-*N*,2,6-triméthyl-*N*-[2-[2-[4-[(1-azétidinyl)méthyl]-1-pipéridinyl]-2-oxoéthoxy]éthyl]benzènesulfonamide, fumarate**

Solide blanc (rendement = 60 %).
F = 75°C

## Exemple 115

***N*,2,4,6-tétraméthyl-*N*-[2-[2-[4-(1-méthyl-4-pipéridinyl)-1-pipéridinyl]-2-oxoéthoxy]éthyl]benzènesulfonamide, trifluoroacétate**

Huile incolore (rendement = 97 %).

RMN $^1$H (250 MHz, CD3CN) δ : 9,40 (m large, 1H) ; 6,97 (s, 2H) ; 4,98 (m, 1H) ; 4,09 (q, 2H) ; 3,71 (m, 1H) ; 3,57 (t, 2H) ; 3,45 (d, 2H) ; 3,28 (t, 2H) ; 2,85 (m, 1H) ; 2,80 (m, 2H) ; 2,77 (s, 3H) ; 2,72 (d, 3H) ; 2,56 (s, 6H) ; 2,45 (m, 1H) ; 2,28 (s, 3H) ; 1,92 (m, 2H) ; 1,71 (m, 2H) ; 1,50 (m, 2H) ; 1,34 (m, 2H) ; 1,09 (m, 2H).

## Exemple 116

***N*-méthyl-*N*-[2-[2-[4-(1-méthyl-4-pipéridinyl)-1-pipéridinyl]-2-oxoéthoxy]éthyl]-2-trifluorométhyl-benzènesulfonamide, trifluoroacétate**

Huile incolore (rendement = 98 %).

RMN $^1$H (300 MHz, CD3CN) δ : 8,50 (m large, 1H) ; 7,97 (m, 2H) ; 7,77 (m, 2H) ; 4,48 (m, 1H) ; 4,13 (q, 2H) ; 3,80 (m, 1H) ; 3,64 (m, 2H) ; 3,49 (t, 2H) ; 3,42 (m, 2H) ; 2,96 (s, 3H) ; 2,90 (m, 1H) ; 2,78 (m, 2H) ; 2,72 (d, 3H) ; 2,50 (m, 1H) ; 1,93 (m, 2H) ; 1,73 (m, 2H) ; 1,38 (m, 4H) ; 1,36 (m, 2H).

## Exemple 117

**4-méthoxy-*N*-méthyl-*N*-[2-[2-[4-(1-méthyl-4-pipéridinyl)-1-pipéridinyl]-2-oxoéthoxy]éthyl]-2-trifluorométhyl-benzènesulfonamide, trifluoroacétate**

Huile incolore (rendement = 95 %).

RMN $^1$H (300 MHz, CD3CN) δ : 8,41 (m large, 1H) ; 7,96 (d, 1H) ; 7,40 (d, 1H) ; 7,25 (dd, 1H) ; 4,45 (m, 1H) ; 4,10 (q, 2H) ; 3,91 (s, 3H) ; 3,80 (m, 1H) ; 3,61 (t, 2H) ; 3,42 (m, 4H) ; 2,90 (s, 3H) ; 2,89 (m, 1H) ; 2,79 (m, 2H) ; 2,73 (d, 3H) ; 2,50 (m, 1H) ; 1,92 (m, 2H) ; 1,73 (m, 2H) ; 1,37 (m, 4H) ; 1,07 (m, 2H).

**Exemple 118**

***N*,2,4,6-tétraméthyl-*N*-[2-[2-[4-[2-(1-pyrrolidinyl)éthyl]-1-pipéridinyl]-2-oxoéthoxy]éthyl]benzènesulfonamide, trifluoroacétate**

Huile incolore (rendement = 99 %).
RMN $^1H$ (300 MHz, CD3CN) $\delta$ : 8,40 (m large, 1H) ; 7,03 (s, 2H) ; 4,37 (m, 1H) ; 4,02 (q, 2H) ; 3,75 (m, 1H) ; 3,58 (m, 4H) ; 3,31 (t, 2H) ; 3,10 (m, 2H) ; 2,91 (m, 3H) ; 2,75 (s, 3H) ; 2,56 (s, 6H) ; 2,54 (m, 1H) ; 2,29 (s, 3H) ; 2,10 (m, 4H) ; 1,61 (m, 5H) ; 1,10 (m, 2H).

**Exemple 119**

**2,6-dichloro-4-méthoxy-*N*-méthyl-*N*-[2-[2-[4-(1-méthyl-4-pipérazinyl)-1-pipéridinyl]-2-oxoéthoxy]éthyl]benzènesulfonamide, difumarate**

Solide blanc (rendement = 76 %).
F =176 ˚C

**Exemple 120**

**4-méthoxy-*N*,2,6-triméthyl-*N*-[2-[2-[4-[2-(diméthylamino)éthyl]-1-pipéridinyl]-2-oxoéthoxy]éthyl]benzènesulfonamide, fumarate**

On prépare une solution de 200 mg (0,6 mM) d'acide obtenu selon la préparation III dans 2 ml de chloroforme et 0,2 ml de diméthylformamide et on ajoute à température ambiante, 0,12 ml de chlorure d'oxalyle. Le mélange est agité pendant 1 heure à température ambiante et concentré sous pression réduite. Le résidu est repris dans 3 ml de chloroforme, on ajoute 103 mg (0,66 mM) de N,N-diméthyl-4-pipéridineéthanamine et 92 µl de triéthylamine, et on agite pendant 2 heures à température ambiante. Le milieu réactionnel est concentré sous pression réduite et le résidu est purifié par chromatographie sur gel de silice en éluant à l'aide d'un mélange dichlorométhane/méthanol/ammoniaque (95/5/0,5 ; v/v/v). On obtient 247 mg d'amide sous forme d'une huile que l'on salifie par l'acide fumarique selon le procédé appliqué à l'exemple 73 pour obtenir le sel sous forme d'un solide blanc (rendement = 83 %).
F = 55˚C

**Exemple 121**

**4-méthoxy-N,2,6-triméthyl-N-[2-[2-[4-(4-cyclopropyl-1-pipérazinyl)-1-pipéridinyl]-2-oxoéthoxy]éthyl]benzènesulfonamide, fumarate**

On met en suspension 2,2 g de résine tétrafluorophénol dans 10 ml de diméthylformamide et 40 ml de dichlorométhane et on ajoute 50 mg de 4-diméthylaminopyridine, 0,66 ml de diisopropylcarbodiimide et 1g (3,1 mM) d'acide obtenu selon la préparation III. Le mélange est agité à température ambiante pendant 16 heures, puis la résine est séparée par filtration et remise en réaction dans 40 ml de dichlorométhane avec 0,5 g (2,39 mM) de 1-cyclopropyl-4-(4-pipéridinyl)pipérazine, pendant 2 heures à température ambiante. Le mélange réactionnel est filtré et le filtrat est concentré sous pression réduite. Le produit brut obtenu est purifié par chromatographie sur gel de silice en éluant à l'aide d'un mélange dichlorométhane/méthanol (99/1 ; v/v). On obtient 700 mg d'amide sous forme d'une huile que l'on salifie par l'acide fumarique selon le procédé appliqué à l'exemple 73 pour obtenir le sel sous forme d'un solide blanc (rendement = 49 %).
F = 80˚C
En opérant de façon analogue à l'exemple 121, au départ des amines obtenues selon les préparations LXXXVIII et LXXIV, on obtient les composés selon l'invention suivants :

**Exemple 122**

**4-méthoxy-N,2,6-triméthyl-N-[2-[2-[4-[4-(1,1-diméthyléthyl)-1-pipérazinyl]-1-pipéridinyl]-2-oxoéthoxy]éthyl] benzènesulfonamide, difumarate**

Solide blanc (rendement = 48 %)
F =170 ˚C

**Exemple 123**

**4-méthoxy-N,2,6-triméthyl-N-[2-[2-[4-[(4-méthyl-1-pipérazinyl)méthyl]-1-pipéridinyl]-2-oxoéthoxy]éthyl]benzènesulfonamide, difumarate**

Solide blanc (rendement = 39 %)
F = 168°C

En opérant de façon analogue aux exemples 70 et 73, au départ de l'amine obtenue selon la préparation CVII, on obtient le composé selon l'invention suivant :

**Exemple 124**

**4-méthoxy-*N*,2,6-triméthyl-*N*-[2-[2-[4-[2-(4-méthyl-1-pipérazinyl)éthyl]-1-pipéridinyl]-2-oxoéthoxyléthyl]benzènesulfonamide, difumarate**

Solide blanc (rendement = 59 %)
F = 178-179 °C

**PREPARATION CXLVII**

**4-méthoxy-*N*,2,6-triméthyl-*N*-[2-[2-[4-(3-hydroxypropyl)-1-pipéridinyl]-2-oxoéthoxy]éthyl]benzènesulfonamide**

En opérant de façon analogue a l'exemple 72, au départ de 3-(4-pipéridinyl)propanol, on obtient le produit attendu sous forme d'une huile incolore (rendement = 79 %).

RMN $^1$H (300 MHz, DMSO) δ : 6,80 (s, 2H) ; 4,34 (t, 1H) ; 4,28 (dm, 1H) ; 4,03 (m, 2H) ; 3,79 (s, 3H) ; 3,64 (dm, 1H) ; 3,53 (t, 2H) ; 3,37 (d, 2H) ; 3,21 (t, 2H) ; 2,86 (t, 1H) ; 2,70 (s, 3H) ; 2,53 (s, 6H) ; 2,45 (m, 1H) ; 1,63 (d, 2H) ; 1,40 (m, 3H) ; 1,19 (m, 2H) ; 0,93 (m, 2H).

**PREPARATION CXLVIII**

**4-méthoxy-*N*,2,6-triméthyl-*N*-[2-[2-[4-[3-[[(4-méthylphényl)sulfonyl]oxyl propyl]-1-pipéridinyl]-2-oxoéthoxyléthyl]benzènesulfonamide**

On prépare une solution de 6,8 g (14,9 mM) du composé obtenu selon la préparation CXLVII dans 50 ml de dichlorométhane et on ajoute à température ambiante, 3,4 ml (24,6 mM) de triéthylamine, puis 4,68 g (24,6 mM) de chlorure de tosyle et 142 mg (1,5 mM) de chlorhydrate de triméthylamine. Le mélange est agité pendant 4 heure à reflux du solvant puis refroidi et hydrolysé sur 50 ml d'eau. La phase organique est lavée à l'eau, séchée sur sulfate de magnésium et concentrée sous pression réduite. Le résidu est purifié par chromatographie sur gel de silice en éluant à l'aide d'un mélange acétate d'éthyle/toluène (8/2 ; v/v). On obtient 5,6 g du composé attendu sous forme d'une huile incolore (rendement = 62 %).

RMN $^1$H (300 MHz, DMSO) δ : 7,79 (dd, 2H) ; 7,48 (d, 2H) ; 6,80 (s, 2H) ; 4,26 (m, 1H) ; 4,00 (m,, 3H) ; 3,79 (s, 3H) ; 3,62 (t, 2H) ; 3,51 (m, 2H) ; 3,23 (m, 2H) ; 2,85 (m, 1H) ; 2,70 (s, 3H) ; 2,53 (s, 6H) ; 2,45 (m, 1H) ; 2,41 (s, 3H) ; 1,55 (m, 3H) ; 1,29 (m, 2H) ; 1,13 (m, 2H) ; 0,89 (m, 2H).

**Exemple 125**

**4-méthoxy-*N*,2,6-triméthyl-*N*-[2-[2-[4-[3-(4-morpholinyl)propyl]-1-pipéridinyl]-2-oxoéthoxy]éthyl]benzènesulfonamide**

On prépare une solution de 400 mg (0,65 mM) du composé obtenu selon la préparation CXLVIII dans 8 ml de diméthylformamide et on ajoute à température ambiante, 90 mg (0,65 mM) de carbonate de potassium, puis 95 μl (1 mM) de morpholine. Le mélange est agité pendant 16 heure à 60°C puis refroidi, hydrolysé sur 20 ml d'eau et extrait par l'acétate d'éthyle. La phase organique est lavée à l'eau, séchée sur sulfate de magnésium et concentrée sous pression réduite. Le résidu est purifié par chromatographie sur gel de silice en éluant à l'aide d'un mélange dichlorométhane/méthanol (98/2 ; v/v). On obtient 150 mg du composé attendu sous forme d'une huile incolore (rendement = 44 %).

RMN $^1$H (300 MHz, DMSO) δ : 6,80 (s, 2H) ; 4,27 (m, 1H) ; 4,05 (q, 2H) ; 3,79 (s, 3H) ; 3,70 (m, 1H) ; 3,54 (m,

6H) ; 3,21 (t, 2H) ; 2,85 (m, 1H) ; 2,70 (s, 3H) ; 2,53 (s, 6H) ; 2,42 (m, 1H) ; 2,31 (m, 4H) ; 2,22 (t, 2H) ; 1,65 (m, 2H) ; 1,41 (m, 3H) ; 1,36 (m, 2H) ; 0,96 (m, 2H).

## Exemple 126

**4-méthoxy-*N*,2,6-triméthyl-*N*-[2-[2-[4-[3-(4-morpholinyl)propyl]-1-pipéridinyl]-2-oxoéthoxy]éthyl]benzènesulfonamide, fumarate**

En opérant de façon analogue a l'exemple 73, au départ du composé obtenu selon l'exemple 125, on obtient le produit attendu sous forme d'un solide blanc (rendement = 99 %).
F = 50°C

En opérant de façon analogue aux exemples 125 et 126, au départ de différentes amines, on obtient les composés selon l'invention suivants :

## Exemple 127

**4-méthoxy-*N*,2,6-triméthyl-*N*-[2-[2-[4-[3-(1-pyrrolidinyl)propyl]-1-pipéridinyl]-2-oxoéthoxyléthyl]benzènesulfonamide**

Huile incolore (rendement = 53%).

RMN $^1$H (300 MHz, DMSO) δ : 6,80 (s, 2H) ; 4,30 (m, 1H) ; 4,06 (q, 2H) ; 3,80 (s, 3H) ; 3,68 (m, 1H) ; 3,53 (t, 2H) ; 3,20 (t, 2H) ; 2,90 (m, 1H) ; 2,71 (s, 3H) ; 2,61 (m, 6H) ; 2,53 (s, 6H) ; 2,48 (m, 1H) ; 1,75 (s, 4H) ; 1,62 (m, 2H) ; 1,48 (m, 2H) ; 1,42 (m, 1H) ; 1,21 (m, 2H) ; 0,92 (m, 2H).

## Exemple 128

**4-méthoxy-*N*,2,6-triméthyl-*N*-[2-[2-[4-[3-(1-pyrrolidinyl)propyl]-1-pipéridinyl]-2-oxoéthoxyléthyl]benzènesulfonamide, fumarate**

Solide blanc (rendement = 92%).
F = 50°C

## Exemple 129

**4-méthoxy-*N*,2,6-triméthyl-*N*-[2-[2-[4-[3-(hexahydro-4-méthyl-1*H*-1,4-diazépin-1-yl)propyl]-1-pipéridinyl]-2-oxoéthoxy]éthyl]benzènesulfonamide** Huile incolore (rendement = 27%).

RMN $^1$H (300 MHz, DMSO) δ : 6,80 (s, 2H) ; 4,29 (m, 1H) ; 4,05 (q, 2H) ; 3,79 (s, 3H) ; 3,65 (m, 1H) ; 3,50 (t, 2H) ; 3,23 (t, 2H) ; 2,85 (m, 1H) ; 2,69 (s, 3H) ; 2,57 (m, 8H) ; 2,46 (t, 2H) ; 2,34 (s, 3H) ; 1,65 (m, 4H) ; 1,37 (m, 3H) ; 1,17 (m, 2H) ; 0,95 (m, 2H).

## Exemple 130

**4-méthoxy-*N*,2,6-triméthyl-*N*-[2-[2-[4-[3-(hexahydro-4-méthyl-1*H*-1,4-diazépin-1-yl)propyl]-1-pipéridinyl]-2-oxoéthoxy]éthyl]benzènesulfonamide, difumarate**

Solide blanc (rendement = 41 %).
F =174°C

## Exemple 131

**4-méthoxy-*N*,2,6-triméthyl-*N*-[2-[2-[4-[3-(4-méthyl-1-pipérazinyl)propyl]-1-pipéridinyl]-2-oxoéthoxy]éthyl]benzènesulfonamide**

Huile incolore (rendement = 43%).

RMN $^1$H (300 MHz, DMSO) δ : 6,80 (s, 2H) ; 4,27 (m, 1H) ; 4,03 (q, 2H) ; 3,79 (s, 3H) ; 3,65 (m, 1H) ; 3,52 (t, 2H) ; 3,23 (t, 2H) ; 2,85 (m, 1H) ; 2,69 (s, 3H) ; 2,53 (s, 6H) ; 2,48 (m, 1H) ; 2,29 (m, 8H) ; 2,23 (t, 2H) ; 2,12 (s, 3H) ; 1,63 (m, 2H) ; 1,38 (m, 3H) ; 1,17 (m, 2H) ; 0,95 (m, 2H).

**Exemple 132**

**4-méthoxy-*N*,2,6-triméthyl-*N*-[2-[2-[4-[3-(4-méthyl-1-pipérazinyl)propyl]-1-pipéridinyl]-2-oxoéthoxy]éthyl]benzènesulfonamide, difumarate**

Solide blanc (rendement = 89%).
F = 174°C

**Exemple 133**

**4-méthoxy-*N*,2,6-triméthyl-*N*-[2-[2-[4-[3-(1-azétidinyl)propyl]-1-pipéridinyl]-2-oxoéthoxy]éthyl]benzènesulfonamide**

Huile incolore (rendement = 23%).
RMN $^1$H (300 MHz, DMSO) δ: 6,80 (s, 2H) ; 4,26 (m, 1H) ; 4,02 (q, 2H) ; 3,80 (s, 3H) ; 3,68 (m, 1H) ; 3,54 (t, 2H) ; 3,21 (t, 2H) ; 3,03 (t, 4H) ; 2,85 (m, 1H) ; 2,70 (s, 3H) ; 2,53 (s, 6H) ; 2,48 (m, 2H) ; 2,47 (m, 1H) ; 2,25 (t, 2H) ; 1,91 (quin, 2H) ; 1,60 (m, 2H) ; 1,40 (m, 1H) ; 1,18 (m, 4H) ; 0,89 (m, 2H).

**Exemple 134**

**4-méthoxy-*N*,2,6-triméthyl-*N*-[2-[2-[4-[3-(1-azétidinyl)propyl]-1-pipéridinyl]-2-oxoéthoxy]éthyl]benzènesulfonamide, fumarate**

Solide blanc (rendement = 93%).
F = 65°C

**Exemple 135**

**4-méthoxy-*N*,2,6-triméthyl-*N*-[2-[2-[4-[3-(diméthylamino)propyl]-1-pipéridinyl]-2-oxoéthoxy]éthyl]benzènesulfonamide**

Huile incolore (rendement = 23%).
RMN $^1$H (250 MHz, DMSO) δ : 6,80 (s, 2H) ; 4,30 (m, 1H) ; 4,02 (q, 2H) ; 3,79 (s, 3H) ; 3,65 (m, 1H) ; 3,52 (t, 2H) ; 3,21 (t, 2H) ; 2,85 (m, 1H) ; 2,70 (s, 3H) ; 2,53 (s, 6H) ; 2,44 (m, 1H) ; 2,14 (t, 2H) ; 2,09 (s, 6H) ; 1,60 (m, 2H) ; 1,38 (m, 3H) ; 1,17 (m, 2H) ; 0,91 (m, 2H).

**Exemple 136**

**4-méthoxy-*N*,2,6-triméthyl-*N*-[2-[2-[4-[3-(diméthylamino)propyl]-1-pipéridinyl]-2-oxoéthoxy]éthyl]benzènesulfonamide, fumarate**

Solide blanc (rendement = 99%).
F = 55°C

**PREPARATION CIL**

**Acide 1'-[[2-[[(4-méthoxy-2,6-diméthylphényl)sulfonyl]méthylamino]éthoxy] acétyl] [4,4'-bipipéridine]-1-carboxylique, 1,1-diméthyléthyl ester**

En opérant de façon analogue a l'exemple 72, au départ du composé obtenu selon la préparation LXXXIX, on obtient le produit attendu sous forme d'une huile incolore (rendement = 88 %).
RMN $^1$H (250 MHz, DMSO) δ : 6,80 (s, 2H) ; 4,39 (m, 1H) ; 3,98 (m, 4H) ; 3,79 (s, 3H) ; 3,70 (m, 1H) ; 3,53 (t, 2H) ; 3,20 (t, 2H) ; 2,85 (m, 1H) ; 2,69 (s, 3H) ; 2,61 (m, 2H) ; 2,53 (s, 6H) ; 2,43 (m, 1H) ; 1,62 (m, 4H) ; 1,38 (s, 9H) ; 1,23 (m, 2H) ; 1,01 (m, 4H).

## Exemple 137

***N*-[2-(2-[4,4'-bipipéridin]-1-yl-2-oxoéthoxy)éthyl]-4-méthoxy-*N*,2,6-triméthylbenzènesulfonamide, trifluoroacétate**

En opérant de façon analogue a la préparation CXXI, au départ du composé obtenu selon la préparation CIL, on obtient le produit attendu sous forme d'une mousse blanche (rendement = 99 %).
RMN $^1$H (250 MHz, CD3CN) δ : 7,00 (m large, 1H) ; 6,74 (s, 2H) ; 4,48 (m, 1H) ; 4,05 (q, 2H) ; 3,80 (s, 3H) ; 3,75 (m, 1H) ; 3,57 (t, 2H) ; 3,35 (m, 2H) ; 3,30 (t, 2H) , 2,92 (m, 3H) ; 2,74 (s, 3H) ; 2,57 (s, 6H) ; 2,48 (m, 1H) ; 1,89 (m, 2H) ; 1,71 (m, 2H) ; 1,43 (m, 4H) ; 1,10 (m, 2H).

## PREPARATION CL

**Acide [2-[1-[[2-[[(4-méthoxy-2,6-diméthylphényl)sulfonyl]méthylamino] éthoxy]acétyl]-4-pipéridinyl]éthyl]méthyl-carbamique, 1,1-diméthyléthyl ester**

En opérant de façon analogue à l'exemple 72, au départ du composé obtenu selon la préparation CXIV, on obtient le produit attendu sous forme d'une huile incolore (rendement = 82 %).
RMN $^1$H (250 MHz, DMSO) δ : 6,80 (s, 2H) ; 4,32 (m, 1H) ; 4,04 (q, 2H) ; 3,80 (s, 3H) ; 3,69 (m, 1H) ; 3,56 (t, 2H) ; 3,21 (t, 2H) ; 3,19 (m, 2H) ; 2,85 (m, 1H) ; 2,74 (s, 3H) ; 2,70 (s, 3H) ; 2,53 (s, 6H) ; 2,46 (m, 1H) ; 1,65 (m, 2H) ; 1,38 (s, 9H) ; 1,34 (m, 3H) ; 1,01 (m, 2H).

## Exemple 138

**4-méthoxy-*N*,2,6-triméthyl-*N*-[2-[2-[4-[2-(méthylamino)éthyl]-1-pipéridinyl]-2-oxoéthoxy]éthyl]benzènesulfonamide**

En opérant de façon analogue à la préparation XXIX, au départ du composé obtenu selon la préparation CL, on obtient le produit attendu sous forme d'une huile incolore (rendement = 99 %).
RMN $^1$H (250 MHz, DMSO) δ : 6,80 (s, 2H) ; 4,25 (m, 1H) ; 4,05 (q, 2H) ; 3,80 (s, 3H) ; 3,65 (m, 1H) ; 3,55 (t, 2H) ; 3,19 (t, 2H) ; 2,85 (m, 1H) ; 2,70 (s, 3H) ; 2,51 (s, 6H) ; 2,48 (m, 3H) ; 2,24 (s, 3H) ; 1,59 (m, 2H) ; 1,52 (m, 1H) ; 1,34 (m, 2H) ; 0,92 (m, 2H).

## Exemple 139

**4-méthoxy-*N*,2,6-triméthyl-*N*-[2-[2-[4-[2-(méthylamino)éthyl]-1-pipéridinyl]-2-oxoéthoxy]éthyl]benzènesulfonamide, fumarate**

En opérant de façon analogue à l'exemple 73, au départ du composé obtenu selon l'exemple 138, on obtient le produit attendu sous forme d'un solide blanc (rendement = 99 %).
F = 50°C

## PREPARATION CLI

**Acide [1-[[2-[[(4-méthoxy-2,6-diméthylphényl)sulfonyl]méthylaminol éthoxy]acétyl]-4-pipéridinyl]carbamique, 1,1-diméthyléthyl ester**

En opérant de façon analogue à l'exemple 72, au départ de (4-pipéridinyl)carbamate de *t*-butyle, on obtient le produit attendu sous forme d'une huile incolore (rendement = 66 %).
RMN $^1$H (250 MHz, DMSO) δ: 6,80 (s, 2H) ; 4,07 (m, 1H) ; 4,04 (m, 2H) ; 3,80 (s, 3H) ; 3,52 (m, 4H) ; 3,22 (t, 2H) ; 2,95 (m, 1H) ; 2,70 (s, 3H) ; 2,67 (m, 1H) ; 2,53 (s, 6H) ; 1,72 (m, 2H) ; 1,38 (s, 9H) ; 1,26 (m, 2H).

## Exemple 140

***N*-[2-[2-(4-amino-1-pipéridinyl)-2-oxoéthoxyléthyl]-4-méthoxy-*N*,2,6-triméthyl-benzènesulfonamide**

En opérant de façon analogue à la préparation XXIX, au départ du composé obtenu selon la préparation CL, on obtient le produit attendu sous forme d'une huile jaune (rendement = 86 %).

RMN $^1H$ (300 MHz, DMSO) δ : 6,80 (s, 2H) ; 4,13 (m, 1H) ; 4,05 (m, 2H) ; 3,80 (s, 3H) ; 3,60 (m, 1H) ; 3,55 (t, 2H) ; 3,46 (m large, 2H) ; 3,22 (t, 2H) ; 2,89 (m, 2H) ; 2,70 (s, 3H) ; 2,67 (m, 1H) ; 2,53 (s, 6H) ; 1,74 (m, 2H) ; 1,51 (m, 2H).

## Exemple 141

**4-méthoxy-*N*,2,6-triméthyl-*N*-[2-[2-[4-[(8-méthyl-8-azabicyclo[3.2.1]oct-3-yl)amino]-1-pipéridinyl]-2-oxoéthoxy]éthyl]benzènesulfonamide**

En opérant de façon analogue à la préparation LXXI, au départ du composé obtenu selon l'exemple 140, on obtient le produit attendu sous forme d'une huile incolore (rendement = 40 %).
RMN $^1H$ (250 MHz, DMSO) δ : 6,80 (s, 2H) ; 4,13 (m, 1H) ; 4,05 (m, 2H) ; 3,79 (s, 3H) ; 3,65 (m, 1H) ; 3,50 (t, 2H) ; 3,22 (t, 2H) ; 2,91 (m, 4H) ; 2,70 (s, 3H) ; 2,60 (m, 2H) ; 2,51 (s, 6H) ; 1,85 (m, 8H) ; 1,45 (m, 2H) ; 0,96 (m, 2H).

## Exemple 142

**4-méthoxy-*N*,2,6-triméthyl-*N*-[2-[2-[4-[(8-méthyl-8-azabicyclo[3.2.1]oct-3-yl)amino]-1-pipéridinyl]-2-oxoéthoxy]éthyl]benzènesulfonamide, bis(trifluoroacétate)**

On agite jusqu'à dissolution un mélange de 140 mg (0,26 mM) du composé obtenu selon l'exemple 141 dans 4 ml d'une solution d'acide trifluoroacétique à 6% dans l'eau. La solution est congelée et lyophilisée et on obtient ainsi 200 mg du produit attendu sous forme d'un solide blanc (rendement quantitatif).
F=70 °C
En opérant de façon analogue à la préparation CLI et aux exemples 140 à 142, au départ du méthyl(4-pipéridinyl) carbamate de *t*-butyle, on obtient les composés suivants :

## PREPARATION CLH

**Acide [1-[[2-[[(4-méthoxy-2,6-diméthylphényl)sulfonyl]méthylamino] éthoxy]acétyl]-4-pipéridinyl]méthylcarbamique, 1,1-diméthyléthyl ester**

Huile incolore (rendement = 49 %).
RMN $^1H$ (300 MHz, DMSO) δ : 6,80 (s, 2H) ; 4,38 (m, 1H) ; 4,32 (m, 1H) ; 4,07 (m, 2H) ; 4,01 (m, 1H) ; 3,80 (s, 3H) ; 3,74 (m, 1H) ; 3,54 (m, 2H) ; 3,23 (t, 2H) ; 2,93 (m, 1H) ; 2,70 (s, 3H) ; 2,63 (s, 3H) ; 2,53 (s, 6H) ; 1,53 (m, 4H) ; 1,39 (s, 9H).

## Exemple 143

**4-méthoxy-*N*,2,6-triméthyl-*N*-[2-[2-[4-(méthylamino)-1-pipéridinyl]-2-oxoéthoxy]éthyl]-benzènesulfonamide**

Huile incolore (rendement = 99 %).
RMN $^1H$ (250 MHz, DMSO) δ: 6,80 (s, 2H) ; 4,18 (m, 1H) ; 4,08 (m, 2H) ; 3,80 (s, 3H) ; 3,68 (m, 1H) ; 3,54 (t, 2H) ; 3,23 (t, 2H) ; 2,95 (m, 1H) ; 2,85 (m, 1H) ; 2,70 (s, 3H) ; 2,67 (m, 1H) ; 2,53 (s, 6H) ; 2,42 (s, 3H) ; 1,85 (m, 2H) ; 1,25 (m, 2H).

## Exemple 144

**4-méthoxy-*N*,2,6-triméthyl-*N*-[2-[2-[4-[méthyl(8-méthyl-8-azabicyclo[3.2.1] oct-3-yl)amino]-1-pipéridinyl]-2-oxoéthoxy]éthyl]benzènesulfonamide**

Huile incolore (rendement = 34 %).
RMN $^1H$ (300 MHz, DMSO) δ : 6,80 (s, 2H) ; 4,30 (m, 1H) ; 4,10 (q, 2H) ; 3,80 (s, 3H) ; 3,67 (m, 1H) ; 3,52 (t, 2H) ; 3,30 (m, 2H) ; 3,21 (t, 2H) ; 2,88 (m, 1H) ; 2,75 (m, 2H) ; 2,70 (s, 3H) ; 2,53 (s, 6H) ; 2,49 (m, 1H) ; 2,14 (s, 3H) ; 2,05 (s, 3H) ; 1,89 (m, 2H) ; 1,45 (m, 8H) ; 1,23 (m, 2H).

**Exemple 145**

**4-méthoxy-*N*,2,6-triméthyl-*N*-[2-[2-[4-[méthyl(8-méthyl-8-azabicyclo[3.2.1] oct-3-yl)amino]-1-pipéridinyl]-2-oxoéthoxy]éthyl]benzènesulfonamide, bis(trifluoroacétate)**

Huile incolore (rendement = 99 %).

RMN $^1H$ (250 MHz, CD3CN) δ : 6,75 (s, 2H) ; 4,62 (m, 1H) ; 4,06 (m, 2H) ; 3,97 (m, 2H) ; 3,92 (m, 1H) ; 3,81 (s, 3H) ; 3,65 (m, 1H) ; 3,59 (m, 3H) ; 3,30 (t, 2H) ; 3,05 (m, 1H) ; 2,74 (s, 3H) ; 2,71 (d, 3H) ; 2,63 (s, 3H) ; 2,70-2,55 (m, 3H) ; 2,53 (s, 6H) ; 2,27 (m, 4H) ; 1,97 (m, 4H) ; 1,62 (m, 2H).

**PREPARATION CLIII**

**Acide 4-[[1-[(1,1-diméthyléthoxy)carbonyl]-4-pipéridinyl]méthyl]-1-pipérazinecarboxylique, phénylméthyl ester**

En opérant de façon analogue à la préparation LXXI, au départ de l'ester *t*-butylique de l'acide 4-formyl-1-pipéridinecarboxylique et d'ester benzylique de l'acide 1-pipérazinecarboxylique, on obtient le produit attendu sous forme d'une huile incolore (rendement = 26 %).

RMN $^1H$ (300 MHz, DMSO) δ : 7,31 (m, 5H) ; 5,11 (s, 2H) ; 3,90 (m, 2H) ; 3,64 (m, 4H) ; 2,75 (m, 8H) ; 1,89 (m, 1H) ; 1,70 (m, 2H) ; 1,39 (s, 9H) ; 1,04 (m, 2H).

**PREPARATION CLIV**

**Acide 4-(4-pipéridinylméthyl)-1-pipérazinecarboxylique, phénylméthyl ester**

En opérant de façon analogue à la préparation XXIX, au départ du composé obtenu selon la préparation CLIII, on obtient le produit attendu sous forme d'une huile incolore (rendement = 83 %).

RMN $^1H$ (250 MHz, DMSO) δ : 7,35 (m, 5H) ; 5,00 (s, 2H) ; 3,35 (m, 4H) ; 2,95 (m, 2H) ; 2,46 (m, 2H) ; 2,30 (m, 4H) ; 2,11 (m, 2H) ; 1,65 (m, 3H) ; 0,98 (m, 2H).

**PREPARATION CLV**

**Acide 4-[[1-[[2-[[(4-méthoxy-2,6-diméthylphényl)sulfonyl]méthylamino] éthoxy]acétyl]-4-pipéridinyl]méthyl]-1-pipérazinecarboxylique, phénylméthyl ester**

En opérant de façon analogue à l'exemple 72, au départ du composé obtenu selon la préparation CLIV, on obtient le produit attendu sous forme d'une huile incolore (rendement = 70 %).

RMN $^1H$ (250 MHz, DMSO) δ : 7,35 (m, 5H) ; 6,80 (s, 2H) ; 5,07 (s, 2H) ; 4,25 (m, 1H) ; 4,05 (m, 2H) ; 3,79 (s, 3H) ; 3,68 (m, 1H) ; 3,53 (t, 2H) ; 3,30 (m, 4H) ; 3,21 (t, 2H) ; 2,88 (m, 1H) ; 2,69 (s, 3H) ; 2,53 (s, 6H) ; 2,51 (m, 1H) ; 2,31 (m, 4H) ; 2,27 (m, 2H) ; 1,70 (m, 3H) ; 0,95 (m, 2H).

**Exemple 146**

**4-méthoxy-*N*,2,6-triméthyl-*N*-[2-[2-oxo-2-[4-(1-pipérazinylméthyl)-1-pipéridinyl]éthoxy]éthyl]benzènesulfonamide**

En opérant de façon analogue à la préparation XXXV, au départ du composé obtenu selon la préparation CLV, on obtient le produit attendu sous forme d'une huile incolore (rendement = 93 %).

RMN $^1H$ (300 MHz, DMSO) δ: 6,80 (s, 2H) ; 4,25 (m, 1H) ; 4,05 (q, 2H) ; 3,79 (s, 3H) ; 3,54 (m, 1H) ; 3,51 (t, 2H) ; 3,23 (t, 2H) ; 2,87 (m, 1H) ; 2,70 (s, 3H) ; 2,66 (m, 4H) ; 2,53 (s, 6H) ; 2,50 (m, 1H) ; 2,46 (m, 4H) ; 2,05 (d, 2H) ; 1,74 (m, 3H) ; 0,98 (m, 2H).

**Exemple 147**

**4-méthoxy-*N*,2,6-triméthyl-*N*-[2-[2-oxo-2-[4-(1-pipérazinylméthyl)-1-pipéridinyl]éthoxy]éthyl]benzènesulfonamide, fumarate**

En opérant de façon analogue à l'exemple 73, au départ du composé obtenu selon l'exemple 146, on obtient

le produit attendu sous forme d'un solide blanc (rendement = 93 %).
F = 85-87 °C

**PREPARATION CLVI**

**Acide 1-[[2-[[(4-méthoxy-2,6-diméthylphényl)sulfonyl]méthylamino] éthoxy]acétyl]-4-pipéridinecarboxylique, éthyl ester**

En opérant de façon analogue à l'exemple 70, au départ d'ester éthylique de l'acide 4-pipéridinecarboxylique, on obtient le produit attendu sous forme d'une huile incolore (rendement = 89 %).

RMN $^1$H (250 MHz, DMSO) δ : 6,80 (s, 2H) ; 4,17 (m, 5H) ; 3,80 (s, 3H) ; 3,65 (m, 1H) ; 3,53 (t, 2H) ; 3,29 (s, 3H) ; 3,21 (t, 2H) ; 2,99 (m, 1H) ; 2,72 (m, 1H) ; 2,70 (s, 3H) ; 2,58 (m, 1H) ; 2,53 (s, 6H) ; 1,83 (m, 2H) ; 1,44 (m, 2H) ; 1,20 (t, 3H).

**PREPARATION CLVII**

**Acide 1-[[2-[[(4-méthoxy-2,6-diméthylphényl)sulfonyl]méthylamino] éthoxylacétyl]- 4-pipéridinecarboxylique**

On prépare une solution de 2,3 g (4,89 mM) d'ester obtenu selon la préparation CLVI dans 40 ml de tétrahydrofurane et on ajoute une solution de 246 mg (5,8 mM) de lithine dans 3 ml d'eau. Le mélange est agité à température ambiante pendant une nuit puis concentré sous pression réduite. Le résidu est repris dans 25 ml d'eau, acidifié jusqu'à pH 2 par une solution d'acide chlorhydrique N et extrait par l'acétate d'éthyle. La phase organique est séchée sur sulfate de magnésium et concentrée sous pression réduite. Le produit brut est purifié par chromatographie sur gel de silice en éluant à l'aide d'un mélange dichlorométhane/méthanol (95/5 ; v/v). On obtient 1,6 g du composé attendu sous forme d'une huile incolore (rendement = 74 %).

RMN $^1$H (300 MHz, DMSO) δ : 6,80 (s, 2H) ; 4,10 (m, 1H) ; 4,06 (m, 2H) ; 3,79 (s, 3H) ; 3,55 (m, 1H) ; 3,53 (t, 2H) ; 3,23 (t, 2H) ; 2,98 (m, 1H) ; 2,74 (m, 1H) ; 2,70 (s, 3H) ; 2,53 (s, 3H) ; 2,50 (s, 6H) ; 2,43 (m, 1H) ; 1,85 (m, 2H) ; 1,40 (m, 2H).

**Exemple 148**

**4-méthoxy-*N*,2,6-triméthyl-*N*-[2-[2-oxo-2-[4-[1-oxo-2-(4-méthyl-1-pipérazinyl)éthyl]-1-pipéridinyl]éthoxy]éthyl] benzènesulfonamide, bis(trifluoroacétate)**

On prépare une solution de 0,65g (1,47 mM) de l'acide obtenu selon la préparation CLVII dans 20 ml de dichlorométhane et on ajoute 0,25 ml de chlorure d'oxalyle. Le mélange est agité pendant 2 heures à température ambiante et concentré sous pression réduite. L'huile jaune obtenue est reprise dans 8 ml de tétrahydrofurane et 8 ml d'acétonitrile et on ajoute, à 0 °C, 0,74 ml (1,47 mM) d'une solution 2M de triméthylsilyldiazométhane dans l'hexane. Le mélange est agité 1 heure à température ambiante puis concentré sous pression réduite. Le résidu est repris en solution dans 10 ml de chloroforme et ajouté goutte à goutte à une solution de 147 mg (1,47 mM) de N-méthylpipérazine dans 10 ml de chloroforme, en présence de 10 mg de chlorocyclopentadiènylbis (triphénylphosphine)ruthénium(II), à 60 °C. Le mélange réactionnel est agité à 60 °C pendant 30 min puis concentré sous pression réduite. Le produit brut est purifié par chromatographie sur gel de silice en éluant à l'aide d'un mélange dichlorométhane/méthanol (85/15 ; v/v). Le produit huileux obtenu est directement salifié par l'acide trifluoroacétique en appliquant le procédé décrit à l'exemple 142. On obtient 0,167 g du composé attendu sous forme d'une huile incolore (rendement = 21 %).

RMN $^1$H (300 MHz; CD3CN) δ : 6,75 (s, 2H) ; 4,35 (m, 1H) ; 4,04 (m, 2H) ; 3,83 (s, 3H) ; 3,81 (s, 2H) ; 3,70 (m, 1H) ; 3,58 (t, 2H) ; 3,38 (m, 4H) ; 3,30 (t, 2H) ; 3,22 (m, 4H) ; 2,95 (m, 1H) ; 2,81 (s, 3H) ; 2,74 (s, 3H) ; 2,69 (m, 2H) ; 2,56 (s, 6H) ; 1,80 (m, 2H) ; 1,45 (m, 2H).

**Exemple 149**

**4-méthoxy-*N*,2,6-triméthyl-*N*-[2-[2-[4-(1-méthyl-4-pipéridinyl)]-1-pipérazinyl]-2-oxoéthoxy]éthyl]benzènesulfonamide**

On prépare une solution de 7 g (21 mM) d'acide obtenu selon la préparation III dans 50 ml de chloroforme et 0,1 ml de diméthylformamide et on ajoute goutte à goutte, à température ambiante, une solution de 3,7 ml (42 mM) de chlorure d'oxalyle dans 8 ml de chloroforme. Le mélange réactionnel est agité pendant 3 heures à température ambiante et concentré sous pression réduite. Le résidu d'évaporation est repris dans 20 ml de chloroforme et ajouté lentement,

à 0°C, à une solution de 4,04 g (22 mM) de 1-(1-méthyl-4-pipéridinyl)pipérazine et 8,7 ml de triéthylamine dans 40 ml de chloroforme. Le mélange réactionnel est agité pendant 1 heure à 0°C puis versé sur 60 ml d'eau glacée. Le mélange obtenu est extrait par 100 ml de chloroforme et la phase organique est lavée par une solution de bicarbonate de sodium, puis à l'eau, séchée sur sulfate de magnésium et concentrée sous pression réduite. On obtient ainsi le composé attendu sous forme d'une huile (rendement = 81,7%) qui est utilisée sans autre purification pour obtenir un sel avec un acide.

## Exemple 150

**4-méthoxy-*N*,2,6-triméthyl-*N*-[2-[2-[4-(1-méthyl-4-pipéridinyl)]-1-pipérazinyl]-2-oxoéthoxy]éthyl]benzènezulfonamide, dichlorhydrate**

On prépare une solution de 6 g (12 mM) du composé obtenu selon l'exemple 149 dans 100 ml d'éthanol chauffé à environ 65°C. On ajoute ensuite, à environ 60°C, 3,2 ml (36 mM) d'acide chlorhydrique concentré. Il se forme rapidement un précipité. Après 2 heures sous agitation à température ambiante, la suspension est refroidie à 0°C et filtrée sur verre fritté. Le solide isolé est lavé par 10 ml d'éthanol froid puis par 15 ml d'éther éthylique et séché sous pression réduite. On obtient ainsi 5,2 g du sel attendu sous forme d'un solide blanc (rendement = 75 %).
F > 250 °C

## Exemple 151

**4-méthoxy-*N*,2,6-triméthyl-*N*-[2-[2-[4-(1-méthyl-4-pipéridinyl)]-1-pipérazinyl]-2-oxoéthoxy]éthyl]benzènesulfonamide, difumarate**

En opérant de façon analogue à l'exemple 150, avec 2,5 équivalents d'acide fumarique, on obtient le produit attendu sous forme d'un solide blanc (rendement = 76 %).
F =185 °C

**Tableau I**

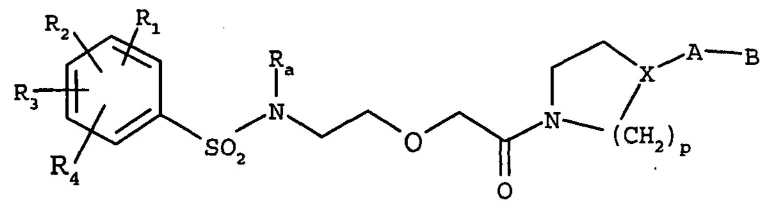

| EX | $R_1$ | $R_2$ | $R_3$ ($R_4$) | $R_a$ | X | p | A | B | Sel |
|---|---|---|---|---|---|---|---|---|---|
| 1 | 4-O-$CH_3$ | 2-$CH_3$ | 6-$CH_3$ | -$CH_3$ | N | 2 | $(CH_2)_2$ | 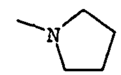 | 2TFA |
| 2 | 4-$CH_3$ | 2-$CH_3$ | 6-$CH_3$ | -$CH_3$ | N | 2 | 1s | 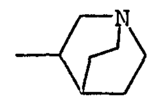 | 2TFA |
| 3 | 4-O-$CH_3$ | 2-$CH_3$ | 6-$CH_3$ | -$CH_3$ | N | 2 | 1s | 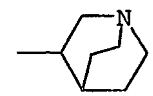 | - |
| 4 | 4-O-$CH_3$ | 2-$CH_3$ | 6-$CH_3$ | -$CH_3$ | N | 2 | 1s | 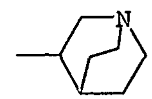 | 2F |
| 5 | 4-O-$CH_3$ | 2-$CH_3$ | 6-$CH_3$ | -$CH_3$ | N | 2 | 1s | 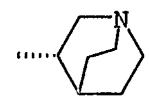 | - |
| 6 | 4-O-$CH_3$ | 2-$CH_3$ | 6-$CH_3$ | -$CH_3$ | N | 2 | 1s | 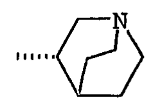 | 1F |
| 7 | 4-O-$CH_3$ | 2-$CH_3$ | 6-$CH_3$ | -$CH_3$ | N | 2 | 1s | 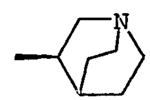 | 1F |

(suite)

| EX | $R_1$ | $R_2$ | $R_3$ ($R_4$) | $R_a$ | X | p | A | B | Sel |
|---|---|---|---|---|---|---|---|---|---|
| 8 | 4-O-$CH_3$ | 2-$CH_3$ | 6-$CH_3$ | -$CH_3$ | N | 2 | $(CH_2)_3$ | 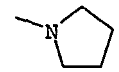 | 2TFA |
| 9 | 4-O-$CH_3$ | 2-$CH_3$ | 6-$CH_3$ | -$CH_3$ | N | 2 | $(CH_2)_2$ | 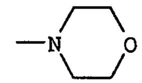 | 2TFA |
| 10 | 4-O-$CH_3$ | 2-$CH_3$ | 6-$CH_3$ | -$CH_3$ | N | 2 | $(CH_2)_2$ | 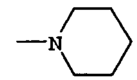 | 2TFA |
| 11 | 4-O-$CH_3$ | 2-$CH_3$ | 6-$CH_3$ | -$CH_3$ | N | 2 | $(CH_2)_3$ | 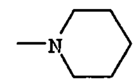 | 2TFA |
| 12 | 4-O-$CH_3$ | 2-$CH_3$ | 6-$CH_3$ | -$CH_3$ | N | 2 | $(CH_2)_3$ | -$N(CH_3)_2$ | 2TFA |
| 13 | 4-O-$CH_3$ | 2-$CH_3$ | 6-$CH_3$ | -$CH_3$ | N | 2 | 1s | 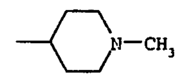 | 2TFA |
| 14 | 4-O-$CH_3$ | 2-$CH_3$ | 6-$CH_3$ | -$CH_3$ | N | 2 | 1s | 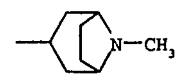 | 1F |
| 15 | 4-O-$CH_3$ | 2-$CH_3$ | 6-$CH_3$ | -$CH_3$ | N | 3 | 1s | 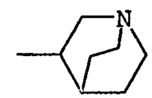 | 1F |
| 16 | 4-O-$CH_3$ | 2-$CH_3$ | 6-$CH_3$ | -$CH_3$ | N | 2 | $(CH_2)_3$ | 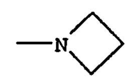 | 2F |
| 17 | 4-O-$CH_3$ | 2-$CH_3$ | 6-$CH_3$ | -$CH_3$ | N | 2 | 1s | 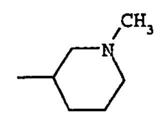 | 2F |
| 18 | 4-O-$CH_3$ | 2-$CH_3$ | 6-$CH_3$ | c-Pr | N | 2 | 1s | 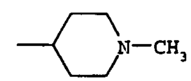 | 2F |

(suite)

| EX | $R_1$ | $R_2$ | $R_3$ ($R_4$) | $R_a$ | X | p | A | B | Sel |
|---|---|---|---|---|---|---|---|---|---|
| 19 | 4-O-$CH_3$ | 2-$CH_3$ | 6-$CH_3$ | c-Pr | N | 2 | $(CH_2)_2$ | 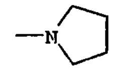 | 2F |
| 20 | 4-O-$CH_3$ | 2-$CH_3$ | 6-$CH_3$ | -$CH_3$ | N | 2 | -$CH_2$- | 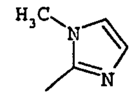 | 2F |
| 21 | 4-O-$CH_3$ | 2-$CH_3$ | 6-$CH_3$ | -$C_2H_5$ | N | 2 | 1s | 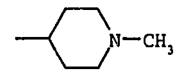 | 2F |
| 22 | 4-O-$CH_3$ | 2-$CH_3$ | 6-$CH_3$ | -$C_2H_5$ | N | 2 | $(CH_2)_3$ | -$N(CH_3)_2$ | 2F |
| 23 | 4-O-$CH_3$ | 2-$CH_3$ | 6-$CH_3$ | -$CH_3$ | N | 2 | 1s | 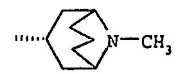 | 1F |
| 24 | 4-O-$CH_3$ | 2-$CH_3$ | 6-$CH_3$ | -$C_2H_5$ | N | 2 | $(CH_2)_3$ | 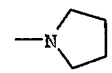 | 2F |
| 25 | 4-O-$CH_3$ | 2-$CH_3$ | 6-$CH_3$ | c-Pr | N | 2 | $(CH_2)_3$ | 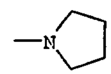 | 2F |
| 26 | 4-O-$CH_3$ | 2-$CH_3$ | 6-$CH_3$ | -$CH_3$ | N | 2 | 1s | 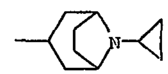 | 1F |
| 27 | 4-O-$CH_3$ | 2-$CH_3$ | 6-$CH_3$ | -$C_2H_5$ | N | 2 | 1s | 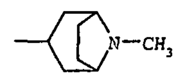 | 2F |
| 28 | 4-O-$CH_3$ | 2-$CH_3$ | 6-$CH_3$ | c-Pr | N | 2 | 1s | 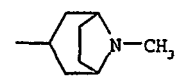 | 2F |
| 29 | 4-O-$CH_3$ | 2-$CH_3$ | 6-$CH_3$ | -$CH_3$ | N | 2 | 1s | 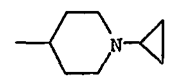 | 2F |
| 30 | 4-O-$CH_3$ | 2-$CH_3$ | 6-$CH_3$ | i-Pr | N | 2 | 1s | 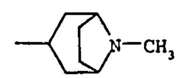 | 2F |

(suite)

| EX | $R_1$ | $R_2$ | $R_3$ ($R_4$) | $R_a$ | X | p | A | B | Sel |
|---|---|---|---|---|---|---|---|---|---|
| 31 | 4-O-$CH_3$ | 2-$CH_3$ | 6-$CH_3$ | -$CH_3$ | N | 2 | 1s | 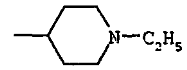 | 1F |
| 32 | 4-O-$CH_3$ | 2-$CH_3$ | 6-$CH_3$ | -$CH_3$ | N | 2 | 1s | 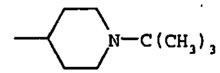 | 2F |
| 33 | 4-O-$CH_3$ | 2-$CH_3$ | 6-$CH_3$ | -$CH_3$ | N | 2 | -$CH_2$- | 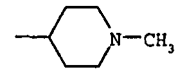 | 1F |
| 34 | 4-O-$CH_3$ | 2-Cl | 6-Cl | -$CH_3$ | N | 2 | -$(CH_2)_3$- | -H$(CH_3)$z | 2F |
| 35 | 4-O-$CH_3$ | 2-Cl | 6-Cl | -$CH_3$ | N | 2 | 1s | 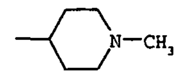 | 2F |
| 36 | 4-O-$CH_3$ | 2-$CH_3$ | 6-$CH_3$ | -$CH_3$ | N | 2 | -$(CH_2)_2$- | 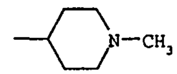 | 2F |
| 37 | 4-O-$CH_3$ | 2-$CH_3$ | 6-$CH_3$ | -$CH_3$ | N | 3 | 1s | 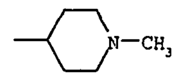 | 1F |
| 38 | 4-O-$CH_3$ | 2-$CH_3$ | 6-$CH_3$ | i-Pr | N | 2 | 1s | 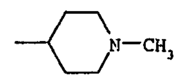 | 2F |
| 39 | 4-O-$CH_3$ | 2-$CH_3$ | 6-$CH_3$ | -$CH_3$ | N | 2 | 1s | 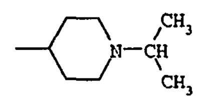 | 1F |
| 40 | 4-O-$CH_3$ | 2-$CH_3$ | 6-$CH_3$ | -$C_2H_5$ | N | 2 | $(CH_2)_3$ | 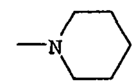 | 2F |
| 41 | 4-Cl | 2-Cl | 6-O-$CH_3$ | -$CH_3$ | N | 2 | 1s | 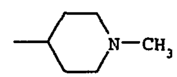 | 2F |
| 42 | 4-O-$CH_3$ | 2-Cl | 6-Cl | -$CH_3$ | N | 2 | 1s | 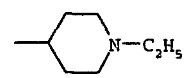 | 1F |

(suite)

| EX | $R_1$ | $R_2$ | $R_3$ ($R_4$) | $R_a$ | X | p | A | B | Sel |
|---|---|---|---|---|---|---|---|---|---|
| 43 | 4-O-$CH_3$ | 2-Cl | 6-Cl | -$CH_3$ | N | 2 | 1s | 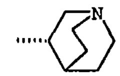 | 1F |
| 44 | 4-O-$CH_3$ | 2-$CH_3$ | 6-$CH_3$ | -$CH_3$ | N | 2 | 1s | 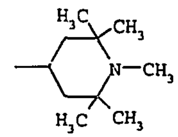 | 2TFA |
| 45 | 4-O-$CH_3$ | 2-$CH_3$ | 6-$CH_3$ | -$CH_3$ | N | 2 | $(CH_2)_3$ | 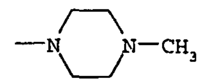 | 2F |
| 46 | 4-O-$CH_3$ | 2-$CH_3$ | 6-$CH_3$ | -$CH_3$ | N | 2 | 1s | 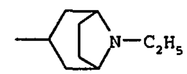 | 2F |
| 47 | 4-O-$CH_3$ | 2-$CH_3$ | 6-$CH_3$ | -$CH_3$ | N | 2 | $(CH_2)_3$ | 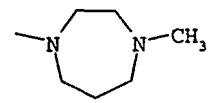 | 2F |
| 48 | 4-O-$CH_3$ | 2-$CH_3$ | 6-$CH_3$ | -$CH_3$ | N | 2 | 1s | 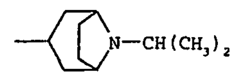 | 2F |
| 49 | 4-O-$CH_3$ | 2-$CH_3$ | 6-$CH_3$ | -$CH_3$ | N | 2 | $-(CH_2)_2$-CO- | 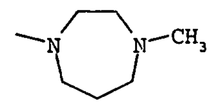 | 2TFA |
| 50 | 4-O-$CH_3$ | 2-$CH_3$ | 6-$CH_3$ | -$CH_3$ | N | 2 | $(CH_2)_2$ | 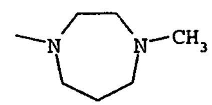 | 3F |
| 51 | 4-O-$CH_3$ | 2-$CH_3$ | 6-$CH_3$ | c-Pr | N | 2 | 1s | 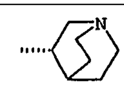 | 2F |
| 52 | 4-O-$CH_3$ | 2-$CH_3$ | 6-$CH_3$ | -$C_2H_5$ | N | 2 | 1s | 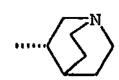 | 2F |
| 53 | 4-O-$CH_3$ | 2-$CH_3$ | 6-$CH_3$ | -$CH_3$ | N | 2 | $(CH_2)_2$ | $-N(C_2H_5)_2$ | 1F |

(suite)

| EX | $R_1$ | $R_2$ | $R_3$ ($R_4$) | $R_a$ | X | p | A | B | Sel |
|---|---|---|---|---|---|---|---|---|---|
| 54 | 4-F | 2-Cl | 6-Cl | $-CH_3$ | N | 2 | 1s | 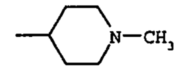 | base |
| 55 | 4-F | 2-Cl | 6-Cl | $-CH_3$ | N | 2 | 1s | 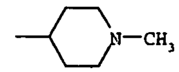 | 2F |
| 56 | 4-Br | 2-Cl | 6-Cl | $-CH_3$ | N | 2 | 1s | 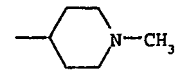 | base |
| 57 | 4-Br | 2-Cl | 6-Cl | $-CH_3$ | N | 2 | 1s | 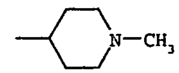 | 2F |
| 58 | 4-Cl | 2-Cl | 6-Cl | $-CH_3$ | N | 2 | 1s | 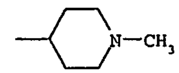 | base |
| 59 | 4-Cl | 2-Cl | 6-Cl | $-CH_3$ | N | 2 | 1s | 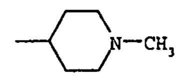 | 2F |
| 60 | 4-Cl | 2-Cl | $6-CH_3$ | $-CH_3$ | N | 2 | 1s | 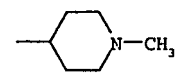 | base |
| 61 | 4-Cl | 2-Cl | $6-CH_3$ | $-CH_3$ | N | 2 | 1s | 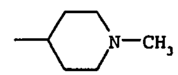 | 2F |
| 62 | $4-O-CH_3$ | $2-CH_3$ | $3,6-diCH_3$ | $-CH_3$ | N | 2 | 1s | 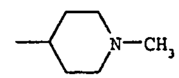 | base |
| 63 | $4-O-CH_3$ | $2-CH_3$ | $3,6-diCH_3$ | $-CH_3$ | N | 2 | 1s | 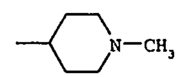 | 2F |
| 64 | $4-O-CH_3$ | $2-CH_3$ | $6-CH_3$ | $-CH_3$ | N | 2 | $(CH_2)_3$ | 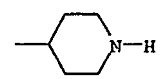 | base |
| 65 | $4-O-CH_3$ | $2-CH_3$ | $6-CH_3$ | $-CH_3$ | N | 2 | $(CH_2)_3$ | 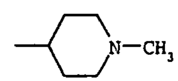 | base |

(suite)

| EX | $R_1$ | $R_2$ | $R_3$ ($R_4$) | $R_a$ | X | p | A | B | Sel |
|---|---|---|---|---|---|---|---|---|---|
| 66 | 4-O-$CH_3$ | 2-$CH_3$ | 6-$CH_3$ | -$CH_3$ | N | 2 | $(CH_2)_3$ | 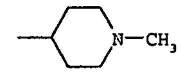 | 2F |
| 67 | 4-O-$CH_3$ | 2-$CH_3$ | 6-$CH_3$ | -$CH_3$ | N | 2 | 1s | 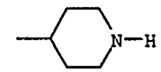 | 2TFA |
| 68 | 4-O-$CH_3$ | 2-$CH_3$ | 6-$CH_3$ | -$CH_3$ | N | 2 | 1s | 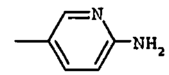 | base |
| 69 | 4-O-$CH_3$ | 2-$CH_3$ | 6-$CH_3$ | -$CH_3$ | N | 2 | 1s | 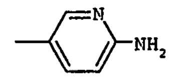 | 1F |
| 70 | 4-O-$CH_3$ | 2-$CH_3$ | 6-$CH_3$ | -$CH_3$ | CH | 2 | $-C(CH_3)_2-CH_2-$ | $-N(CH_3)_2$ | 1TFA |
| 71 | 4-O-$CH_3$ | 2-$CH_3$ | 6-$CH_3$ | -$CH_3$ | CH | 2 | $-CH(OH)-CH_2-$ | $-N(CH_3)_2$ | 1TFA |
| 72 | 4-O-$CH_3$ | 2-$CH_3$ | 6-$CH_3$ | -$CH_3$ | CH | 2 | 1s | 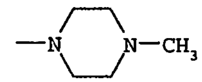 | base |
| 73 | 4-O-$CH_3$ | 2-$CH_3$ | 6-$CH_3$ | -$CH_3$ | CH | 2 | 1s | 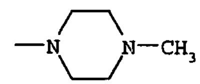 | 1F |
| 74 | 4-O-$CH_3$ | 2-$CH_3$ | 6-$CH_3$ | -$CH_3$ | CH | 2 | 1s | 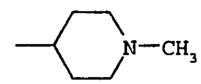 | 1F |
| 75 | 4-O-$CH_3$ | 2-$CH_3$ | 6-$CH_3$ | cPr | CH | 2 | 1s | 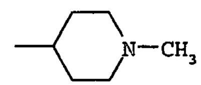 | 2F |
| 76 | 4-O-$CH_3$ | 2-$CH_3$ | 6-$CH_3$ | -$CH_3$ | CH | 2 | $(CH_2)_2$ | 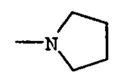 | 1F |
| 77 | 4-O-$CH_3$ | 2-$CH_3$ | 6-$CH_3$ | -$CH_3$ | CH | 2 | 1s | 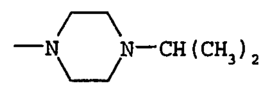 | 1F |

(suite)

| EX | $R_1$ | $R_2$ | $R_3$ ($R_4$) | $R_a$ | X | p | A | B | Sel |
|---|---|---|---|---|---|---|---|---|---|
| 78 | 4-O-$CH_3$ | 2-$CH_3$ | 6-$CH_3$ | -$C_2H_5$ | CH | 2 | $(CH_2)_2$ | 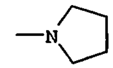 | 1F |
| 79 | 4-O-$CH_3$ | 2-$CH_3$ | 6-$CH_3$ | cPr | CH | 2 | $(CH_2)_2$ | 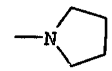 | 1F |
| 80 | 4-O-$CH_3$ | 2-$CH_3$ | 6-$CH_3$ | -$CH_3$ | CH | 2 | $(CH_2)_2$ | 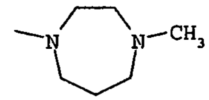 | 2F |
| 81 | 4-O-$CH_3$ | 2-$CH_3$ | 6-$CH_3$ | -$CH_3$ | CH | 2 | $(CH_2)_2$ | 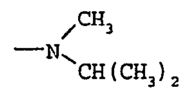 | 1F |
| 82 | 4-O-$CH_3$ | 2-$CH_3$ | 6-$CH_3$ | -$CH_3$ | CH | 2 | -N($CH_3$)- | 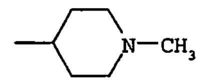 | 1F |
| 83 | 4-O-$CH_3$ | 2-$CH_3$ | 6-$CH_3$ | -$CH_3$ | CH | 2 | 1s | 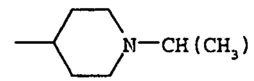 | 1F |
| 84 | 4-O-$CH_3$ | 2-$CH_3$ | 6-$CH_3$ | -$CH_3$ | CH | 2 | 1s | 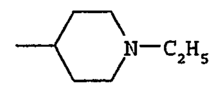 | 1F |
| 85 | 4-O-$CH_3$ | 2-$CH_3$ | 6-$CH_3$ | -$CH_3$ | CH | 2 | 1s | 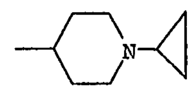 | 1F |
| 86 | 4-O-$CH_3$ | 2-$CH_3$ | 6-$CH_3$ | -$CH_3$ | CH | 2 | $(CH_2)_2$ | 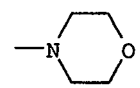 | 1F |
| 87 | 4-O-$CH_3$ | 2-$CH_3$ | 6-$CH_3$ | -$CH_3$ | CH | 2 | -C$(CH_3)_2$-$CH_2$- | 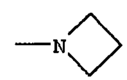 | 1F |

(suite)

| EX | $R_1$ | $R_2$ | $R_3$ ($R_4$) | $R_a$ | X | p | A | B | Sel |
|---|---|---|---|---|---|---|---|---|---|
| 88 | 4-O-$CH_3$ | 2-$CH_3$ | 6-$CH_3$ | -$C_2H_5$ | CH | 2 | 1s | 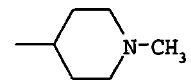 | 1F |
| 89 | 4-O-$CH_3$ | 2-$CH_3$ | 6-$CH_3$ | -$CH_3$ | CH | 2 | 1s | 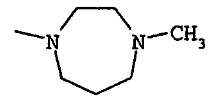 | 1F |
| 90 | 4-O-$CH_3$ | 2-$CH_3$ | 6-$CH_3$ | cPr | CH | 2 | 1s | 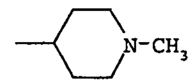 | 1F |
| 91 | 4-Cl | 2-Cl | 3-$CH_3$ | -$CH_3$ | CH | 2 | 1s | 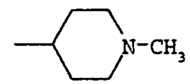 | 1F |
| 92 | 4-O-$CH_3$ | 2-$CH_3$ | 6-$CH_3$ | -$CH_3$ | CH | 2 | -$(CH_2)_2$- | 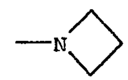 | 1F |
| 93 | 4-O-$CH_3$ | 2-Cl | 6-Cl | -$CH_3$ | CH | 2 | $(CH_2)_2$ | -$N(CH_3)_2$ | 1F |
| 94 | 4-O-$CH_3$ | 2-Cl | 6-Cl | -$CH_3$ | CH | 2 | 1s | 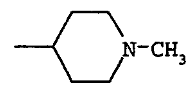 | 1F |
| 95 | 4-O-$CH_3$ | 2-$CH_3$ | 6-$CH_3$ | -$CH_3$ | CH | 2 | -$CH_2$- | 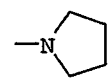 | 1F |
| 96 | 4-O-$CH_3$ | 2-$CH_3$ | 6-$CH_3$ | -$CH_3$ | CH | 2 | -$CH_2$- | 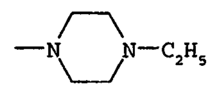 | 1F |
| 97 | 4-O-$CH_3$ | 2-$CH_3$ | 6-$CH_3$ | cPr | CH | 2 | -$CH_2$- | 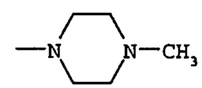 | 2F |
| 98 | 4-O-$CH_3$ | 2-$CH_3$ | 6-$CH_3$ | -$C_2H_5$ | CH | 2 | -$CH_2$- | 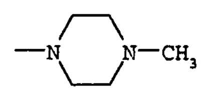 | 2F |

(suite)

| EX | $R_1$ | $R_2$ | $R_3$ ($R_4$) | $R_a$ | X | p | A | B | Sel |
|---|---|---|---|---|---|---|---|---|---|
| 99 | 4-O-$CH_3$ | 2-Cl | 6-Cl | -$CH_3$ | CH | 2 | -$CH_2$- | 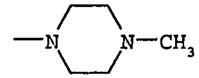 | 2F |
| 100 | 4-O-$CH_3$ | 2-$CH_3$ | 6-$CH_3$ | -$CH_3$ | CH | 2 | -C($CH_3$)$_2$-$CH_2$- | 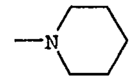 | 1TFA |
| 101 | 4-O-$CH_3$ | 2-$CH_3$ | 6-$CH_3$ | -$CH_3$ | CH | 2 | -C($CH_3$)$_2$-$CH_2$- | 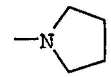 | 1F |
| 102 | 4-O-$CH_3$ | 2-$CH_3$ | 6-$CH_3$ | -$CH_3$ | CH | 2 | ($CH_2$)$_2$ | 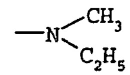 | 1F |
| 103 | 4-O-$CH_3$ | 2-$CH_3$ | 6-$CH_3$ | -$CH_3$ | CH | 2 | ($CH_2$)$_2$ | -N($C_2H_5$)$_2$ | 1F |
| 104 | 4-O-$CH_3$ | 2-$CH_3$ | 6-$CH_3$ | iPr | CH | 2 | ($CH_2$)$_2$ | 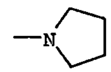 | 1F |
| 105 | 4-O-$CH_3$ | 2-$CH_3$ | 6-$CH_3$ | -$CH_3$ | CH | 2 | -C($CH_3$)$_2$-$CH_2$- | 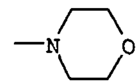 | 1F |
| 106 | 4-O-$CH_3$ | 2-$CH_3$ | 6-$CH_3$ | -$CH_3$ | CH | 2 | ($CH_2$)$_2$ | 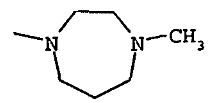 | 2F |
| 107 | 4-O-$CH_3$ | 2-$CH_3$ | 6-$CH_3$ | -$CH_3$ | CH | 2 | -$CH_2$- | 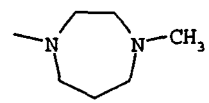 | 2F |
| 108 | 4-O-$CH_3$ | 2-$CH_3$ | 6-$CH_3$ | -$CH_3$ | CH | 2 | ($CH_2$)$_2$ | 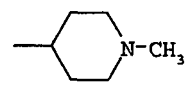 | 1F |

(suite)

| EX | $R_1$ | $R_2$ | $R_3$ ($R_4$) | $R_a$ | X | p | A | B | Sel |
|---|---|---|---|---|---|---|---|---|---|
| 109 | 4-O-$CH_3$ | 2-$CH_3$ | 6-$CH_3$ | -$CH_3$ | CH | 2 | $(CH_2)_2$ | 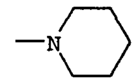 | 1F |
| 110 | 4-O-C | 2-$CF_3$ | H | -$CH_3$ | CH | 2 | $(CH_2)_2$ | 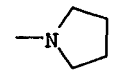 | 1TFA |
| 111 | 4-O-$CH_3$ | 2-$CH_3$ | 6-$CH_3$ | -$CH_3$ | CH | 2 | -$CH_2$-CO- | 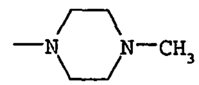 | 1F |
| 112 | 4-O-$CH_3$ | 2-$CH_3$ | 6-$CH_3$ | -$CH_3$ | CH | 2 | -$C(CH_3)_2$-$CH_2$- | -$N(C_2H_5)_2$ | 1TFA |
| 113 | 4-O-$CH_3$ | 2-$CH_3$ | 6-$CH_3$ | -$CH_3$ | CH | 2 | -$CH_2$- | -$N(CH_3)_2$ | 1F |
| 114 | 4-O-$CH_3$ | 2-$CH_3$ | 6-$CH_3$ | -$CH_3$ | CH | 2 | -$CH_2$- | 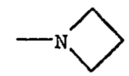 | 1F |
| 115 | 4-$CH_3$ | 2-$CH_3$ | 6-$CH_3$ | -$CH_3$ | CH | 2 | 1s | 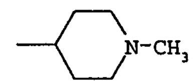 | 1TFA |
| 116 | H | 2-$CF_3$ | H | -$CH_3$ | CH | 2 | 1s | 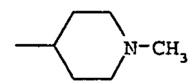 | 1TFA |
| 117 | 4-O-$CH_3$ | 2-$CF_3$ | H | -$CH_3$ | CH | 2 | 1s | 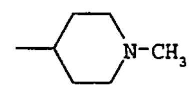 | 1TFA |
| 118 | 4-$CH_3$ | 2-$CH_3$ | 6-$CH_3$ | -$CH_3$ | CH | 2 | $(CH_2)_2$ | 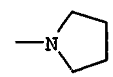 | 1TFA |
| 119 | 4-O-$CH_3$ | 2-Cl | 6-Cl | -$CH_3$ | CH | 2 | 1s | 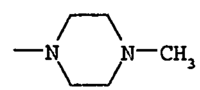 | 2F |
| 120 | 4-O-$CH_3$ | 2-$CH_3$ | 6-$CH_3$ | -$CH_3$ | CH | 2 | $(CH_2)_2$ | -$N(CH_3)_2$ | 1F |

(suite)

| EX | $R_1$ | $R_2$ | $R_3$ ($R_4$) | $R_a$ | X | p | A | B | Sel |
|---|---|---|---|---|---|---|---|---|---|
| 121 | 4-O-$CH_3$ | 2-$CH_3$ | 6-$CH_3$ | -$CH_3$ | CH | 2 | 1s | 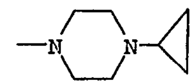 | 1F |
| 122 | 4-O-$CH_3$ | 2-$CH_3$ | 6-$CH_3$ | -$CH_3$ | CH | 2 | 1s | 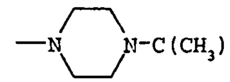 | 2F |
| 123 | 4-O-$CH_3$ | 2-$CH_3$ | 6-$CH_3$ | -$CH_3$ | CH | 2 | -$CH_2$- | 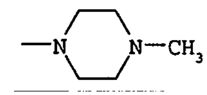 | 2F |
| 124 | 4-O-$CH_3$ | 2-$CH_3$ | 6-$CH_3$ | -$CH_3$ | CH | 2 | $(CH_2)_2$ | 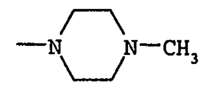 | 2F |
| 125 | 4-O-$CH_3$ | 2-$CH_3$ | 6-$CH_3$ | -$CH_3$ | CH | 2 | $(CH_2)_3$ | 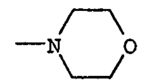 | base |
| 126 | 4-O-$CH_3$ | 2-$CH_3$ | 6-$CH_3$ | -$CH_3$ | CH | 2 | $(CH_2)_3$ | 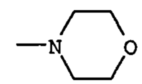 | 1F |
| 127 | 4-O-$CH_3$ | 2-$CH_3$ | 6-$CH_3$ | -$CH_3$ | CH | 2 | $(CH_2)_3$ | 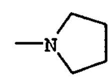 | base |
| 128 | 4-O-$CH_3$ | 2-$CH_3$ | 6-$CH_3$ | -$CH_3$ | CH | 2 | $(CH_2)_3$ | 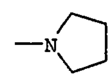 | 1F |
| 129 | 4-O-$CH_3$ | 2-$CH_3$ | 6-$CH_3$ | -$CH_3$ | CH | 2 | $(CH_2)_3$ | 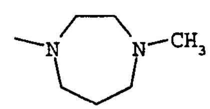 | base |
| 130 | 4-O-$CH_3$ | 2-$CH_3$ | 6-$CH_3$ | -$CH_3$ | CH | 2 | $(CH_2)_3$ | 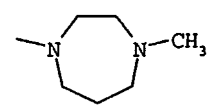 | 2F |

(suite)

| EX | $R_1$ | $R_2$ | $R_3$ ($R_4$) | $R_a$ | X | p | A | B | Sel |
|---|---|---|---|---|---|---|---|---|---|
| 131 | 4-O-$CH_3$ | 2-$CH_3$ | 6-$CH_3$ | -$CH_3$ | CH | 2 | $(CH_2)_3$ | 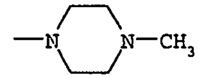 | base |
| 132 | 4-O-$CH_3$ | 2-$CH_3$ | 6-$CH_3$ | -$CH_3$ | CH | 2 | $(CH_2)_3$ | 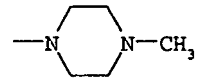 | 2F |
| 133 | 4-O-$CH_3$ | 2-$CH_3$ | 6-$CH_3$ | -$CH_3$ | CH | 2 | $(CH_2)_3$ | 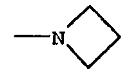 | base |
| 134 | 4-O-$CH_3$ | 2-$CH_3$ | 6-$CH_3$ | -$CH_3$ | CH | 2 | $(CH_2)_3$ | 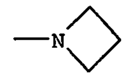 | 1F |
| 135 | 4-O-$CH_3$ | 2-$CH_3$ | 6-$CH_3$ | -$CH_3$ | CH | 2 | $(CH_2)_3$ | -$N(CH_3)_2$ | base |
| 136 | 4-O-$CH_3$ | 2-$CH_3$ | 6-$CH_3$ | -$CH_3$ | CH | 2 | $(CH_2)_3$ | $N(CH_3)_2$ | 1F |
| 137 | 4-O-$CH_3$ | 2-$CH_3$ | 6-$CH_3$ | -$CH_3$ | CH | 2 | 1s | 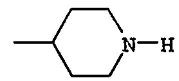 | 1TFA |
| 138 | 4-O-$CH_3$ | 2-$CH_3$ | 6-$CH_3$ | -$CH_3$ | CH | 2 | $(CH_2)_2$ | -$NH(CH_3)$ | Base |
| 139 | 4-O-$CH_3$ | 2-$CH_3$ | 6-$CH_3$ | -$CH_3$ | CH | 2 | $(CH_2)_2$ | -$NH(CH_3)$ | 1F |
| 140 | 4-O-$CH_3$ | 2-$CH_3$ | 6-$CH_3$ | -$CH_3$ | CH | 2 | 1s | -$NH_2$ | base |
| 141 | 4-O-$CH_3$ | 2-$CH_3$ | 6-$CH_3$ | -$CH_3$ | CH | 2 | -NH- | 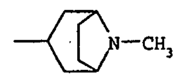 | base |
| 142 | 4-O-$CH_3$ | 2-$CH_3$ | 6-$CH_3$ | -$CH_3$ | CH | 2 | -NH- | 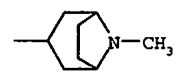 | 2TFA |
| 143 | 4-O-$CH_3$ | 2-$CH_3$ | 6-$CH_3$ | -$CH_3$ | CH | 2 | 1s | -$NH(CH_3)$ | base |
| 144 | 4-O-$CH_3$ | 2-$CH_3$ | 6-$CH_3$ | -$CH_3$ | CH | 2 | -$N(CH_3)$- | 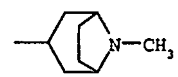 | base |

(suite)

| EX | $R_1$ | $R_2$ | $R_3$ ($R_4$) | $R_a$ | X | p | A | B | Sel |
|---|---|---|---|---|---|---|---|---|---|
| 145 | 4-O-$CH_3$ | 2-$CH_3$ | 6-$CH_3$ | -$CH_3$ | CH | 2 | -N($CH_3$)- | 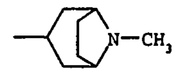 | 2TFA |
| 146 | 4-O-$CH_3$ | 2-$CH_3$ | 6-$CH_3$ | -$CH_3$ | CH | 2 | -$CH_2$- | 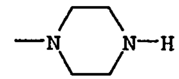 | base |
| 147 | 4-O-$CH_3$ | 2-$CH_3$ | 6-$CH_3$ | -$CH_3$ | CH | 2 | -$CH_2$- | 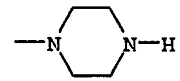 | 1F |
| 148 | 4-O-$CH_3$ | 2-$CH_3$ | 6-$CH_3$ | -$CH_3$ | CH | 2 | -CO-$CH_2$- | 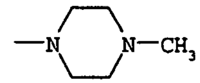 | 2TFA |
| 149 | 4-O-$CH_3$ | 2-$CH_3$ | 6-$CH_3$ | -$CH_3$ | N | 2 | 1s | 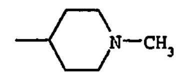 | base |
| 150 | 4-O-$CH_3$ | 2-$CH_3$ | 6-$CH_3$ | -$CH_3$ | N | 2 | 1s | 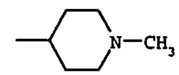 | 2HCl |
| 151 | 4-O-$CH_3$ | 2-$CH_3$ | 6-$CH_3$ | -$CH_3$ | N | 2 | 1s | 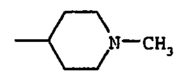 | 2F |

Dans le tableau qui précède, 1s signifie liaison simple, TFA signifie que le composé se présente sous forme d'un sel avec l'acide trifluoroacétique, F signifie que le composé se présente sous forme d'un sel avec l'acide fumarique, HCl signifie que le composé se présente sous forme d'un sel avec l'acide chlorhydrique.

## Activité biologique

Les composés de la présente invention ont été évalués pour leur propriété analgésique dans le test de douleur induite par le formaldéhyde chez la souris (Shibata, M., Ohkubo, T., Takahashi, H. & R. Inoki. Modified formalin test: characteristic biphasic pain response. Pain, 38, 347-352). En résumé, une administration de formaldéhyde (0,92 % dans le sérum physiologique) est effectuée dans la patte arrière et la durée de léchage, qui reflète l'intensité de la douleur, est enregistrée de 0 à 5 min (1ère phase) et de 15 à 30 min (2nde phase) après l'injection. Selon ce test, le pourcentage d'inhibition de la seconde phase de léchage induite par le formaldéhyde est de 43% pour le composé de l'exemple 4 administré par voie orale à la dose de 3 mg/kg et de 40 % pour le composé de l'exemple 6 administré par voie orale à la dose de 1 mg/kg.
Ces résultats témoignent d'une baisse très sensible de la douleur après administration des composés.
Suite aux résultats de l'essai précédent, les composés selon l'invention ont été soumis à un test visant à démontrer leur mode d'action et mettant en jeu le récepteur B1 de la bradykinine.
Ce test utilise la veine ombilicale humaine et est réalisé selon le protocole suivant :
Des cordons ombilicaux humains de 15-25 cm de long sont récupérés juste après la délivrance et placés immédiatement dans un flacon contenant une solution de Krebs de composition (en mM) : NaCl 119, KCl 4,7, $KH_2PO_4$ 1,18, $MgSO_4$ 1,17, $NaHCO_2$ 25, $CaCl_2$ 2,5, Glucose 5,5, EDTA 0,026 puis stockés à 4°C.
Le cordon est disséqué sous solution de Krebs afin de dégager la veine ombilicale. La veine est nettoyée de tout tissu adhérent et coupée en petits anneaux de 3-4 mm de large. L'endothélium est enlevé précautionneusement par introduction dans la lumière du vaisseau d'un fin cathéter n°1, rendu légèrement abrasif.
Afin d'induire l'expression du récepteur $B_1$ de la bradykinine, les segments de veine sont mis à incuber à 37°C dans une cuve de 25 ml pendant 16 heures dans un milieu de culture EMEM oxygéné par un mélange 95% $O_2$ + 5% $CO_2$ auquel on ajoute des antibiotiques : pénicilline 10 000 UI/ml et streptomycine 10 000 UI/ml. Le lendemain, les anneaux de veine sont montés sur un support en acier inoxydable, relié à un capteur isométrique et placés dans une cuve à organes isolés de 8 ml thermostatée à 37°C, contenant de la solution de Krebs oxygénée par un mélange 95% $O_2$ + 5% $CO_2$.
Après une période de repos d'une heure pendant laquelle les anneaux sont rincés 5 à 6 fois avec la solution de Krebs (maintenue à 37°C pendant toute la manipulation et oxygénée par le mélange 95% $O_2$ + 5% $CO_2$), la veine est soumise progressivement à une tension de 1 g. Lorsque la tension est stable, après 45 minutes environ, la solution de Krebs est remplacée par une solution hyperpotassique (KPSS : à température de 37°C) de même composition, mais contenant du KCl 125 mM et pas de NaCl.
Après une série de rinçages, repos et réajustement de la tension, la contraction maximale de chaque segment est déterminée par une nouvelle dépolarisation avec la solution de KPSS.
Après une nouvelle période de repos pendant laquelle la tension à 1 g est réajustée constamment, les composés suivants sont ajoutés dans le bain d'organe isolé : Mépyramine (1 $\mu$M), Atropine (1 $\mu$M), Indométacine (3 $\mu$M), LNA (30 $\mu$M), Captopril (10 $\mu$M), DL-Thiorphan (1 $\mu$M) et Nifédipine (0,1 $\mu$M).
20 minutes après, la molécule à tester ou le solvant de la molécule est ajouté dans le bain d'organe isolé. Les molécules sont étudiées à 10 $\mu$M ; si une molécule présente un degré d'activité suffisant, elle est étudiée à des concentrations plus faibles (ex : 1 - 0,1-0,01 $\mu$M).
Après 15 minutes d'incubation, les segments de veine sont contractés par l'ajout de concentrations croissantes de des-Arg10-Kallidin (0,1 nM à 30 000 nM) dans la cuve.
Les $EC_{50}$ (concentrations effectives d'agonistes requises pour produire 50% de la réponse maximale obtenue avec le KPSS) sont calculées par la méthode des moindres carrés.
Le $pK_B$ = [-log$K_a$] est obtenue à partir de l'équation :

$$K_B = [A] / (\text{concentration ratio-1})$$

où [A] est la concentration d'antagoniste et la (concentration ratio) représente le rapport entre l'$EC_{50}$ en présence d'antagoniste, et l'$EC_{50}$ en l'absence d'antagoniste.
Selon ce test, les composés selon l'invention cités dans la description présentent un $pK_B$ supérieur à 7.
A titre d'exemple, les $pK_B$ de certains composés selon l'invention sont reportés dans le tableau II :

| Ex. | 13 | 19 | 21 | 32 | 33 | 45 | 46 |
|---|---|---|---|---|---|---|---|
| $pK_B$ | 8,4 | 7,5 | 7,8 | 9,2 | 8,6 | 7,9 | 8,6 |

| Ex | 74 | 76 | 77 | 79 | 84 | 89 | 99 | 120 |
|---|---|---|---|---|---|---|---|---|
| $pK_B$ | 9 | 8 | 8,5 | 8,1 | 9,2 | 8,1 | 8 | 7,6 |

Les composés de la présente invention sont utiles pour le traitement de diverses formes de douleur telles que l'hyperalgésie inflammatoire, l'allodynie, la douleur neuropathique associée, par exemple, au diabète, à des neuropathies (constriction du nerf sciatique, lombalgies), à toute forme de traumatisme, à une intervention chirurgicale (extraction dentaire, ablation des amygdales), à une cystite interstitielle, à une maladie inflammatoire du colon, à un cancer.

Les composés de la présente invention peuvent aussi être utiles pour traiter toute pathologie associée à un recrutement de neutrophiles comme par exemple, le syndrome de détresse respiratoire aiguë, le psoriasis, les obstructions pulmonaires chroniques, les maladies inflammatoires, notamment les maladies inflammatoires du colon, la polyarthrite rhumatoïde.

L'activité des composés selon l'invention, mise en évidence au cours des tests biologiques, est significative, de propriétés antalgiques et permet d'envisager leur utilisation en thérapeutique.

Selon l'invention, on préconise l'utilisation des composés définis par la formule I, ainsi que de leurs sels avec des acides non toxiques, de préférence leurs sels pharmaceutiquement acceptables, en tant que principes actifs de médicaments destinés à un traitement chez les mammifères, notamment chez l'homme, vis à vis de la douleur ou de certaines maladies généralement caractérisées par une migration massive de neutrophiles.

Parmi les maladies qui peuvent être traitées au moyen d'une administration d'une quantité thérapeutiquement efficace d'au moins l'un des composés de formule I, on peut citer les hyperalgésies inflammatoires, les douleurs neuropathiques, les douleurs associées à un traumatisme ou à un cancer, les maladies inflammatoires du côlon, la polyarthrite rhumatoïde, le psoriasis, les obstructions pulmonaires chroniques ou le syndrome de détresse respiratoire aiguë.

L'invention concerne également une méthode de traitement de la douleur ou des maladies sus-mentionnées qui consiste à administrer, à un sujet en ayant besoin, une quantité thérapeutiquement efficace de composé de formule I.

La dose de principe actif dépend du mode d'administration et du type de pathologie ; elle est généralement comprise entre 0,05 et 10 mg/kg du sujet à traiter. En fonction du traitement envisagé, les composés de formule I ou leurs sels pourront être associés à d'autres principes actifs, et seront formulés avec des excipients couramment utilisés.

Dans le but d'obtenir une action rapide, notamment lorsqu'il s'agit de traiter une douleur aiguë, le mode d'administration du médicament se fera de préférence par injection, par exemple par voie intramusculaire ou sous-cutanée. Dans le cas de douleurs chroniques, l'administration du médicament peut être faite par le moyen de formulations galéniques communes, par exemple par voie orale sous forme de gélules ou de comprimés dans lesquels un composé selon l'invention est associé à des excipients connus de l'homme du métier, ou sous forme d'un patch adhésif dans lequel un composé selon l'invention est formulé avec des excipients connus de l'homme du métier pour favoriser le passage transdermique du principe actif.

## Revendications

**1.** Composé dérivé de benzènesulfonamide, **caractérisé en ce qu'**il est choisi parmi l'ensemble constitué par :

a) les composés de formule :

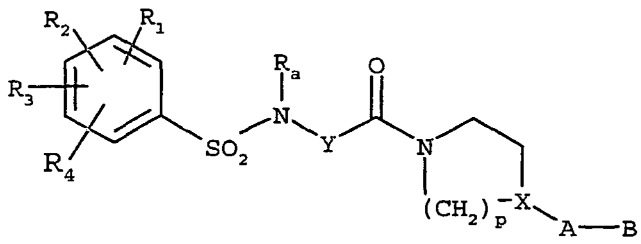

I

dans laquelle

- $R_1$, $R_2$, $R_3$, $R_4$ représentent chacun indépendamment un ou plusieurs des atomes

ou groupes d'atomes choisi(s) parmi l'atome d'hydrogène, les halogènes, les groupes alkyle en $C_1$-$C_3$, alcoxy en $C_1$-$C_3$, $CF_3$ ou $OCF_3$,

- $R_a$ représente un groupe alkyle en $C_1$-$C_4$,
- Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH_2-$,
- X représente CH ou un atome d'azote,
- p représente 2 ou 3,
- A représente une liaison simple, un atome d'azote éventuellement substitué par un groupe méthyle, ou un groupe alkylène en $C_1$-$C_5$, linéaire ou ramifié, éventuellement hydroxylé ou dont l'un des atomes de carbone est oxydé en une fonction cétone, à la condition que A et X ne représentent pas ensemble un atome d'azote,
- B représente un hétérocycle azoté ou un groupe amine éventuellement substitué par un ou deux groupes alkyle en $C_1$-$C_4$,

b) les sels d'addition des composés de formule I ci-dessus avec un acide.

2. Composé selon la revendication 1 **caractérisé en ce que** Y représente un groupe alkylène en $C_3$-$C_5$ interrompu par un atome d'oxygène, préférentiellement un groupe $-CH_2-CH_2-O-CH_2-$.

3. Composé selon la revendication 1 ou 2, **caractérisé en ce que** $R_2$ et $R_3$ représentent un groupe méthyle en position 2,6 sur le cycle aromatique.

4. Procédé de préparation d'un composé de formule I tel que défini à la revendication 1, et de ses sels d'addition, comprenant les étapes consistant à :

a) faire réagir un acide de formule :

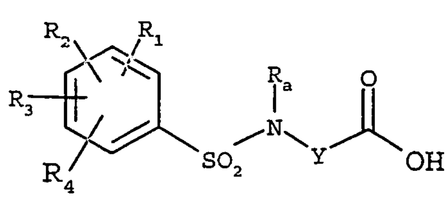

II

dans laquelle
$R_1$, $R_2$, $R_3$ et $R_4$ représentent chacun indépendamment un atome d'hydrogène ou d'halogène, un groupe allyle en $C_1$-$C_3$, alcoxy en $C_1$-$C_3$, $CF_3$ ou $OCF_3$,
$R_a$ représente un groupe alkyle en $C_1$-$C_4$,
Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH_2-$,
avec un hétérocyle azoté de formule :

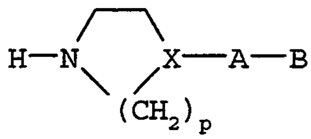

III

dans laquelle
X représente CH ou un atome d'azote,
p représente 2 ou 3,
A représente une liaison simple, un atome d'azote éventuellement substitué par un groupe méthyle (si X ne représente pas un atome d'azote), ou un groupe alkylène en $C_1$-$C_5$, linéaire ou ramifié, éventuellement hydroxylé ou dont l'un des atomes de carbone est oxydé en une fonction cétone,
B représente un hétérocycle azoté ou un groupe amine éventuellement substitué par un ou deux groupes alkyle en $C_1$-$C_4$, étant entendu que, en cas de présence d'un atome d'azote non substitué, cet atome d'azote est protégé par un groupe aminoprotecteur,
dans un solvant, en présence d'activateurs, à une température comprise entre la température ambiante et la température d'ébullition du solvant, pendant environ 2 à 15 heures, pour obtenir l'amide de formule :

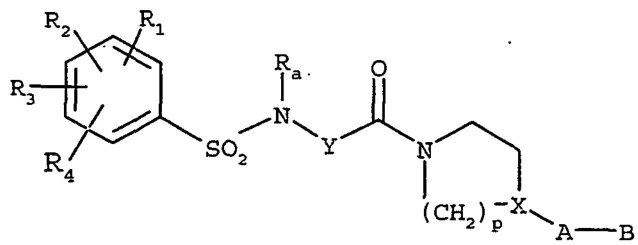

I

dans laquelle $R_1$, $R_2$. $R_3$, $R_4$, $R_a$, Y, p, X, A et B conservent la même signification que dans les composés de départ,
b) si nécessaire éliminer les groupes aminoprotecteurs,
c) si nécessaire obtenir un sel d'addition du composé de formule I avec un acide minéral ou organique.

**5.** Procédé de préparation d'un composé de formule I tel que défini à la revendication 1, et de ses sels d'addition, comprenant les étapes consistant à :

a) faire réagir un acide de formule :

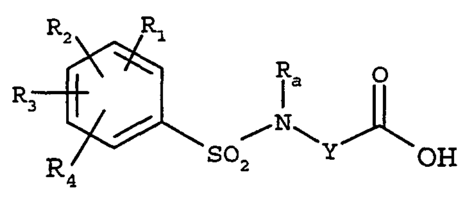

II

dans laquelle
$R_1$, $R_2$, $R_3$ et $R_4$ représentent chacun indépendamment un atome d'hydrogène ou d'halogène, un groupe alkyle en $C_1$-$C_3$ , alcoxy en $C_1$-$C_3$, $CF_3$ ou $OCF_3$,
$R_a$ représente un groupe alkyle en $C_1$-$C_4$,
Y représente un groupe alkylène en $C_2$-$C_5$ saturé, éventuellement interrompu par un atome d'oxygène, un groupe alkylène en $C_2$-$C_4$ insaturé, ou un groupe $-CH_2-CO-NH-CH$,
avec un agent de chloration, pour obtenir le chlorure d'acide de formule :

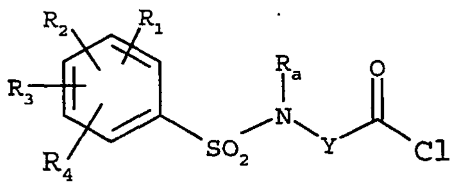

IIa

dans laquelle $R_1$, $R_2$, $R_3$, $R_4$, $R_a$ et Y ont la même signification que dans le composé de départ,
b) faire réagir le chlorure d'acide de formule IIa avec une amine de formule III telle que définie dans la revendication 4, pour obtenir le composé de formule I,
c) si nécessaire obtenir un sel d'addition du composé de formule I avec un acide minéral ou organique.

**6.** Procédé de préparation d'un composé de formule I tel que défini à la revendication 1, et de ses sels d'addition, comprenant les étapes consistant à :

a) faire réagir un composé acide de formule

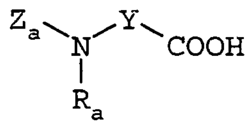

VII

dans laquelle Ra représente un groupe alkyle en $C_1$-$C_4$,
Y représente un groupe alkylène en $C_2$-$C_5$ saturé, éventuellement interrompu par un atome d'oxygène, et $Z_a$ représente un groupe aminoprotecteur,
avec un hétérocycle azoté de formule :

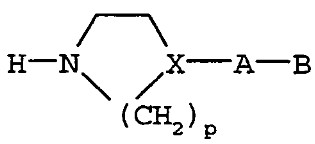

III

dans laquelle
X représente CH ou un atome d'azote,
p représente 2 ou 3,
A représente une liaison simple, un atome d'azote éventuellement substitué par un groupe méthyle (si X ne représente pas aussi un atome d'azote), ou un groupe alkylène en $C_1$-$C_5$, linéaire ou ramifié, éventuellement hydroxylé ou dont l'un des atomes de carbone est oxydé en une fonction cétone,
B représente un hétérocycle azoté ou un groupe amine éventuellement substitué par un ou deux groupes alkyle en $C_1$-$C_4$, étant entendu que, en cas de présence d'un atome d'azote non substitué sur ledit hétérocycle azoté, cet atome d'azote est protégé par un groupe aminoprotecteur différent du groupe aminoprotecteur utilisé pour le composé acide VII,
dans un solvant, en présence d'activateurs, à une température généralement comprise entre la température ambiante et la température d'ébullition du solvant, pendant environ 2 à 15 heures, pour obtenir l'amide de formule :

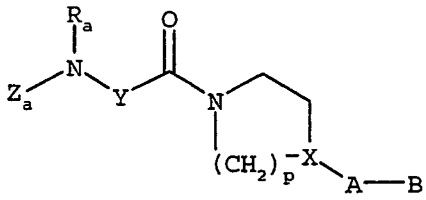

VIII

dans laquelle $Z_a$, $R_a$, Y, p, X, A et B conservent la même signification que dans les composés de départ,

b) éliminer le groupe amino protecteur $Z_a$, pour obtenir l'amine secondaire de formule

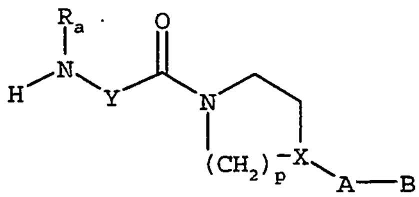

IX

dans laquelle $R_a$, Y, p, X, A et B conservent la même signification que dans le composé précédent,

c) faire réagir cette amine secondaire IX avec un chlorure de benzènesulfonyle de formule :

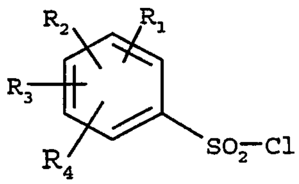

IV

dans laquelle $R_1$, $R_2$, $R_3$ et $R_4$ représentent chacun indépendamment un atome d'hydrogène ou d'halogène, un groupe alkyle en $C_1$-$C_3$, alcoxy en $C_1$-$C_3$, $CF_3$ ou $OCF_3$, dans un solvant, en présence d'une base organique aprotique, à une température comprise entre environ 0 et 50 °C, pendant environ 1 à 3 heures, pour obtenir le sulfonamide de formule :

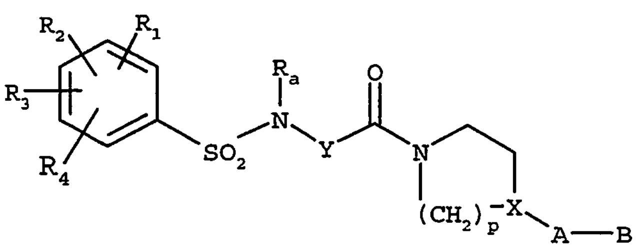

I

dans laquelle $R_1$, $R_2$, $R_3$, $R_4$, $R_a$, Y, p, X, A et B conservent la même signification que dans les composés de départ,

d) si nécessaire éliminer les groupes aminoprotecteurs,

e) si nécessaire obtenir un sel d'addition du composé de formule I avec un acide minéral ou organique.

7. Composition thérapeutique, **caractérisée en ce qu'**elle renferme, en association avec au moins un excipient physiologiquement acceptable, au moins un composé de formule I selon l'une des revendications 1 à 3, ou l'un de ses sels d'addition pharmaceutiquement acceptables avec un acide.

**8.** Utilisation d'un composé de formule I selon l'une des revendications 1 à 3 ou de l'un de ses sels d'addition pharmaceutiquement acceptables avec un acide, pour la préparation d'un médicament destiné au traitement de la douleur.

**9.** Utilisation d'un composé de formule I selon l'une des revendications 1 à 3 ou de l'un de ses sels d'addition pharmaceutiquement acceptables avec un acide, pour la préparation d'un médicament destiné au traitement de maladies inflammatoires.

## Claims

**1.** A benzenesulphonamide derivative compound, **characterized in that** it is selected from the group consisting of:

a) compounds of formula:

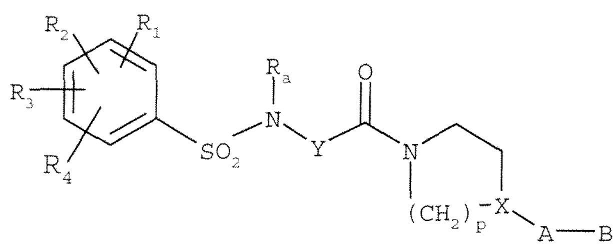

I

in which

- $R_1$, $R_2$, $R_3$, $R_4$ each independently represent one or more atoms or groups of atoms selected from a hydrogen atom, the halogens, $C_1$-$C_3$ alkyl groups, or $C_1$-$C_3$ alkoxy groups, $CF_3$ or $OCF_3$ groups,
- $R_a$ represents a $C_1$-$C_4$ alkyl group,
- Y represents a saturated $C_2$-$C_5$ alkylene group, optionally interrupted by an oxygen atom, an unsaturated $C_2$-$C_4$ alkylene group, or a $-CH_2$-CO-NH-$CH_2$-group,
- X represents CH or a nitrogen atom,
- p represents 2 or 3,
- A represents a single bond, a nitrogen atom optionally substituted with a methyl group, or a straight or branched $C_1$-$C_5$ alkylene group optionally hydroxylated or of which one of the carbon atoms is oxidized into a ketone function, provided that A and X together do not represent a nitrogen atom,
- B represents a nitrogen-containing heterocycle or an amine group optionally substituted with one or two $C_1$-$C_4$ alkyl groups,

b) addition salts of the above formula I compounds with an acid.

**2.** A compound according to claim 1, **characterized in that** Y represents a $C_3$-$C_5$ alkylene group interrupted by an oxygen atom, preferably a-$CH_2$-$CH_2$-O-$CH_2$- group.

**3.** A compound according to claim 1 or 2, **characterized in that** $R_2$ and $R_3$ represent a methyl group at position 2,6 on the aromatic ring.

**4.** A method for preparing a formula I compound as defined in claim 1, and its addition salts, comprising the steps consisting of:

a) allowing an acid of formula:

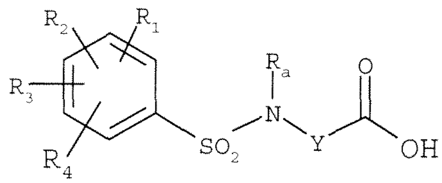

II

in which
$R_1$, $R_2$, $R_3$ and $R_4$ each independently represent a hydrogen or halogen atom, a $C_1$-$C_3$ alkyl group, or a $C_1$-$C_3$ alkoxy group, $CF_3$ or $OCF_3$ group,
$R_a$ represents a $C_1$-$C_4$ alkyl group,
Y represents a saturated $C_2$-$C_5$ alkylene group, optionally interrupted by an oxygen atom, an unsaturated $C_2$-$C_4$ alkylene group, or a $-CH_2-CO-NH-CH_2-$ group,
to react with a nitrogen-containing heterocycle of formula:

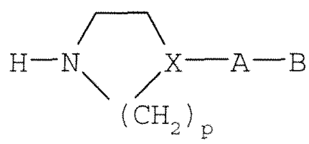

III

in which
X represents CH or a nitrogen atom,
p represents 2 or 3,
A represents a single bond, a nitrogen atom optionally substituted with a methyl group (if X does not represent a nitrogen atom), or a straight or branched $C_1$-$C_5$ alkylene group, optionally hydroxylated or of which one of the carbon atoms is oxidized into a ketone function,
B represents a nitrogen-containing heterocycle or an amine group optionally substituted with one or two $C_1$-$C_4$ alkyl groups, non the understanding that, should a non-substituted nitrogen atom be present, this nitrogen atom is protected by an amino-protecting group,
in a solvent, in the presence of activators, at a temperature lying between ambient temperature and the boiling point of the solvent, for approximately 2 to 15 hours, to obtain the amide of formula:

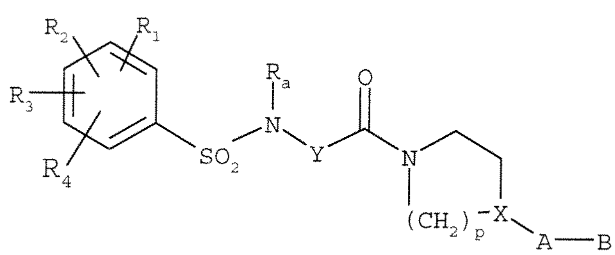

I

in which $R_1$, $R_2$, $R_3$, $R_4$, $R_a$, Y, p, X, A and B maintain the same meaning as in the starting products,
b) if necessary, removing the amino-protecting groups,
c) if necessary, obtaining an addition salt of the formula I compound with a mineral or organic acid.

**5.** A method for preparing a formula I compound as defined in claim 1, and its addition salts, comprising the steps consisting of:

a) allowing an acid of formula:

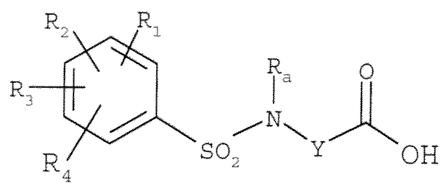

II

in which
$R_1$, $R_2$, $R_3$ and $R_4$ each independently represent a hydrogen or halogen atom, a $C_1$-$C_3$ alkyl group, or a $C_1$-$C_3$ alkoxy group, $CF_3$ or $OCF_3$ group,
$R_a$ represents a $C_1$-$C_4$ alkyl group,
Y represents a saturated $C_2$-$C_5$ alkylene group, optionally interrupted by an oxygen atom, an unsaturated $C_2$-$C_4$ alkylene group, or a $-CH_2-CO-NH-CH_2-$ group,
to react with a chlorination agent, to obtain the acid chloride of formula:

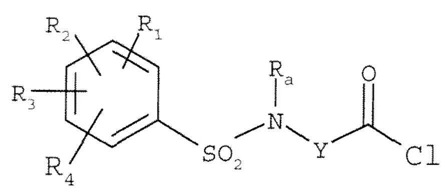

IIa

in which $R_1$, $R_2$, $R_3$, $R_4$, $R_a$ and Y have the same meaning as in the starting compound,
b) allowing the acid chloride of formula IIa to react with an amine of formula III as defined in claim 4, to obtain the compound of formula I,
c) if necessary, obtaining an addition salt of the formula I compound with a
mineral or organic acid.

**6.** A method for preparing a formula I compound such as defined in claim 1, and its addition salts, comprising the steps consisting of:

a) allowing an acid compound of formula:

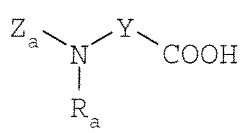

VII

in which Ra represents a $C_1$-$C_4$ alkyl group,
Y represents a saturated $C_2$-$C_5$ alkylene group, optionally interrupted by an oxygen atom, and $Z_a$ represents an amino-protecting group,
to react with a nitrogen-containing heterocycle of formula:

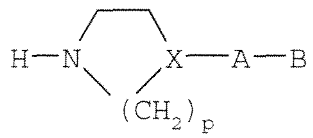

III

in which
X represents CH or a nitrogen atom,
p represents 2 or 3,
A represents a single bond, a nitrogen atom optionally substituted with a methyl group (if X does not also represent a nitrogen atom) or a straight or branched $C_1$-$C_5$ alkylene group, optionally hydroxylated or of which one of the carbon atoms is oxidized into a ketone function,
B represents a nitrogen-containing heterocycle or an amine group optionally substituted with one or two $C_1$-$C_4$ alkyl groups, on the understanding that, should a non-substituted nitrogen atom be present on said nitrogen-containing heterocycle, this nitrogen atom is protected by a different amino-protecting group to the amino-protecting group used for acid compound VII,
in a solvent, in the presence of activators, at a temperature generally lying between ambient temperature and the boiling point of the solvent, for approximately 2 to 15 hours, to obtain the amide of formula:

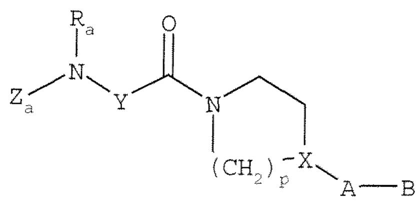

VIII

in which $Z_a$, $R_a$, Y, p, X, A and B maintain the same meaning as in the starting compounds,
b) removing the $Z_a$ amino-protecting group to obtain the secondary amine of formula:

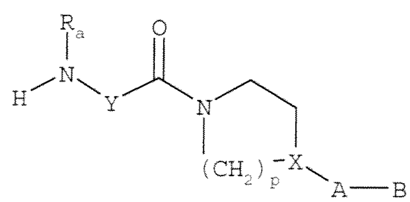

IX

in which $R_a$, Y, p, X, A and B maintain the same meaning as in the preceding compound,
c) allowing this secondary amine IX to react with a benzenesulphonyl chloride of formula:

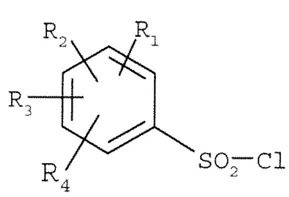

IV

in which $R_1$, $R_2$, $R_3$ and $R_4$ each independently represent a hydrogen or halogen atom, a $C_1$-$C_3$ alkyl group, or a $C_1$-$C_3$ alkoxy group, $CF_3$ or $OCF_3$ group,
in a solvent, in the presence of an aprotic organic base, at a temperature between approximately 0 and 50°C, for approximately 1 to 3 hours, to obtain the sulphonamide of formula:

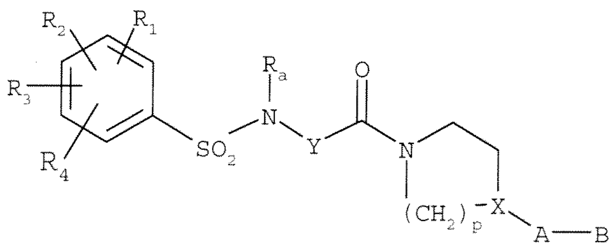

I

in which $R_1$, $R_2$, $R_3$, $R_4$, $R_a$, Y, p, X, A and B maintain the same meaning as in the starting compounds,
d) if necessary, removing the amino-protecting groups,
e) if necessary, obtaining an addition salt of the formula I compound with a mineral or organic acid.

**7.** A therapeutic composition, **characterized in that**, in association with at least one physiologically acceptable excipient, it contains at least one formula I compound according to any of claims 1 to 3, or one of its pharmaceutically acceptable addition salts with an acid.

**8.** Use of a formula I compound according to any of claims 1 to 3, or of one of its pharmaceutically acceptable addition salts with an acid, for the preparation of a medicinal product intended to treat pain.

**9.** Use of a formula I compound according to any of claims 1 to 3, or of one of its pharmaceutically acceptable addition salts with an acid, for the preparation of a medicinal product intended to treat inflammatory diseases.

**Patentansprüche**

**1.** Abgeleitete Benzolsulfonamidverbindung, **dadurch gekennzeichnet, daß** sie aus der Einheit ausgewählt ist, welche besteht aus:

a) den Verbindungen der Formel:

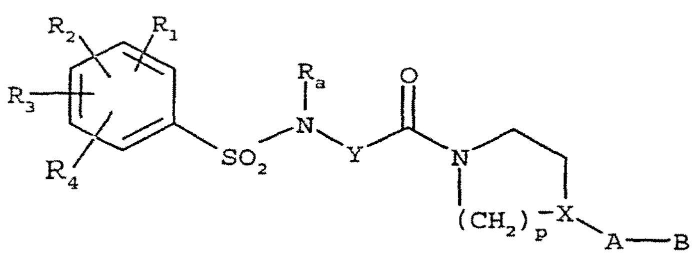

I

worin

- $R_1$, $R_2$, $R_3$, $R_4$ jeweils unabhängig ein(e) oder mehrere der Atome oder Atomgruppen, ausgewählt aus dem Wasserstoffatom, den Halogenen, den $C_1$-$C_3$-Alkyl-, $C_1$-$C_3$-Alkoxy-, $CF_3$- oder $OCF_3$-Gruppen darstellen,
- $R_a$ eine $C_1$-$C_4$-Alkylgruppe darstellt,
- Y eine gesättigte $C_2$-$C_5$-Alkylengruppe, eventuell unterbrochen durch ein Sauerstoffatom, eine ungesättigte $C_2$-$C_4$-Alkylengruppe oder eine -$CH_2$-CO-NH-$CH_2$-Gruppe darstellt,
- X CH oder ein Stickstoffatom darstellt,

- p 2 oder 3 darstellt,
- A eine einfache Bindung, ein Stickstoffatom, eventuell substituiert durch eine Methylgruppe, oder eine lineare oder verzweigte $C_1$-$C_5$-Alkylengruppe, die eventuell hydroxyliert ist oder von welcher eines der Kohlenstoffatome zu einer Keton-Funktion oxidiert ist, darstellt, unter der Bedingung, daß A und X nicht zusammen ein Stickstoffatom darstellen,
- B einen Stickstoff-Heterocyclus oder eine Amingruppe, eventuell substituiert durch eine oder zwei $C_1$-$C_4$-Alkylgruppe(n), darstellt,

b) den Additionssalzen der Verbindungen der obigen Formel I mit einer Säure.

**2.** Verbindung nach Anspruch 1, **dadurch gekennzeichnet, daß** Y eine $C_3$-$C_5$-Alkylengruppe, unterbrochen durch ein Sauerstoffatom, vorzugsweise eine $-CH_2-CH_2-O-CH_2$-Gruppe darstellt.

**3.** Verbindung nach Anspruch 1 oder 2, **dadurch gekennzeichnet, daß** $R_2$ und $R_3$ eine Methylgruppe in 2,6-Stellung an dem aromatischen Ring darstellen.

**4.** Verfahren zur Herstellung einer Verbindung der Formel I, wie sie in Anspruch 1 definiert ist, sowie ihrer Additionssalze, das die Schritte umfaßt, die darin bestehen:

a) eine Säure der Formel:

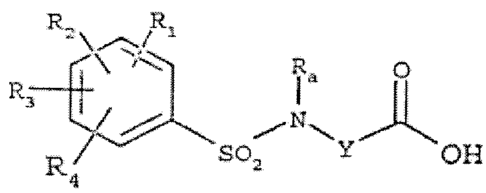

II

worin
$R_1$, $R_2$, $R_3$ und $R_4$ jeweils unabhängig ein Wasserstoff- oder Halogenatom, eine $C_1$-$C_3$-Alkyl-, $C_1$-$C_3$-Alkoxy-, $CF_3$- oder $OCF_3$-Gruppe darstellen,
$R_a$ eine $C_1$-$C_4$-Alkylgruppe darstellt,
Y eine gesättigte $C_2$-$C_5$-Alkylengruppe, eventuell unterbrochen durch ein Sauerstoffatom, eine ungesättigte $C_2$-$C_4$-Alkylengruppe oder eine $-CH_2-CO-NH-CH_2$-Gruppe darstellt,
mit einem Stickstoff-Heterocyclus der Formel:

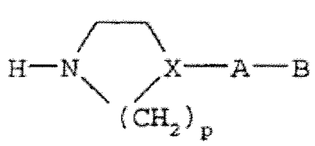

III

worin
X CH oder ein Stickstoffatom darstellt,
p 2 oder 3 darstellt,
A eine einfache Bindung, ein Stickstoffatom, eventuell substituiert durch eine Methylgruppe (wenn X nicht ein Stickstoffatom darstellt), oder eine lineare oder verzweigte $C_1$-$C_5$-Alkylengruppe darstellt, die eventuell hydroxyliert ist oder von welcher eines der Kohlenstoffatome zu einer Keton-Funktion oxidiert ist,
B einen Stickstoff-Heterocyclus oder eine Amingruppe, eventuell substituiert durch eine oder zwei $C_1$-$C_4$-Alkylgruppe(n) darstellt, mit der Maßgabe, daß im Falle des Vorliegens eines unsubstituierten Stickstoffatoms, dieses Stickstoffatom durch eine Aminoschutzgruppe geschützt ist,
in einem Lösungsmittel in Gegenwart von Aktivierungsmitteln, bei einer Temperatur zwischen der Umgebungstemperatur und der Siedetemperatur des Lösungsmitteln, für etwa 2 bis 15 Stunden reagieren zu lassen, um

das Amid folgender Formel zu erhalten:

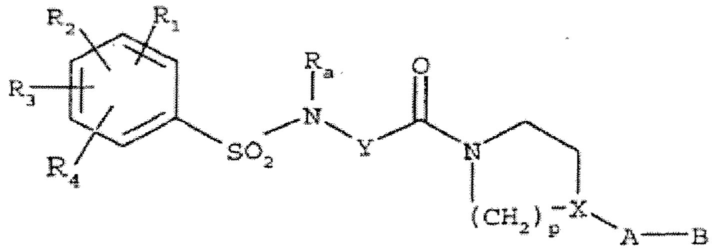

I

worin $R_1$, $R_2$, $R_3$, $R_4$, $R_a$, Y, p, X, A und B die gleiche Bedeutung wie bei den Ausgangsverbindungen behalten,
b) falls erforderlich die Aminoschutzgruppen zu entfernen,
c) falls erforderlich ein Additionssalz der Verbindung der Formel I mit einer mineralischen oder organischen Säure zu erhalten.

**5.** Verfahren zur Herstellung einer Verbindung der Formel I, wie sie in Anspruch 1 definiert ist, sowie ihrer Additionssalze, das die Schritte umfaßt, die darin bestehen:

a) eine Säure der Formel:

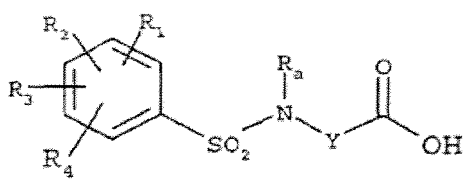

II

worin
$R_1$, $R_2$, $R_3$ und $R_4$ jeweils unabhängig ein Wasserstoff- oder Halogenatom, eine $C_1$-$C_3$-Alkyl-, $C_1$-$C_3$-Alkoxy-, $CF_3$- oder $OCF_3$-Gruppe darstellen,
$R_a$ eine $C_1$-$C_4$-Alkylgruppe darstellt,
Y eine gesättigte $C_2$-$C_5$-Alkylengruppe, eventuell unterbrochen durch ein Sauerstoffatom, eine ungesättigte $C_2$-$C_4$-Alkylengruppe oder eine $-CH_2-CO-NH-CH_2-$Gruppe darstellt,
mit einem Chlorierungsmittel reagieren zu lassen, um das Säurechlorid folgender Formel zu erhalten:

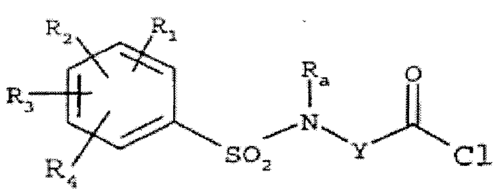

IIa

worin $R_1$, $R_2$, $R_3$, $R_4$, $R_a$ und Y die gleiche Bedeutung wie bei der Ausgangsverbindung haben,
b) das Säurechlorid der Formel IIa mit einem Amin der Formel III, wie es in Patentanspruch 4 definiert ist, reagieren zu lassen, um die Verbindung der Formel I zur erhalten,
c) falls erforderlich ein Additionssalz der Verbindung der Formel I mit einer mineralischen oder organischen Säure zu erhalten.

**6.** Verfahren zur Herstellung einer Verbindung der Formel I, wie sie in Anspruch 1 definiert ist, sowie ihrer Additionssalze, das die Schritte umfaßt, die darin bestehen:

a) eine saure Verbindung der Formel

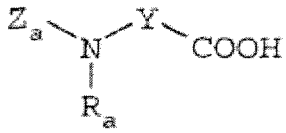

VII

worin $R_a$ eine $C_1$-$C_4$-Alkylgruppe darstellt,
Y eine gesättigte $C_2$-$C_5$-Alkylengruppe, eventuell unterbrochen durch ein Sauerstoffatom, und $Z_a$ eine Aminoschutzgruppe darstellt,
mit einem Stickstoff-Heterocyclus der Formel:

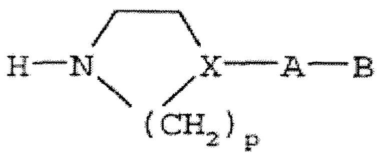

III

worin
X CH oder ein Stickstoffatom darstellt,
p 2 oder 3 darstellt,
A eine einfache Bindung, ein Stickstoffatom, eventuell substituiert durch eine Methylgruppe (wenn X nicht auch ein Stickstoffatom darstellt), oder eine lineare oder verzweigte $C_1$-$C_5$-Alkylengruppe darstellt, die eventuell hydroxyliert ist oder von welcher eines der Kohlenstoffatome zu einer Keton-Funktion oxidiert ist,
B einen Stickstoff-Heterocyclus oder eine Amingruppe, eventuell substituiert durch eine oder zwei $C_1$-$C_4$-Alkylgruppe(n), darstellt, mit der Maßgabe, daß im Falle des Vorliegens eines unsubstituierten Stickstoffatoms an dem Stickstoff-Heterocyclus, dieses Stickstoffatom durch eine Aminoschutzgruppe, die sich von der für die saure Verbindung VII verwendeten Aminoschutzgruppe unterscheidet, geschützt ist,
in einem Lösungsmittel in Gegenwart von Aktivierungsmitteln, bei einer Temperatur im allgemeinen zwischen der Umgebungstemperatur und der Siedetemperatur des Lösungsmitteln, für etwa 2 bis 15 Stunden reagieren zu lassen, um das Amid folgender Formel zu erhalten:

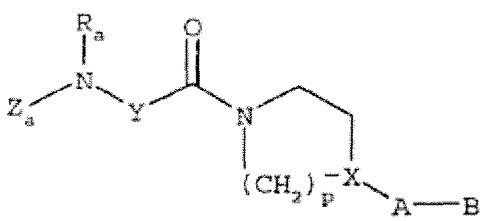

VIII

worin $Z_a$, $R_a$, Y, p, X, A und B die gleiche Bedeutung wie bei den Ausgangsverbindungen behalten,
b) die Aminoschutzgruppe $Z_a$ zu entfernen, um das sekundäre Amin folgender Formel zu erhalten:

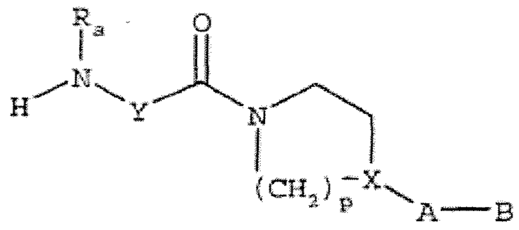

IX

worin $R_a$, Y, p, X, A und B die gleiche Bedeutung wie bei der vorhergehenden Verbindung behalten,
c) dieses sekundäre Amin IX mit einem Benzolsulfonylchlorid der Formel:

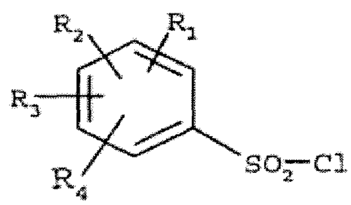

IV

worin $R_1$, $R_2$, $R_3$ und $R_4$ jeweils unabhängig ein Wasserstoff- oder Halogenatom, eine $C_1$-$C_3$-Alkyl-, $C_1$-$C_3$-Alkoxy-, $CF_3$- oder $OCF_3$-Gruppe darstellen,
in einem Lösungsmittel, in Anwesenheit einer aprotischen organischen Base, bei einer Temperatur zwischen etwa 0 und 50°C, für ca. 1 bis 3 Stunden reagieren zu lassen, um das Sulfonamid folgender Formel zu erhalten:

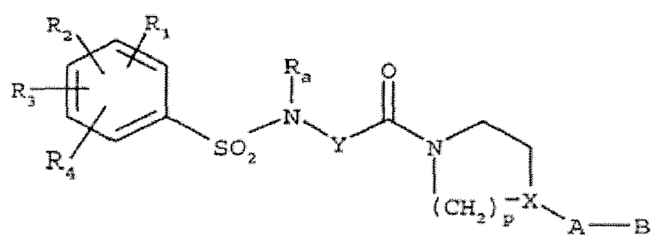

I

worin $R_1$, $R_2$, $R_3$, $R_4$, $R_a$, Y, p, X, A und B die gleiche Bedeutung wie bei den Ausgangsverbindungen behalten,
d) falls erforderlich die Aminoschutzgruppen zu entfernen,
e) falls erforderlich ein Additionssalz der Verbindung der Formel I mit einer mineralischen oder organischen Säure zu erhalten.

**7.** Therapeutische Zusammensetzung, **dadurch gekennzeichnet, daß** sie in Verbindung mit wenigstens einem physiologisch akzeptablen Bindemittel wenigstens eine Verbindung der Formel I nach einem der Ansprüche 1 bis 3 oder eines ihrer pharmazeutisch akzeptablen Additionssalze mit einer Säure enthält.

**8.** Verwendung einer Verbindung der Formel I nach einem der Ansprüche 1 bis 3 oder eines ihrer pharmazeutisch akzeptablen Additionssalze mit einer Säure für die Herstellung eines Medikaments, das für die Schmerzbehandlung bestimmt ist.

**9.** Verwendung einer Verbindung der Formel I nach einem der Ansprüche 1 bis 3 oder eines ihrer pharmazeutisch akzeptablen Additionssalze mit einer Säure für die Herstellung eines Medikaments, das für die Behandlung von Entzündungskrankheiten bestimmt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*


### Documents brevets cités dans la description

- EP 236163 A
- EP 236164 A
- EP 614911 A
- EP 558961 A
- WO 9216549 A1
- WO 9725315 A
- WO 9640639 A
- WO 9724349 A
- WO 9803503 A
- WO 9824783 A
- WO 9900387 A
- WO 0105783 A
- WO 02099388 A
- WO 9709346 A

### Littérature non-brevet citée dans la description

- *Pharmazie,* 1984, vol. 39 (5), 315-317
- *Pharmazie,* 1986, vol. 41 (4), 233-235
- **Shibata, M. ; Ohkubo, T. ; Takahashi, H. ; R. Inoki.** Modified formalin test: characteristic biphasic pain response. *Pain,* vol. 38, 347-352